(19) Europäisches Patentamt
European Patent Office
Office européen des brevets

(11) EP 3 674 291 A1

(12) **EUROPEAN PATENT APPLICATION**
published in accordance with Art. 153(4) EPC

(43) Date of publication:
**01.07.2020 Bulletin 2020/27**

(21) Application number: **18848338.2**

(22) Date of filing: **20.08.2018**

(51) Int Cl.:
***C07D 213/85*** *(2006.01)* ***A01N 43/40*** *(2006.01)*
***A01N 43/56*** *(2006.01)* ***A01N 43/60*** *(2006.01)*
***A01N 43/72*** *(2006.01)* ***A01N 43/76*** *(2006.01)*
***A01N 43/80*** *(2006.01)* ***A01N 43/836*** *(2006.01)*
***A01N 43/84*** *(2006.01)* ***A01N 43/86*** *(2006.01)*
***A01N 43/90*** *(2006.01)* ***A01N 47/02*** *(2006.01)*
***A01N 47/16*** *(2006.01)* ***A01N 47/40*** *(2006.01)*
***A01P 7/02*** *(2006.01)* ***A01P 7/04*** *(2006.01)*
***A61K 31/444*** *(2006.01)* ***A61K 31/4545*** *(2006.01)*
***A61K 31/496*** *(2006.01)* ***A61K 31/5355*** *(2006.01)*
***A61K 31/5377*** *(2006.01)*

(86) International application number:
**PCT/JP2018/030637**

(87) International publication number:
**WO 2019/039429 (28.02.2019 Gazette 2019/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.08.2017 JP 2017159583**
**12.09.2017 JP 2017174777**
**16.11.2017 JP 2017221113**

(71) Applicant: **Nippon Soda Co., Ltd.**
**Tokyo 100-8165 (JP)**

(72) Inventors:
- **NISHIO Fumiya**
**Odawara-shi**
**Kanagawa 250-0280 (JP)**
- **IWATA Jun**
**Odawara-shi**
**Kanagawa 250-0280 (JP)**
- **MAKINO Satoshi**
**Odawara-shi**
**Kanagawa 250-0280 (JP)**
- **IWASA Takao**
**Odawara-shi**
**Kanagawa 250-0280 (JP)**
- **HIRATA Koichi**
**Odawara-shi**
**Kanagawa 250-0280 (JP)**
- **HAMAMOTO Isami**
**Odawara-shi**
**Kanagawa 250-0280 (JP)**

(74) Representative: **Mewburn Ellis LLP**
**Aurora Building**
**Counterslip**
**Bristol BS1 6BX (GB)**

# (54) CYCLIC AMINE COMPOUND AND PEST CONTROL AGENT

(57) A pest control agent according to the present invention contains a compound of formula (I) (in the formula, Ar represents a substituted or unsubstituted C6-10 aryl group, $R^1$ represents a cyano group or a substituted or unsubstituted thiocarbamoyl group, $R^2$ represents a hydrogen atom, a halogeno group, a substituted or unsubstituted C1-6 alkyl group, a hydroxyl group, or a substituted or unsubstituted C1-6 alkoxy group, $R^3$ represents a hydrogen atom, a halogeno group, a substituted or unsubstituted CI-6 alkyl group, or the like, and Q represents a cyclic amino group) or a salt thereof.

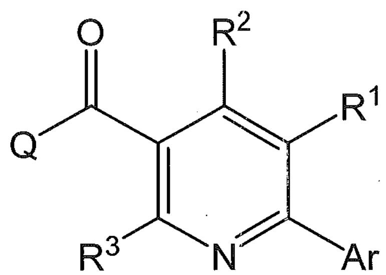

(I)

EP 3 674 291 A1

## Description

TECHNICAL FIELD

The present invention relates to a cyclic amine compound and a pest control agent. Furthermore, the present invention relates to a cyclic amine compound that has excellent insecticidal and/or acaricidal activity and excellent safety and that can be synthesized industrially advantageously, and to a pest control agent containing the same as an active ingredient thereof.

The present invention claims priority on the basis of Japanese Patent Application No. 2017-159583 filed in Japan on August 22, 2017, Japanese Patent Application No. 2017-174777 filed in Japan on September 12, 2017, and Japanese Patent Application No. 2017-221113 filed in Japan on November 16, 2017, the contents of which are incorporated herein by reference.

BACKGROUND OF THE INVENTION

With respect to a compound structurally related to a cyclic amine compound according to the present invention, Patent Document 1 discloses compounds of formula (A) or (B) shown below. These compounds have been shown to be useful in insecticides or acaricides.

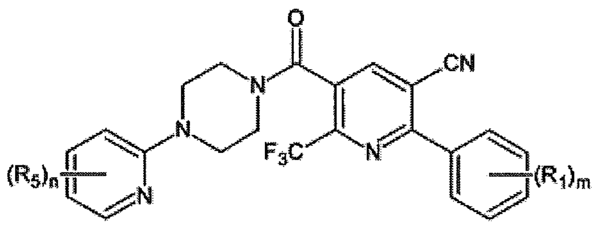

(A)

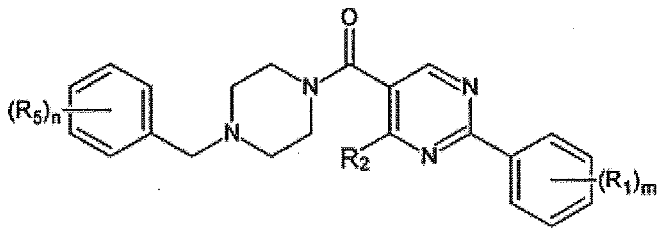

(B)

DOCUMENTS OF RELATED ART

Patent Documents

Patent Document 1: WO 2015/032280 A1

SUMMARY OF THE INVENTION

PROBLEMS TO BE SOLVED BY THE INVENTION

The present invention aims to provide a cyclic amine compound which is excellent in pest control activity, particularly insecticidal and/or acaricidal activity, is excellent in safety, and can be synthesized in an industrially advantageous manner, and provide a pest control agent containing the cyclic amine compound as an active ingredient thereof.

MEANS TO SOLVE THE PROBLEMS

The present invention encompassing the following aspects has been completed as a result of studying to solve the above-described problems.

(1) A compound of formula (I) or a salt thereof.

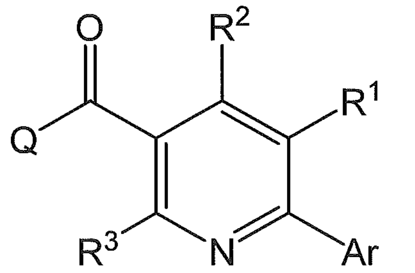

(I)

In the formula (I),
Ar represents a substituted or unsubstituted C6-10 aryl group,
$R^1$ represents a cyano group or a substituted or unsubstituted thiocarbamoyl group,
$R^2$ represents a hydrogen atom, a halogeno group, a substituted or unsubstituted C1-6 alkyl group, a hydroxyl group, or a substituted or unsubstituted C1-6 alkoxy group,
$R^3$ represents a hydrogen atom, a halogeno group, a substituted or unsubstituted Cl-6 alkyl group, a substituted or unsubstituted C3-8 cycloalkyl group, a hydroxyl group, a substituted or unsubstituted C1-6 alkoxy group, a substituted or unsubstituted C6-10 aryl group, or a substituted or unsubstituted 5- or 6-membered heteroaryl group, and
Q represents a cyclic amino group of formula (II-a), a cyclic amino group of formula (II-b), or a cyclic amino group of formula (II-c).

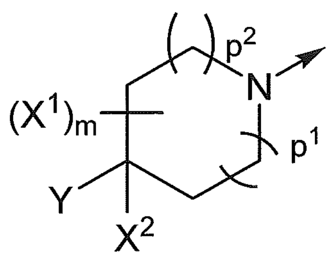

(II-a)

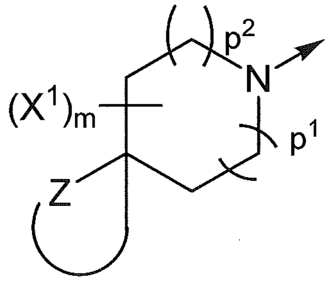

(II-b)

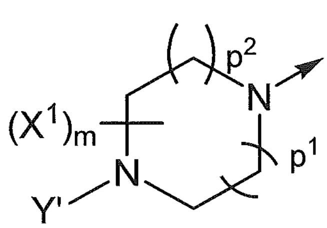

(II-c)

In the formula (II-a), an arrow indicates a binding position,
$p^1$ indicates the number of methylenes in parentheses and is 0 or 1,
$p^2$ indicates the number of methylenes in parentheses and is an integer of 0 to 2,
$X^1$ indicates a substituent on the cyclic amino group, and represents a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group,
m indicates the number of $X^1$ and is an integer of 0 to 4,

$X^2$ represents a hydrogen atom, a halogeno group, a substituted or unsubstituted C1-6 alkyl group, a hydroxyl group, or a substituted or unsubstituted C1-6 alkoxy group,
Y represents a halogeno group, a substituted or unsubstituted C1-6 alkyl group, a substituted or unsubstituted C2-6 alkenyl group, a substituted or unsubstituted C1-6 alkoxy group, a substituted or unsubstituted C1-6 alkylcarbonyl group, a substituted or unsubstituted C1-6 alkoxycarbonyl group, a thiocarbamoyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 arylcarbonyl group, a group of $R^aO-N=CR^b-$, a group of $R^cCONR^d-$, a group of $R^cCOCH_2NR^d-$, a group of $R^eSO_2NR^f-$, a group of $R^gR^hN-CO-$, a group of $R^gR^hN-SO_2-$, or a cyanothio group,
$R^a$ represents a hydrogen atom or a substituted or unsubstituted C1-6 alkyl group,
$R^b$ represents a hydrogen atom or a substituted or unsubstituted C1-6 alkyl group,
$R^c$ represents a substituted or unsubstituted CI-6 alkyl group, a substituted or unsubstituted C1-6 alkoxy group, or a substituted or unsubstituted C6-10 aryl group,
$R^d$ represents a hydrogen atom or a substituted or unsubstituted C1-6 alkyl group,
$R^c$ and $R^d$ may be combined to form a substituted or unsubstituted C3-6 alkylene group, a substituted or unsubstituted C2-6 alkyleneoxy group, or a substituted or unsubstituted C1-6 alkyleneoxy C1-6 alkylene group,
$R^e$ represents a substituted or unsubstituted C1-6 alkyl group, or a substituted or unsubstituted C6-10 aryl group,
$R^f$ represents a hydrogen atom or a substituted or unsubstituted C1-6 alkyl group,
$R^e$ and $R^f$ may be combined to form a substituted or unsubstituted C3-6 alkylene group,
$R^g$ represents a substituted or unsubstituted CI-6 alkyl group, a substituted or unsubstituted C1-6 alkoxy group, a substituted or unsubstituted C6-10 aryl group, or a substituted or unsubstituted C1-6 alkylsulfonyl group,
$R^h$ represents a hydrogen atom or a substituted or unsubstituted C1-6 alkyl group,
$R^g$ and $R^h$ may be combined to form a substituted or unsubstituted C3-6 alkylene group.
In the formula (II-b), an arrow indicates a binding position,
$p^1$ indicates the number of methylenes in parentheses and is 0 or 1,
$p^2$ indicates the number of methylenes in parentheses and is an integer of 0 to 2,
$X^1$ indicates a substituent on the cyclic amino group, and is a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group,
m indicates the number of $X^1$ and is an integer of 0 to 4,
Z represents an oxo group, a group of $G^b-O-N=$, a substituted or unsubstituted C2-6 alkylene group, a group of $-O-G^a-$, a group of $-O-G^a-O-$, a group of $-O-CO-G^a-$, a group of $-G^a-O-G^a-$, a group of $-NG^b-CO-G^a-$, a group of $-CO-NG^b-G^a-$, or a group of $-G^a-NG^b-CO-G^a-$,
$G^a$ each independently represents a substituted or unsubstituted C1-6 alkylene group, and $G^b$ each independently represents a substituted or unsubstituted C1-6 alkyl group.
In the formula (II-c), an arrow indicates a binding position,
$p^1$ indicates the number of methylenes in parentheses and is 0 or 1,
$p^2$ indicates the number of methylenes in parentheses and is an integer of 0 to 2,
$X^1$ indicates a substituent on the cyclic amino group, and is a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group,
m represents the number of $X^1$ and is an integer of 0 to 4,
Y' represents a substituted or unsubstituted C1-6 alkylcarbonyl group, a substituted or unsubstituted C1-6 alkoxycarbonyl group, a group of $R^{g'}R^hN-CO-$, a group of $R^{g'}R^hN-CS-$, a group of $R^{g'}R^hN-CO-CO-$, a substituted or unsubstituted C1-6 alkylsulfonyl group, or a group of $R^{g'}R^hN-SO_2-$,
$R^{g'}$ represents a substituted or unsubstituted C1-6 alkyl group, a substituted or unsubstituted C1-6 alkoxy group, or a substituted or unsubstituted C6-10 aryl group,
$R^h$ represents a hydrogen atom or a substituted or unsubstituted C1-6 alkyl group, and
$R^{g'}$ and $R^h$ may be combined to form a substituted or unsubstituted C3-6 alkylene group.
(2) The compound of formula (I) or the salt thereof according to (1), wherein Q represents a cyclic amino group of formula (II-a), Y represents a substituted C1-6 alkyl group, a substituent on the substituted C1-6 alkyl group is a halogeno group, a hydroxyl group, a C1-6 alkoxy group, a C1-6 haloalkoxy group, a C1-6 alkylthio group, a C1-6 alkylsulfinyl group, a C1-6 alkylsulfonyl group, a C1-6 haloalkylthio group, a C1-6 haloalkylsulfinyl group, a C1-6 haloalkylsulfonyl group, a substituted or unsubstituted C6-10 aryl group, a substituted or unsubstituted C6-10 aryloxy group, a substituted or unsubstituted 5- or 6-membered heteroaryl group, a substituted or unsubstituted 5- or 6-membered heteroaryloxy group, a S-C1-6 alkylsulfonimidoyl group, a N-cyano-S-C1-6 alkylsulfonimidoyl group, a carboxyl group, a C1-6 alkylcarbonyl group, a C1-6 alkoxycarbonyl group, a C1-6 alkylcarbonylthio group, a group of $R^jR^kN-$, a group of $R^jR^kN-CO-$, a group of $R^jR^kN-SO_2-$, a group of $R^mCONR^n-$, a group of $R^mSO_2NR^n-$, a group

of $R^pO-N=CR^q-$, or a cyano group,

$R^j$ represents a C1-6 alkyl group or a CI-6 alkoxy group, $R^k$ represents a hydrogen atom or a C1-6 alkyl group, $R^j$ and $R^k$ may be combined to form a C3-6 alkylene group,

$R^m$ represents a C1-6 alkyl group, $R^n$ represents a hydrogen atom or a C1-6 alkyl group,

$R^p$ represents a C1-6 alkyl group, and $R^q$ represents a hydrogen atom or a C1-6 alkyl group.

(3) A pest control agent containing, as an active ingredient thereof, at least one selected from the group consisting of the compound and the salt thereof of the above-mentioned (1).

(4) An insecticide or acaricide containing, as an active ingredient thereof, at least one selected from the group consisting of the compound and the salt thereof of the above-mentioned (1).

(5) An ectoparasite control or expellant agent containing, as an active ingredient thereof, at least one selected from the group consisting of the compound and the salt thereof of the above-mentioned (1).

EFFECTS OF THE INVENTION

A cyclic amine compound according to the present invention makes it possible to control pests that cause problems in agricultural crops and hygiene. The cyclic amine compound particularly makes it possible to control agricultural insect pests and acarians at a low concentration effectively. In addition, the cyclic amine compound makes it possible to effectively control ectoparasites that cause harm to humans or livestock.

EMBODIMENTS FOR CARRYING OUT THE INVENTION

[Cyclic amine compound]

A cyclic amine compound according to the present invention is a compound of formula (I) (hereinafter, may be indicated as compound (I)) or a salt of the compound (I).

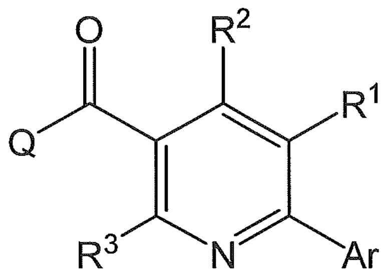

(I)

In the present invention, the term "unsubstituted" refers to a group consisting of a mother nucleus. In the case where only the name of a group serving as a mother nucleus is provided, this refers to "unsubstituted" unless specifically indicated otherwise.

On the other hand, the term "substituted" means that any hydrogen atom of a group serving as a mother nucleus is substituted with a group having a structure that is identical to or different from the mother nucleus. Thus, a "substituent" is another group bound to a group serving as the mother nucleus. The number of substituent may be one or two or more. Two or more substituents may be identical to or different from each other.

There are no particular limitations on a "substituent" provided that the substituent is chemically acceptable and achieves effects of the present invention.

Examples of a group that can be a "substituent" include the following groups:

halogeno groups such as a fluoro group, a chloro group, a bromo group, and an iodo group;

C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;

C2-6 alkenyl groups such as a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group;

C2-6 alkynyl groups such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a

2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group;
C3-8 cycloalkyl groups such as a cyclopropyl group, a cyclobutyl group, a cyclopentyl group, a cyclohexyl group, a cycloheptyl group, and a cubanyl group;
C3-8 cycloalkenyl groups such as a 2-cyclopropenyl group, a 2-cyclopentenyl group, a 3-cyclohexenyl group, and a 4-cyclooctenyl group;
C6-10 aryl groups such as a phenyl group and a naphthyl group;
3- to 6-membered heterocyclyl groups;
a hydroxyl group; an oxo group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, and an allyloxy group;
C2-6 alkynyloxy groups such as an ethynyloxy group, and a propargyloxy group;
C6-10 aryloxy groups such as a phenoxy group, and a naphthoxy group;
3- to 6-membered heterocyclyloxy groups;
hydroxy C1-6 alkyl groups such as a hydroxymethyl group, and a hydroxyethyl group;
C1-6 alkoxyalkyl groups such as a methoxymethyl group and an ethoxymethyl group;
C1-6 alkoxy C1-6 alkoxy groups such as amethoxymethoxy group, and an ethoxymethoxy group;
a carboxyl group;
a formyl group; C1-6 alkylcarbonyl groups such as an acetyl group, and a propionyl group;
a formyloxy group; C1-6 alkylcarbonyloxy groups such as an acetyloxy group, a propionyloxy group, a n-propylcarbonyloxy group, and an i-propylcarbonyloxy group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
C6-10 arylcarbonyl group such as a benzoyl group;
C1-6 haloalkyl groups such as a chloromethyl group, a chloroethyl group, a difluoromethyl group, a trifluoromethyl group, a 2,2,2-trifluoroethyl group, a perfluoroethyl group, a perfluoropropan-2-yl group, a 1-fluoro-n-butyl group, and a perfluoro-n-pentyl group;
C1-6 alkoxy C1-6 haloalkyl groups such as a 1,1,1,3,3,3-hexafluoro-2-methoxypropan-2-yl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C3-6 halocycloalkyl groups such as a 1,1-difluorocyclopropyl group, and a 3,3-difluorocyclobutyl group;
C1-6 haloalkoxy groups such as a difluoromethoxy group, a trifluoromethoxy group, a 2,2,2-trifluoroethoxy group, a 1,1,2,2-tetrafluoroethoxy group, a 2,2,3,3,3-pentafluoropropoxy group, a 4,4,4-trifluorobutoxy group, and a (1,1,1,3,3,3-hexafluoropropan-2-yl)oxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
a cyano group; a nitro group; an amino group;
C1-6 alkylamino groups such as a methylamino group, a dimethylamino group, and a diethylamino group;
C6-10 arylamino group such as an anilino group, and a naphthylamino group;
a formylamino group; C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
C1-6 alkylsulfoximino groups such as a S,S-dimethylsulfoximino group;
a carbamoyl group; a N'-hydroxycarbamimidoyl group;
C1-6 alkylaminocarbonyl group such as a methylaminocarbonyl group, a dimethylaminocarbonyl group, an ethylaminocarbonyl group, and an i-propylaminocarbonyl group;
imino C1-6 alkyl groups such as an iminomethyl group, a (1-imino)ethyl group, and a (1-imino)-n-propyl group;
hydroxyimino C1-6 alkyl groups such as a hydroxyiminomethyl group, a (1-hydroxyimino)ethyl group, and a (1-hydroxyimino)propyl group;
(C1-6 alkoxyimino)C1-6 alkyl groups such as a methoxyiminomethyl group, and a (1-methoxyimino)ethyl group;
(C1-6 alkylcarbonyloxyimino) C1-6 alkyl groups such as an acetoxyiminomethyl group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2, 2, 2-trifluoroethylthio group;

C2-6 alkenylthio groups such as a vinylthio group, and an allylthio group;
C2-6 alkynylthio groups such as an ethynylthio group, and a propargylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
C2-6 alkenylsulfinyl groups such as an allylsulfinyl group;
C2-6 alkynylsulfinyl groups such as a propargylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
C2-6 alkenylsulfonyl groups such as an allylsulfonyl group;
C2-6 alkynylsulfonyl groups such as a propargylsulfonyl group;
a thiocarbamoyl group;
imino(C1-6 alkylthio)methyl groups such as an imino(methylthio)methyl group;
a pentafluorosulfanyl group;
tri-C1-6 alkylsilyl groups such as a trimethylsilyl group, a triethylsilyl group, and a t-butyldimethylsilyl group; and
tri-C6-10 arylsilyl groups such as a triphenylsilyl group.

In addition, any hydrogen atom in the "substituent" may also be substituted with another group having a different structure.

The term "C1-6" means that the number of carbon atoms constituting a group serving as a mother nucleus is 1 to 6. The number of carbon atoms does not include the number of carbon atoms constituting a substituent. For example, an ethoxybutyl group is classified as a C2 alkoxy C4 alkyl group.

The "3- to 6-membered heterocyclyl group" is a group formed by removing one hydrogen atom from an arbitrary ring atom of a "3- to 6-membered heterocyclic compound", which is a 3- to 6-membered cyclic compound containing, as an atom constituting a ring (which may be referred to as ring member atom), 1 to 4 hetero atoms selected from the group consisting of a nitrogen atom, an oxygen atom and a sulfur atom.

The "3- to 6-membered heterocyclyl group" may be described as a "3- to 6-membered heterocyclyl group containing, as a ring member atom, 1 o 4 hetero atoms selected from the group consisting of a nitrogen atom, an oxygen atom and a sulfur atom".

The heterocyclyl group may be monocyclic or polycyclic. If at least one ring of a polycyclic heterocyclyl group is a hetero ring, remaining rings thereof may be any of saturated alicyclic rings, unsaturated alicyclic rings and aromatic rings. Examples of the "3- to 6-membered heterocyclyl group" include 3- to 6-membered saturated heterocyclyl groups, 5- to 6-membered heteroaryl groups, and 5- to 6-membered partially unsaturated heterocyclyl group.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

[Ar]

In the formula (I), Ar represents a substituted or unsubstituted C6-10 aryl group.

A C6-10 aryl group as Ar is a group formed by removing one hydrogen from a ring of a monocyclic or polycyclic aromatic hydrocarbon. Examples of the C6-10 aryl group include a phenyl group and a naphthyl group, and the C6-10 aryl group is preferably a phenyl group.

Examples of a substituent on the C6-10 aryl group include halogeno groups, substituted or unsubstituted C1-6 alkyl groups, substituted or unsubstituted C2-6 alkenyl groups, substituted or unsubstituted C2-6 alkynyl groups, substituted or unsubstituted C1-6 alkoxy groups, substituted or unsubstituted C2-6 alkenyloxy groups, a formyl group, substituted or unsubstituted C1-6 alkylcarbonyl groups, substituted or unsubstituted C1-6 alkoxycarbonyl groups, substituted or unsubstituted C1-6 alkylthio groups, substituted or unsubstituted C1-6 alkylsulfinyl groups, substituted or unsubstituted CI-6 alkylsulfonyl groups, substituted or unsubstituted C3-8 cycloalkyl groups, substituted or unsubstituted C6-10 aryl groups, substituted or unsubstituted 5- or 6-membered heteroaryl groups, substituted or unsubstituted C6-10 aryloxy groups, substituted or unsubstituted 5- or 6-membered heteroaryloxy groups, a group of $R^{31}O-N=CR^{32}-$, a thiocarbamoyl group, a hydroxyl group, a nitro group, and a cyano group.

$R^{31}$ represents a hydrogen atom or a substituted or unsubstituted C1-6 alkyl group, and $R^{32}$ represents a hydrogen atom or a substituted or unsubstituted C1-6 alkyl group.

Examples of the "halogeno group" as a substituent on the C6-10 aryl group include a fluoro group, a chloro group, a bromo group, and an iodo group.

The "C1-6 alkyl group" as a substituent on the C6-10 aryl group may be linear or branched. Examples of the alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Examples of the "C2-6 alkenyl group" as a substituent on the C6-10 aryl group include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "C2-6 alkynyl group" as a substituent on the C6-10 aryl group include an ethynyl group, a 1-propynyl group, a 2-propynyl group, a 1-butynyl group; a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1-6 alkoxy group" as a substituent on the C6-10 aryl group include a methoxy group, an ethoxy group, a n-propoxy group, a n-butoxy group, a n-pentyloxy group, a n-hexyloxy group, an i-propoxy group, an i-butoxy group, a s-butoxy group, a t-butoxy group, and an i-hexyloxy group.

Examples of the "C2-6 alkenyloxy group" as a substituent on the C6-10 aryl group include a vinyloxy group, an allyloxy group, a propenyloxy group, and a butenyloxy group.

Examples of the "C1-6 alkylcarbonyl group" as a substituent on the C6-10 aryl group include an acetyl group and a propionyl group.

Examples of the "C1-6 alkoxycarbonyl group" as a substituent on the C6-10 aryl group include a methoxycarbonyl group, an ethoxycarbonyl group, a n-propoxycarbonyl group, an i-propoxycarbonyl group, and a t-butoxycarbonyl group.

Examples of the "C1-6 alkylthio group" as a substituent on the C6-10 aryl group include a methylthio group, an ethylthio group, a n-propylthio group, a n-butylthio group, a n-pentylthio group, a n-hexylthio group, and an i-propylthio group.

Examples of the "C1-6 alkylsulfinyl group" as a substituent on the C6-10 aryl group include a methylsulfinyl group, an ethylsulfinyl group, and a t-butylsulfinyl group.

Examples of the "C1-6 alkylsulfonyl group" as a substituent on the C6-10 aryl group include a methylsulfonyl group, an ethylsulfonyl group, and a t-butylsulfonyl group.

Preferable examples of a substituent on the "C1-6 alkyl group", "C2-6 alkenyl group", "C2-6 alkynyl group", "C1-6 alkoxy group", "C2-6 alkenyloxy group", "C1-6 alkylcarbonyl group", "C1-6 alkoxycarbonyl group", "C1-6 alkylthio group", "C1-6 alkylsulfinyl group", or "C1-6 alkylsulfonyl group", which are substituents on the C6-10 aryl group, include: halogeno groups such as a fluoro group, a chloro group, a bromo group, and an iodo group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, 2,3-dichlorobutoxy group, and a trifluoromethoxy group; C6-10 aryl groups such as a phenyl group and a naphthyl group; and a cyano group.

Examples of the "C3-8 cycloalkyl group" as a substituent on the C6-10 aryl group include a cyclopropyl group, a cyclobutyl group, a cyclopentyl group, a cyclohexyl group, and a cycloheptyl group.

Examples of the "C6-10 aryl group" as a substituent on the C6-10 aryl group include a phenyl group and a naphthyl group.

Examples of the "5- or 6-membered heteroaryl group" as a substituent on the C6-10 aryl group include: 5-membered heteroaryl groups such as a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, and a tetrazolyl group; and 6-membered heteroaryl groups such as a pyridyl group, a pyrazinyl group, a pyrimidinyl group, a pyridazinyl group, and a triazinyl group.

Examples of the "C6-10 aryloxy group" as a substituent on the C6-10 aryl group include a phenoxy group, and a naphthoxy group.

Examples of the "5- or 6-membered heteroaryloxy group" as a substituent on the C6-10 aryl group include 5- or 6-membered heteroaryloxy groups such as a thiazolyloxy group, and a pyridyloxy group.

Preferable examples of a substituent on the "C3-8 cycloalkyl group", "C6-10 aryl group", "5- or 6-membered heteroaryl group", "C6-10 aryloxy group", or "5- or 6-membered heteroaryloxy group", which are substituents on the C6-10 aryl group, include: halogeno groups such as a fluoro group, a chloro group, a bromo group, and an iodo group; C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group; C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; and a cyano group.

In the "group of $R^{31}O-N=CR^{32}-$" as a substituent on the C6-10 aryl group, $R^{31}$ represents a hydrogen atom or a substituted or unsubstituted C1-6 alkyl group, and $R^{32}$ represents a hydrogen atom or a substituted or unsubstituted C1-6 alkyl group.

Examples of the "substituted or unsubstituted Cl-6 alkyl group" as $R^{31}$ and $R^{32}$ include the same groups as those mentioned above.

Examples of the group of $R^{31}O-N=CR^{32}-$ include a (methoxyimino)methyl group, an (ethoxyimino)methyl group, a (t-butoxyimino)methyl group, a (benzyloxyimino)methyl group, and a (2,2,2-trifluoroethoxyimino)methyl group.

Among these, the substituent on the C6-10 aryl group is preferably a halogeno group, a substituted or unsubstituted C1-6 alkyl group (more preferably a C1-6 alkyl group which may be substituted with a halogeno group), a substituted or unsubstituted C2-6 alkenyl group, a substituted or unsubstituted C1-6 alkoxy group (more preferably a C1-6 alkoxy group which may be substituted with a halogeno group), a substituted or unsubstituted C1-6 alkylthio group (more preferably a C1-6 alkylthio group which may be substituted with a halogeno group), a substituted or unsubstituted C3-8 cycloalkyl group (more preferably a C3-8 cycloalkyl group which may be substituted with a halogeno group), a substituted or unsubstituted C6-10 aryl group (more preferably a phenyl group which may be substituted with a halogeno group and/or a C1-6 alkoxy group which may be substituted with a halogeno group), a substituted or unsubstituted 5- or 6-membered heteroaryl group (more preferably a pyrazolyl group which may be substituted with a C1-6 alkyl group which may be substituted with a halogeno group), a group of $R^{31}O-N=CR^{32}-$, a thiocarbamoyl group, a hydroxyl group, or a cyano group.

[$R^1$, $R^2$, $R^3$]

$R^1$ in the formula (I) represents a cyano group or a substituted or unsubstituted thiocarbamoyl group.

$R^2$ in the formula (I) represents a hydrogen atom, a halogeno group, a substituted or unsubstituted C1-6 alkyl group, a hydroxyl group, or a substituted or unsubstituted C1-6 alkoxy group.

$R^3$ in the formula (I) represents a hydrogen atom, a halogeno group, a substituted or unsubstituted C1-6 alkyl group, a substituted or unsubstituted C3-8 cycloalkyl group, a hydroxyl group, a substituted or unsubstituted C1-6 alkoxy group, a substituted or unsubstituted C6-10 aryl group, or a substituted or unsubstituted 5- or 6-membered heteroaryl group.

Examples of the "substituted thiocarbamoyl group" as $R^1$ include mono or di C1-6 alkyl-substituted thiocarbamoyl groups, such as a methylthiocarbamoyl group, a dimethylthiocarbamoyl group, and an ethyl(methyl)thiocarbamoyl group.

Examples of the "halogeno group", "substituted or unsubstituted C1-6 alkyl group", and "substituted or unsubstituted C1-6 alkoxy group" as $R^2$ and $R^3$ include the same groups as those specifically mentioned as Ar.

In addition to the above, a C1-6 alkylthio group, a C1-6 alkylsulfinyl group, or a C1-6 alkylsulfonyl group is mentioned as a substituent of the "substituted C1-6 alkyl group" as $R^3$.

Examples of the "substituted or unsubstituted C3-8 cycloalkyl group", "substituted or unsubstituted C6-10 aryl group", or "5- or 6-membered heteroaryl group" as $R^3$ include the same groups as those specifically mentioned as Ar.

Among these, $R^2$ is preferably a hydrogen atom, and $R^3$ is preferably a hydrogen atom, a halogeno group, a substituted or unsubstituted C1-6 alkyl group (preferably a C1-6 alkyl group which may be substituted with a halogeno group, a C1-6 alkoxy group, a C1-6 alkylthio group, a C1-6 alkylsulfinyl group, or a C1-6 alkylsulfonyl group), a substituted or unsubstituted C3-8 cycloalkyl group, a substituted or unsubstituted C1-6 alkoxy group, or a substituted or unsubstituted C6-10 aryl group (preferably a phenyl group).

[Q]

Q represents a cyclic amino group of formula (II-a), a cyclic amino group of formula (II-b), or a cyclic amino group of formula (II-c).

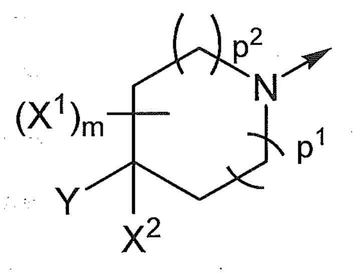

(II-a)

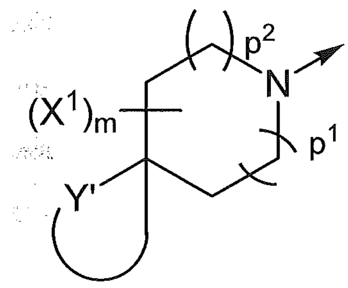

(II-b)

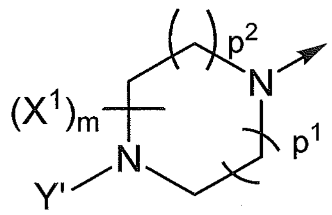

(II-c)

[Formula (II-a)]

In the formula (II-a), an arrow indicates a binding position.

In the formula (II-a), $p^1$ indicates the number of methylenes in parentheses and is 0 or 1, and preferably 1, and $p^2$ indicates the number of methylenes in parentheses and is an integer of 0 to 2, and preferably 1.

The cyclic amino group of formula (II-a) is represented by formula (II-a-1), when $p^1$ is 1 and $p^2$ is 1.

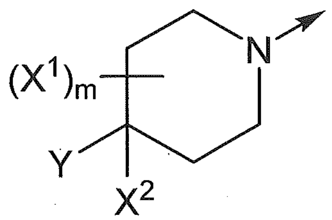

(II-a-1)

In the formula (II-a) or (II-a-1), $X^1$ indicates a substituent on the cyclic amino group, and is a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group, m indicates the number of $X^1$ and is an integer of 0 to 4 (preferably 0), and $X^2$ is a hydrogen atom, a halogeno group, a substituted or unsubstituted C1-6 alkyl group, a hydroxyl group, or a substituted or unsubstituted C1-6 alkoxy group.

Examples of the "halogeno group", "substituted or unsubstituted C1-6 alkyl group", or "substituted or unsubstituted C1-6 alkylthio group" as $X^1$ include the same groups as specifically mentioned as Ar.

Examples of the "C1-6 alkylsulfonyloxy group" as $X^1$ include a methylsulfonyloxy group, an ethylsulfonyloxy group, and a t-butylsulfonyloxy group.

Preferable examples of a substituent on the "C1-6 alkylsulfonyloxy group" include halogeno groups such as a fluoro group, a chloro group, a bromo group, and an iodo group.

Examples of the "halogeno group", "substituted or unsubstituted C1-6 alkyl group", or "substituted or unsubstituted C1-6 alkoxy group" as $X^2$ include the same groups as those specifically mentioned as Ar.

$X^2$ preferably represents a hydrogen atom, a halogeno group, a hydroxyl group, or a substituted or unsubstituted C1-6 alkoxy group.

In the formula (II-a) or (II-a-1), Y represents a halogeno group, a substituted or unsubstituted C1-6 alkyl group, a substituted or unsubstituted C2-6 alkenyl group, a substituted or unsubstituted C1-6 alkoxy group, a substituted or unsubstituted C1-6 alkylcarbonyl group, a substituted or unsubstituted C1-6 alkoxycarbonyl group, a thiocarbamoyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 arylcarbonyl group, a group of $R^aO-N=CR^b-$, a group of $R^cCONR^d-$, a group of $R^cCOCH_2NR^d-$, a group of $R^eSO_2NR^f-$, a group of $R^gR^hN-CO-$, a group of $R^gR^hN-SO_2-$, or a cyanothio group.

(1) Examples of the "halogeno group" as Y include a fluoro group, a chloro group, a bromo group, and an iodo group.
(2) The "C1-6 alkyl group" as Y may be linear or branched. Examples of the alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Examples of a substituent on the "C1-6 alkyl group" as Y include halogeno groups, a hydroxyl group, C1-6 alkoxy groups, C1-6 haloalkoxy groups, C1-6 alkylthio groups, C1-6 alkylsulfinyl groups, C1-6 alkylsulfonyl groups, C1-6 haloalkylthio groups, C1-6 haloalkylsulfinyl groups, C1-6 haloalkylsulfonyl groups, substituted or unsubstituted C6-10 aryl groups, substituted or unsubstituted C6-10 aryloxy groups, substituted or unsubstituted 5- or 6-membered heteroaryl groups, substituted or unsubstituted 5- or 6-membered heteroaryloxy groups, S-C1-6 alkylsulfonimidoyl groups, N-cyano-S-C1-6 alkylsulfonimidoyl groups, a carboxyl group, C1-6 alkylcarbonyl groups, C1-6 alkoxycarbonyl groups, C1-6 alkylcarbonylthio groups, groups of $R^jR^kN$-, groups of $R^jR^kN$-CO-, groups of $R^jR^kN$-$SO_2$-, groups of $R^mCONR^n$-, groups of $R^mSO_2NR^n$-, groups of $R^pO$-N=$CR^q$-, and a cyano group.

Specific examples thereof include: halogeno groups such as a fluoro group, a chloro group, a bromo group, and an iodo group; a hydroxyl group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; C1-6 alkylthio groups such as a methylthio group, and an ethylthio group; C1-6 alkylsulfinyl groups such as a methylsulfinyl group, and an ethylsulfinyl group; C1-6 alkylsulfonyl groups such as a methylsulfonyl group, and an ethylsulfonyl group; Cl-6 haloalkylthio groups such as a trifluoromethylthio group, and a 2,2,2-trifluoroethylthio group; C1-6 haloalkylsulfinyl groups such as a trifluoromethylsulfinyl group, and a 2,2,2-trifluoroethylsulfinyl group; C1-6 haloalkylsulfonyl groups such as a trifluoromethylsulfonyl group, and a 2,2,2-trifluoroethylsulfonyl group; halo-substituted, trifluoromethyl-substituted, or unsubstituted C6-10 aryl groups, such as a phenyl group, a 4-chlorophenyl group, and a 4-trifluoromethylphenyl group; halo-substituted, trifluoromethyl-substituted, or unsubstituted C6-10 aryloxy groups, such as a phenyloxy group, 4-chlorophenyloxy group, and a 4-trifluoromethylphenyloxy group; halo-substituted, C1-6 alkyl-substituted, trifluoromethyl-substituted or unsubstituted 5- or 6-membered heteroaryl groups (preferably an oxadiazolyl group); halo-substituted, C1-6 alkyl-substituted, trifluoromethyl-substituted or unsubstituted 5- or 6-membered heteroaryloxy groups (preferably a pyridyloxy group); S-C1-6 alkylsulfonimidoyl groups such as a S-methylsulfonimidoyl group; N-cyano-S-C1-6 alkylsulfonimidoyl groups such as a N-cyano-S-methylsulfonimidoyl group; a carboxyl group; C1-6 alkylcarbonyl groups such as an acetyl group, and a propionyl group; C1-6 alkoxycarbonyl groups such as a methoxycarbonyl group, an ethoxycarbonyl group, a n-propoxycarbonyl group, an i-propoxycarbonyl group, and a t-butoxycarbonyl group; C1-6 alkylcarbonylthio groups such as an acetylthio group; and a cyano group.

In the group of $R^jR^kN$-" which is one of the preferable substituents on the "C1-6 alkyl group", $R^j$ represents a C1-6 alkyl group or a C1-6 alkoxy group, $R^k$ represents a hydrogen atom or a C1-6 alkyl group, and $R^j$ and $R^k$ may be combined to form a C3-6 alkylene group.

Examples of the "C1-6 alkyl group" as $R^j$ and $R^k$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Examples of the "C1-6 alkoxy group" include a methoxy group, an ethoxy group, a n-propoxy group, an i-propoxy group, a n-butoxy group, a s-butoxy group, an i-butoxy group, and a t-butoxy group.

Examples of the "C3-6 alkylene group" formed by combining $R^j$ and $R^k$ include a trimethylene group, a tetramethylene group, and a pentamethylene group.

Examples of the group of $R^jR^kN$- include an amino group, a N-methylamino group, a N,N-dimethylamino group, a N-ethyl-N-methylamino group, a N-methoxy-N-methylamino group, and a pyrrolidin-1-yl group.

$R^j$ and $R^k$ in the "group of $R^jR^kN$-CO-" as one of the preferable substituents on the "C1-6 alkyl group" means the same groups as those mentioned above.

Examples of the group of $R^jR^kN$-CO- include a N-methylaminocarbonyl group, a N,N-dimethylaminocarbonyl group, a N-ethyl-N-methylaminocarbonyl group, a N-methoxy-N-methylaminocarbonyl group, and a pyrrolidine-1-carbonyl group.

$R^j$ and $R^k$ in the "group of $R^jR^kN$-$SO_2$-" as one of the preferable substituents on the "C1-6 alkyl group" means the same groups as those mentioned above.

Examples of the group of $R^jR^kN$-$SO_2$- include a N-methylaminosulfonyl group, a N,N-dimethylaminosulfonyl group, a N-ethyl-N-methylaminosulfonyl group, a N-methoxy-N-methylaminosulfonyl group, and a pyrrolidin-1-yl sulfonyl group.

In the "group of $R^mCONR^n$-" as one of the preferable substituents on the "C1-6 alkyl group", $R^m$ represents a C1-6 alkyl group, and $R^n$ represents a hydrogen atom or a C1-6 alkyl group.

Examples of the "C1-6 alkyl group" as $R^m$ and $R^n$ include the same groups as those specifically mentioned as $R^j$

and $R^k$.
Examples of the group of $R^mCONR^n$- include an acetamide group and a N-methylacetamide group.
$R^m$ and $R^n$ in the "group of $R^mSO_2NR^n$-" as one of the preferable substituents on the "C1-6 alkyl group" means the same groups as those mentioned above.
Examples of the group of $R^mSO_2NR^n$- include a methylsulfonamide group, and a N-methylmethylsulfonamide group.
In the "group of $R^pO$-N=$CR^q$-" as one of the preferable substituents on the "C1-6 alkyl group", $R^p$ represents a C1-6 alkyl group, and $R^q$ represents a C1-6 alkyl group.
Examples of the "C1-6 alkyl group" as $R^p$ and $R^q$ include the same groups as those specifically mentioned as $R^j$ and $R^k$.
Examples of the group of $R^pO$-N=$CR^q$- include a (methoxyimino)ethyl group, and an (ethoxyimino)ethyl group.
(3) Examples of the "C2-6 alkenyl group" as Y include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.
(4) Examples of the "C1-6 alkoxy group" as Y include a methoxy group, an ethoxy group, a n-propoxy group, a n-butoxy group, a n-pentyloxy group, a n-hexyloxy group, an i-propoxy group, an i-butoxy group, a s-butoxy group, a t-butoxy group, and an i-hexyloxy group.
(5) Examples of the "C1-6 alkylcarbonyl group" as Y include an acetyl group, and a propionyl group.
(6) Examples of the "C1-6 alkoxycarbonyl group" as Y include a methoxycarbonyl group, an ethoxycarbonyl group, a n-propoxycarbonyl group, an i-propoxycarbonyl group, and a t-butoxycarbonyl group.
(7) Examples of the "C1-6 alkylthio group" as Y include a methylthio group, an ethylthio group, a n-propylthio group, a n-butylthio group, a n-pentylthio group, a n-hexylthio group, and an i-propylthio group.
(8) Examples of the "C1-6 alkylsulfinyl group" as Y include a methylsulfinyl group, an ethylsulfinyl group, and a t-butylsulfinyl group.
(9) Examples of the "C1-6 alkylsulfonyl group" as Y include a methylsulfonyl group, an ethylsulfonyl group, and a t-butylsulfonyl group.
Preferable examples of a substituent on the "C2-6 alkenyl group", "C1-6 alkoxy group", "C1-6 alkylcarbonyl group", "C1-6 alkoxycarbonyl group", "C1-6 alkylthio group", "C1-6 alkylsulfinyl group", or "C1-6 alkylsulfonyl group" include: halogeno groups, such as a fluoro group, a chloro group, a bromo group, and an iodo group; and halo-substituted or unsubstituted C6-10 aryl group, such as a phenyl group and a 4-chlorophenyl group.
(10) Examples of the "C3-8 cycloalkyl group" as Y include a cyclopropyl group, a cyclobutyl group, a cyclopentyl group, a cyclohexyl group, and a cycloheptyl group.
(11) Examples of the "C6-10 arylcarbonyl group" as Y include a benzoyl group.
Preferable examples of a substituent on the "C3-8 cycloalkyl group", or "C6-10 arylcarbonyl group" include: halogeno groups such as a fluoro group, a chloro group, a bromo group, and an iodo group; C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group; C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; and a cyano group.
(12) In the "group of $R^aO$-N=$CR^b$-" as Y, $R^a$ represents a hydrogen atom or a substituted or unsubstituted CI-6 alkyl group, and $R^b$ represents a hydrogen atom or a substituted or unsubstituted C1-6 alkyl group.
Examples of the "C1-6 alkyl group" as $R^a$ and $R^b$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.
Examples of a substituent on the "C1-6 alkyl group" include halogeno groups, C1-6 alkoxy groups, C1-6 haloalkoxy groups, and a cyano group.
Examples of the group of $R^aO$-N=$CR^b$- include a (methoxyimino)methyl group, a (t-butoxyimino)methyl group, a (benzyloxyimino)methyl group, and a (2,2,2-trifluoroethoxyimino)methyl group.
(13) In the "group of $R^cCONR^d$-" as Y, $R^c$ represents a substituted or unsubstituted C1-6 alkyl group, a substituted or unsubstituted C1-6 alkoxy group, or a substituted or unsubstituted C6-10 aryl group, $R^d$ represents a hydrogen atom or a substituted or unsubstituted C1-6 alkyl group, and $R^c$ and $R^d$ may be combined to form a substituted or unsubstituted C3-6 alkylene group, a substituted or unsubstituted C2-6 alkyleneoxy group, or a substituted or unsubstituted C1-6 alkyleneoxy C1-6 alkylene group.
Examples of the "substituted or unsubstituted C1-6 alkyl group" as $R^c$ and $R^d$ include the same groups as those specifically mentioned as $R^a$ and $R^b$.

Examples of the "C1-6 alkoxy group" as $R^c$ include a methoxy group, an ethoxy group, a n-propoxy group, an i-propoxy group, a n-butoxy group, a s-butoxy group, an i-butoxy group, and a t-butoxy group.
Examples of a substituent on the "C1-6 alkoxy group" include halogeno groups, C1-6 alkoxy groups, C1-6 haloalkoxy groups, and a cyano group.
Examples of the "C6-10 aryl group" as $R^c$ include a phenyl group and a naphthyl group.
Examples of a substituent on the "C6-10 aryl group" include halogeno groups, C1-6 alkyl groups, C1-6 haloalkyl groups, C1-6 alkoxy groups, C1-6 haloalkoxy groups, and a cyano group.
Examples of the "C3-6 alkylene group" formed by combining $R^c$ and $R^d$ include a trimethylene group, a tetramethylene group, and a pentamethylene group.
Examples of the "C2-6 alkyleneoxy group" formed by combining $R^c$ and $R^d$ include a dimethyleneoxy group, a trimethyleneoxy group, and a tetramethyleneoxy group.
Examples of the "C1-6 alkyleneoxy C1-6 alkylene group" formed by combining $R^c$ and $R^d$ include a methyleneoxy methylene group, and a methyleneoxy dimethylene group.
Examples of a substituent on the "C3-6 alkylene group", "C2-6 alkyleneoxy group", or "C1-6 alkyleneoxy C1-6 alkylene group" include halogeno groups, and C1-6 alkyl groups.
(14) In the "group of $R^cCOCH_2NR^d$-" as Y, $R^c$ and $R^d$ mean the same groups as those mentioned above.
Examples of the group of $R^cCOCH_2NR^d$- include a (2-oxopropyl)amino group, and a N-methyl(2-oxopropyl)amino group.
(15) In the "group of $R^eSO_2NR^f$-" as Y, $R^e$ represents a substituted or unsubstituted C1-6 alkyl group, or a substituted or unsubstituted C6-10 aryl group, $R^f$ represents a hydrogen atom or a substituted or unsubstituted C1-6 alkyl group, and $R^e$ and $R^f$ may be combined to form a substituted or unsubstituted C3-6 alkylene group.
Examples of the "substituted or unsubstituted C1-6 alkyl group" or "substituted or unsubstituted C6-10 aryl group" as $R^e$ include the same groups as those specifically mentioned above.
Examples of the "substituted or unsubstituted C1-6 alkyl group" as $R^f$ include the same groups as those specifically mentioned above.
Examples of the "C3-6 alkylene group" formed by combining $R^e$ and $R^f$ include the same groups as those specifically mentioned above.
(16) In the "group of $R^gR^hN$-CO-" as Y, $R^g$ represents a substituted or unsubstituted C1-6 alkyl group, a substituted or unsubstituted C1-6 alkoxy group, a substituted or unsubstituted C6-10 aryl group, or a substituted or unsubstituted C1-6 alkylsulfonyl group, $R^h$ represents a hydrogen atom or a substituted or unsubstituted C1-6 alkyl group, and $R^g$ and $R^h$ may be combined to form a substituted or unsubstituted C3-6 alkylene group.
Examples of the "substituted or unsubstituted C1-6 alkyl group", "substituted or unsubstituted C1-6 alkoxy group", or "substituted or unsubstituted C6-10 aryl group", as $R^g$ include the same groups as those specifically mentioned above.
Examples of the "substituted or unsubstituted C1-6 alkyl group" as $R^h$ include the same groups as those specifically mentioned above.
Examples of the "C3-6 alkylene group" formed by combining $R^g$ and $R^h$ include the same groups as those specifically mentioned above.
Examples of the group of $R^gR^hN$-CO- include a N-methylaminocarbonyl group, a N-isopropylaminocarbonyl group, a N,N-dimethylaminocarbonyl group, a N-ethyl-N-methylaminocarbonyl group, a N-methoxy-N-methylaminocarbonyl group, a pyrrolidine-1-carbonyl group, a N-(4-chlorophenyl)aminocarbonyl group, and a N-(4-chlorophenylmethyl)aminocarbonyl group.
(17) In the "group of $R^gR^hN$-$SO_2$-" as Y, $R^g$ and $R^h$ mean the same groups as those mentioned above.

Examples of the group of $R^gR^hN$-$SO_2$- include a N-methylaminosulfonyl group, a N,N-dimethylaminosulfonyl group, a N-ethyl-N-methylaminosulfonyl group, a N-methoxy-N-methylaminosulfonyl group, and a pyrrolidin-1-yl sulfonyl group, a N-(4-chlorophenyl)aminosulfonyl group, and a N-(4-chlorophenylmethyl)aminosulfonyl group.

[Formula (II-b)]

In the formula (II-b), an arrow indicates a binding position.
In the formula (II-b), $p^1$ indicates the number of methylenes in parentheses and is 0 or 1, preferably 1, and $p^2$ indicates the number of methylenes in parentheses and is an integer of 0 to 2, preferably 1.
The cyclic amino group of formula (II-b) is represented by formula (II-b-1), when $p^1$ is 1 and $p^2$ is 1.

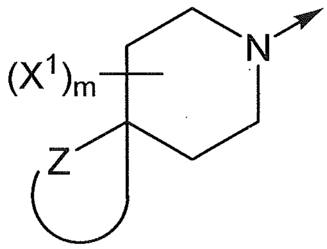

(II-b-1)

In the formula (II-b) or (II-b-1), $X^1$ indicates a substituent on the cyclic amino group, and is a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group, m indicates the number of $X^1$ and is an integer of 0 to 4 (preferably 0).

Examples of the "halogeno group", "substituted or unsubstituted C1-6 alkyl group", or "substituted or unsubstituted C1-6 alkylthio group" as $X^1$ include the same groups as those specifically mentioned as Ar.

Examples of the "C1-6 alkylsulfonyloxy group" as $X^1$ include a methylsulfonyloxy group, an ethylsulfonyloxy group, and a t-butylsulfonyloxy group.

Preferable examples of a substituent on the "C1-6 alkylsulfonyloxy group" include halogeno groups such as a fluoro group, a chloro group, a bromo group, and an iodo group.

In the formula (II-b) or (II-b-1), Z represents an oxo group, a group of $G^b$-O-N=, a substituted or unsubstituted C2-6 alkylene group, a group of -O-$G^a$-, a group of -O-$G^a$-O-, a group of -O-CO-$G^a$-, a group of -$G^a$-O-$G^a$-, a group of -N$G^b$-CO-$G^a$-, a group of -CO-N$G^b$-$G^a$-, or a group of -$G^a$-N$G^b$-CO-$G^a$-, $G^a$ each independently represents a substituted or unsubstituted C1-6 alkylene group, and $G^b$ each independently represents a substituted or unsubstituted C1-6 alkyl group.

Examples of the "C2-6 alkylene group" as Z include a dimethylene group, a trimethylene group, a tetramethylene group, and a pentamethylene group.

Examples of a substituent on the "C2-6 alkylene group" include: halogeno groups such as a fluoro group, a chloro group, a bromo group, and an iodo group; and C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group. Among these, the substituent is preferably a halogeno group.

Examples of the "C1-6 alkylene group" as $G^a$ include a methylene group, a dimethylene group, a trimethylene group, a tetramethylene group, and a pentamethylene group.

Examples of a substituent on the "C1-6 alkylene group" include: halogeno groups such as a fluoro group, a chloro group, a bromo group, and an iodo group; and C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group.

The "C1-6 alkyl group" as $G^b$ may be linear or branched. Examples of the alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Preferable examples of a substituent on the "C1-6 alkyl group" as $G^b$ include: halogeno groups such as a fluoro group, a chloro group, a bromo group, and an iodo group; a hydroxyl group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; and halo-substituted, C1-6 haloalkyl-substituted, C1-6 haloalkoxy-substituted or unsubstituted C6-10 aryl group, such as a phenyl group, a 4-chlorophenyl group, a 4-trifluorophenyl group, and a 4-trifluoromethoxyphenyl group.

[Formula (II-c)]

In the formula (II-c), an arrow indicates a binding position.

In the formula (II-c), $p^1$ indicates the number of methylenes in parentheses and is 0 or 1, preferably 1, and $p^2$ indicates the number of methylenes in parentheses and is an integer of 0 to 2, preferably 1.

The cyclic amino group of formula (II-c) is represented by formula (II-c-1), when $p^1$ is 1 and $p^2$ is 1.

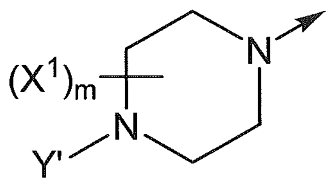

(II-c-1)

In the formula (II-c) or (II-c-1), $X^1$ indicates a substituent on the cyclic amino group, and is a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group, m indicates the number of $X^1$ and is an integer of 0 to 4.

Examples of the "halogeno group", "substituted or unsubstituted C1-6 alkyl group", or "substituted or unsubstituted C1-6 alkylthio group" as $X^1$ include the same groups as those specifically mentioned as Ar.

Examples of the "C1-6 alkylsulfonyloxy group" as $X^1$ include a methylsulfonyloxy group, an ethylsulfonyloxy group, and a t-butylsulfonyloxy group.

Preferable examples of a substituent on the "C1-6 alkylsulfonyloxy group" include halogeno groups such as a fluoro group, a chloro group, a bromo group, and an iodo group.

In the formula (II-c) or (II-c-1), Y' represents a substituted or unsubstituted C1-6 alkylcarbonyl group, a substituted or unsubstituted C1-6 alkoxycarbonyl group, a group of $R^{g'}R^hN$-CO-, a group of $R^{g'}R^hN$-CS-, a group of $R^{g'}R^hN$-CO-CO-, a substituted or unsubstituted C1-6 alkylsulfonyl group, or a group of $R^{g'}R^hN$-$SO_2$-,
$R^{g'}$ represents a substituted or unsubstituted C1-6 alkyl group, a substituted or unsubstituted C1-6 alkoxy group, or a substituted or unsubstituted C6-10 aryl group,
$R^h$ represents a hydrogen atom or a substituted or unsubstituted C1-6 alkyl group, and
$R^{g'}$ and $R^h$ may be combined to form a substituted or unsubstituted C3-6 alkylene group.

Examples of the "C1-6 alkylcarbonyl group" as Y' include an acetyl group, and a propionyl group.

Examples of the "C1-6 alkoxycarbonyl group" as Y' include a methoxycarbonyl group, an ethoxycarbonyl group, a n-propoxycarbonyl group, an i-propoxycarbonyl group, and a t-butoxycarbonyl group.

Examples of the "C1-6 alkylsulfonyl group" as Y' include a methylsulfonyl group, an ethylsulfonyl group, and a t-butylsulfonyl group.

Preferable examples of a substituent on the "C1-6 alkylcarbonyl group", "C1-6 alkoxycarbonyl group", or "C1-6 alkylsulfonyl group" as Y' include halogeno groups such as a fluoro group, a chloro group, a bromo group, and an iodo group.

In the "group of $R^{g'}R^hN$-CO-" as Y', $R^{g'}$ represents a substituted or unsubstituted C1-6 alkyl group, a substituted or unsubstituted C1-6 alkoxy group, or a substituted or unsubstituted C6-10 aryl group, $R^h$ represents a hydrogen atom or a substituted or unsubstituted C1-6 alkyl group, and $R^{g'}$ and $R^h$ may be combined to form a substituted or unsubstituted C3-6 alkylene group.

Examples of the "C1-6 alkyl group" as $R^{g'}$ and $R^h$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, 2-methylbutyl group, a 2,2-dimethylpropyl group, and an i-hexyl group.

Examples of a substituent on the "C1-6 alkyl group" include halogeno groups, C1-6 alkoxy groups, C1-6 haloalkoxy groups, and a cyano group.

Examples of the "C1-6 alkoxy group" as $R^{g'}$ include a methoxy group, an ethoxy group, a n-propoxy group, an i-propoxy group, a n-butoxy group, a s-butoxy group, an i-butoxy group, and a t-butoxy group.

Examples of a substituent on the "C1-6 alkoxy group" include halogeno groups, C1-6 alkoxy groups, C1-6 haloalkoxy groups, and a cyano group.

Examples of the "C6-10 aryl group" as $R^{g'}$ include a phenyl group, and a naphthyl group.

Examples of a substituent on the "C6-10 aryl group" include halogeno groups, C1-6 alkyl groups, C1-6 haloalkyl groups, C1-6 alkoxy groups, C1-6 haloalkoxy groups, and a cyano group.

Examples of the "C3-6 alkylene group" formed by combining $R^{g'}$ and $R^h$ include a trimethylene group, a tetramethylene group, and a pentamethylene group.

Examples of a substituent on the "C3-6 alkylene group" include halogeno groups such as a fluoro group, a chloro group, a bromo group, and an iodo group; and C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group.

Examples of the group of $R^{g'}R^hN$-CO- include a N-methylaminocarbonyl group, a N-isopropylaminocarbonyl group, a N,N-dimethylaminocarbonyl group, a N-ethyl-N-methylaminocarbonyl group, a N-methoxy-N-methylaminocarbonyl group, a pyrrolidine-1-carbonyl group, a N-(4-chlorophenyl)aminocarbonyl group, and a N-(4-chlorophenylmethyl)aminocarbonyl group.

In the "group of $R^{g'}R^hN$-CS-" as Y', $R^{g'}$ and $R^h$ means the same groups as those mentioned above.

Examples of the group of $R^{g'}R^hN$-CS- include: mono C1-6 alkylamino(thiocarbonyl) groups, such as a N-methylamino(thiocarbonyl) group, a N-ethylamino(thiocarbonyl) group, a N-n-propylamino(thiocarbonyl) group, a N-i-propylamino(thiocarbonyl) group, a N-t-butylamino(thiocarbonyl) group; and di C1-6 alkylamino(thiocarbonyl) groups such as a N,N-dimethylamino(thiocarbonyl) group.

In the "group of $R^{g'}R^hN$-CO-CO-" as Y', $R^{g'}$ and $R^h$ mean the same groups as those mentioned above.

Examples of the group of $R^{g'}R^hN$-CO-CO- include: mono C1-6 alkylaminooxalyl groups, such as a N-methylaminooxalyl group, a N-ethylaminooxalyl group, a N-n-propylaminooxalyl group, a N-i-propylaminooxalyl group, and a N-t-butylaminooxalyl group; and di C1-6 alkylaminooxalyl groups such as a N,N-dimethylaminooxalyl group.

**[0107]** - In the "group of $R^{g'}R^hN-SO_2$-" as Y', $R^{g'}$ and $R^h$ mean the same groups as those mentioned above.

Examples of the group of $R^{g'}R^hN-SO_2$- include a N-methylaminosulfonyl group, a N,N-dimethylaminosulfonyl group, a N-ethyl-N-methylaminosulfonyl group, a N-methoxy-N-methylaminosulfonyl group, a pyrrolidin-1-yl sulfonyl group, a N-(4-chlorophenyl)aminosulfonyl group, and a N-(4-chlorophenylmethyl)aminosulfonyl group.

Among these, it is preferable that Y' represent a substituted or unsubstituted Cl-6 alkoxycarbonyl group, a substituted or unsubstituted C1-6 alkylsulfonyl group, or a group of $R^{g'}R^hN-SO_2$-, $R^{g'}$ represent a substituted or unsubstituted C1-6 alkyl group, and $R^h$ represent a substituted or unsubstituted C1-6 alkyl group.

[Formula (I-a)]

As the cyclic amine compound according to the present invention, Ar in the formula (I) is preferably a substituted or unsubstituted phenyl group. As one preferable example of the cyclic amine compound according to the present invention, a compound of formula (I-a) is mentioned.

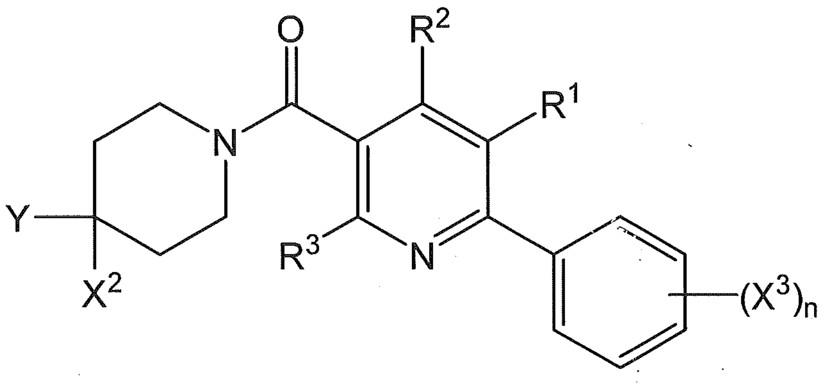

(I-a)

In the formula (I-a), $R^1$, $R^2$, $R^3$, $X^2$, and Y mean the same groups as those in the formula (I), respectively. $X^3$ indicates a substituent on a phenyl group, and n indicates the number of the substituent and is an integer of 0 to 5.

Examples of the substituent on the phenyl group include the same substituents as those mentioned as the "substituents on a C6-10 aryl group" for Ar mentioned above.

Salts of the compound (I) are not particularly limited in the case of agriculturally or horticulturally acceptable salts. Examples thereof include salts of inorganic acids such as a hydrochloric acid or a sulfuric acid; salts of organic acids such as an acetic acid or a lactic acid; salts of alkali metals such as lithium, sodium or potassium; salts of alkaline earth metals such as calcium or magnesium; salts of transition metals such as iron or copper; and salts of organic bases such as ammonia, triethylamine, tributylamine, pyridine, or hydrazine.

The compound (I) or the salts of the compound (I) are not particularly limited by the preparation process thereof. Salts of the compound (I) may be obtained from the compound (I) by known methods. For example, a compound (I) or a salt of the compound (I) according to the present invention may be obtained by the process described in Examples, or the like.

The cyclic amine compound according to the present invention (hereinafter, may be referred to as "compound according to the present invention") has excellent control effects against pests such as various agricultural insect pests and acarians that affect plant growth.

The compound according to the present invention is a highly safe compound as it has no phytotoxicity against crop plants and low toxicity to fish and warm-blooded animals. Thus, the compound according to the present invention is useful as an active ingredient of an insecticide or acaricide.

In addition, in recent years, resistance to various existing drugs has developed in many insect pests such as plutellidae, delphacidae, cicadellidae, and aphids, and the problem of the lack of efficacy of these drugs has arisen, and

it is desirable that drugs effective against insect pests in resistant strains are also desired. The compound according to the present invention has excellent control effects not only on susceptible strains but also on insect pests of various resistant strains and even acarians of acaricide-resistant strains.

The compound according to the present invention has excellent control effects against ectoparasites that cause harm to humans or livestock. In addition, the compound according to the present invention is a highly safe compound as it has low toxicity to fish and warm-blooded animals. Thus, the compound according to the present invention is useful as an active ingredient of an ectoparasite-control agent.

The compound according to the present invention shows efficacy in all developmental stages of organisms to be controlled, and exhibits excellent control effects against eggs, nymphs, larvae, pupae, and adults of acarians or insects, for example.

[Pest control agent, insecticide or acaricide]

A pest control agent, insecticide or acaricide according to the present invention contains at least one selected from the cyclic amine compounds according to the present invention, as an active ingredient thereof. The amount of the compound according to the present invention contained in the pest control agent, insecticide or acaricide according to the present invention is not particularly limited, provided that pest control effects are exhibited.

It is preferable that the pest control agent, insecticide or acaricide, according to the present invention be applied to cereals; vegetables; root vegetables; potatoes; flowers and ornamental plants; fruit-bearing trees, foliage plants; trees, such as tea, coffee, or cacao; feed crops; lawn grasses; or plants such as cotton.

In the application to plants, the pest control agent, insecticide or acaricide, according to the present invention may be applied to any portions, such as leaves, stems, stalks, flowers, buds, fruits, seeds, sprouts, roots, tubers, tuberous roots, shoots, or slips. The pest control agent, insecticide or acaricide, according to the present invention is not particularly limited by the species of the plant to be applied. Examples of the plant species include original species, varieties, improved varieties, cultivars, mutants, hybrid bodies, and gene recombinants (GMO).

The pest control agent according to the present invention may be used to control various agricultural insect pests and acarians by conducting seed treatment, foliage application, soil application, or submerged application.

Specific examples of agricultural insect pests and acarians to be controlled with the pest control agent according to the present invention are shown below.

(1) Butterflies and moths of the order Lepidoptera

(a) Moths belonging to the family Arctiidae, such as Hyphantria cunea, and Lemyra imparilis;
(b) Moths belonging to the family Bucculatricidae, such as Bucculatrix pyrivorella;
(c) Moths belonging to the family Carposinidae, such as Carposina sasakii;
(d) Moths belonging to the family Crambidae, such as Diaphania indica, and Diaphania nitidalis, of Diaphania spp.; Ostrinia furnacalis, Ostrinia nubilalis, and Ostrinia scapulalis, of Ostrinia spp.; and others such as Chilo suppressalis, Cnaphalocrocis medinalis, Conogethes punctiferalis, Diatraea grandiosella, Glyphodes pyloalis, Hellula undalis, and Parapediasia teterrella;
(e) Moths belonging to the family Gelechiidae, such as Helcystogramma triannulella, Pectinophora gossypiella, Phthorimaea operculella, and Sitotroga cerealella;
(f) Moths belonging to the family Geometridae, such as Ascotis selenaria;
(g) Moths belonging to the family Gracillariidae, such as Caloptilia theivora, Phyllocnistis citrella, and Phyllonorycter ringoniella;
(h) Butterflies belonging to the family Hesperiidae, such as Parnara guttata;
(i) Moths belonging to the family Lasiocampidae, such as Malacosoma neustria;
(j) Moths belonging to the family Lymantriidae, such as Lymantria dispar, and Lymantria monacha, of Lymantria spp.; and others such as Euproctis pseudoconspersa, and Orgyia thyellina;
(k) Moths belonging to the family Lyonetiidae, such as Lyonetia clerkella, and Lyonetia prunifoliella malinella, of Lyonetia spp.;
(l) Moths belonging to the family Noctuidae, such as Spodoptera depravata, Spodoptera eridania, Spodoptera exigua, Spodoptera frugiperda, Spodoptera littoralis, and Spodoptera litura, of Spodoptera spp.; Autographa gamma, and Autographa nigrisigna, of Autographa spp.; Agrotis ipsilon, and Agrotis segetum, of Agrotis spp.; Helicoverpa armigera, Helicoverpa assulta, and Helicoverpa zea, of Helicoverpa spp.; Heliothis armigera, and Heliothis virescens, of Heliothis spp.; and others such as Aedia leucomelas, Ctenoplusia agnata, Eudocima tyrannus, Mamestra brassicae, Mythimna separata, Naranga aenescens, Panolis japonica, Peridroma saucia, Pseudoplusia includens, and

Trichoplusia ni;
(m) Moths belonging to the family Nolidae, such as Earias insulana;
(n) Butterflies belonging to the family Pieridae, such as Pieris brassicae, and Pieris rapae crucivora, of Pieris spp.;
(o) Moths belonging to the family Plutellidae, such as Acrolepiopsis sapporensis, and Acrolepiopsis suzukiella, of Acrolepiopsis spp.; and others such as Plutella xylostella;
(p) Moths belonging to the family Pyralidae, such as Cadra cautella, Elasmopalpus lignosellus, Etiella zinckenella, and Galleria mellonella;
(q) Moths belonging to the family Sphingidae, such as Manduca quinquemaculata, and Manduca sexta, of Manduca spp.;
(r) Moths belonging to the family Stathmopodidae, such as Stathmopoda masinissa;
(s) Moths belonging to the family Tineidae, such as Tinea translucens;
(t) Moths belonging to the family Tortricidae, such as Adoxophyes honmai, and Adoxophyes orana, of Adoxophyes spp.; Archips breviplicanus, and Archips fuscocupreanus of Archips spp.; and others such as Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana, and Sparganothis pilleriana;
(u) Moths belonging to the family Yponomeutidae, such as Argyresthia conjugella.

(2) Insect pests of the order Thysanoptera

(a) Insect pests belonging to the family Phlaeothripidae, such as Ponticulothrips diospyrosi;
(b) Insect pests belonging to the family Thripidae, such as Frankliniella intonsa, and Frankliniella occidentalis, of Frankliniella spp.; Thrips palmi, and Thrips tabaci, of Thrips spp.; and others such as Heliothrips haemorrhoidalis, and Scirtothrips dorsalis.

(3) Insect pests of the order Hemiptera

(A) The infraorder Archaeorrhyncha

(a) Insect pests belonging to the family Delphacidae, such as Laodelphax striatella, Nilaparvata lugens, Perkinsiella saccharicida, and Sogatella furcifera.

(B) The infraorder Clypeorrhyncha

(a) Insect pests belonging to the family Cicadellidae, such as Empoasca fabae, Empoasca nipponica, Empoasca onukii, and Empoasca sakaii, of Empoasca spp.; and others such as Arboridia apicalis, Balclutha saltuella, Epiacanthus stramineus, Macrosteles striifrons, and Nephotettix cinctinceps.

(C) The infraorder Heteroptera

(a) Insect pests belonging to the family Alydidae, such as Riptortus clavatus;
(b) Insect pests belonging to the family Coreidae, such as Cletus punctiger, and Leptocorisa chinensis;
(c) Insect pests belonging to the family Lygaeidae, such as Blissus leucopterus, Cavelerius saccharivorus, and Togo hemipterus;
(d) Insect pests belonging to the family Miridae, such as Halticus insularis, Lygus lineolaris, Psuedatomoscelis seriatus, Stenodema sibiricum, Stenotus rubrovittatus, and Trigonotylus caelestialium;
(e) Insect pests belonging to the family Pentatomidae, such as Nezara antennata, and Nezara viridula, of Nezara spp.; Eysarcoris aeneus, Eysarcoris lewisi, and Eysarcoris ventralis, of Eysarcoris spp., and others such as Dolycoris baccarum, Eurydema rugosum, Glaucias subpunctatus, Halyomorpha halys, Piezodorus hybneri, Plautia crossota, and Scotinophora lurida;
(f) Insect pests belonging to the family Pyrrhocoridae, such as Dysdercus cingulatus;

(g) Insect pests belonging to the family Rhopalidae, such as Rhopalus msculatus;
(h) Insect pests belonging to the family Scutelleridae, such as Eurygaster integriceps);
(i) Insect pests belonging to the family Tingidae, such as Stephanitis nashi.

(D) The infraorder Sternorrhyncha

(a) Insect pests belonging to the family Adelgidae, such as Adelges laricis;
(b) Insect pests belonging to the family Aleyrodidae, such as Bemisia argentifolii, and Bemisia tabaci, of Bemisia spp.; and others such as Aleurocanthus spiniferus, Dialeurodes citri, and Trialeurodes vaporariorum;
(c) Insect pests belonging to the family Aphididae, such as Aphis craccivora, Aphis fabae, Aphis forbesi, Aphis gossypii, Aphis pomi, Aphis sambuci, and Aphis spiraecola, of Aphis spp.; Rhopalosiphum maidis, and Rhopalosiphum padi, of Rhopalosiphum spp.; Dysaphis plantaginea, and Dysaphis radicola, of Dysaphis spp.; Macrosiphum avenae, and Macrosiphum euphorbiae, of Macrosiphum spp.; Myzus cerasi, Myzus persicae, and Myzus varians, of Myzus spp.; and others such as Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion avenae, and Toxoptera aurantii;
(d) Insect pests belonging to the family Coccidae, such as Ceroplastes ceriferus, and Ceroplastes rubens, of Ceroplastes spp.;
(e) Insect pests belonging to the family Diaspididae, such as Pseudaulacaspis pentagona, and Pseudaulacaspis prunicola, of Pseudaulacaspis spp.; Unaspis euonymi, and Unaspis yanonensis, of Unaspis spp.; and others such as Aonidiella aurantii, Comstockaspis perniciosa, Fiorinia theae, and Pseudaonidia paeoniae;
(f) Insect pests belonging to the family Margarodidae, such as Drosicha corpulenta, and Icerya purchasi;
(g) Insect pests belonging to the family Phylloxeridae, such as Viteus vitifolii;
(h) Insect pests belonging to the family Pseudococcidae, such as Planococcus citri, and Planococcus kuraunhiae, of Planococcus spp.; and others such as Phenacoccus solani, and Pseudococcus comstocki;
(i) Insect pests belonging to the family Psyllidae, such as Psylla mali, and Psylla pyrisuga, of Psylla spp.; and other such as Diaphorina citri.

(4) Insect pests of the infraorder Polyphaga

(a) Insect pests belonging to the family Anobiidae, such as Lasioderma serricorne;
(b) Insect pests belonging to the family Attelabidae, such as Byctiscus betulae, and Rhynchites heros;
(c) Insect pests belonging to the family Bostrichidae, such as Lyctus brunneus;
(d) Insect pests belonging to the family Brentidae, such as Cylas formicarius;
(e) Insect pests belonging to the family Buprestidae, such as Agrilus sinuatus;
(f) Insect pests belonging to the family Cerambycidae, such as Anoplophora malasiaca, Monochamus alternatus, Psacothea hilaris, and Xylotrechus pyrrhoderus;
(g) Insect pests belonging to the family Chrysomelidae, such as Bruchus pisorum, and Bruchus rufimanus, of Bruchus spp.; Diabrotica barberi, Diabrotica undecimpunctata, and Diabrotica virgifera, of Diabrotica spp.; Phyllotreta nemorum, and Phyllotreta striolata, of Phyllotreta spp.; and others such as Aulacophora femoralis, Callosobruchus chinensis, Cassida nebulosa, Chaetocnema concinna, Leptinotarsa decemlineata, Oulema oryzae, and Psylliodes angusticollis;
(h) Insect pests belonging to the family Coccinellidae, such as Epilachna varivestis, and Epilachna vigintioctopunctata, of Epilachna spp.;
(i) Insect pests belonging to the family Curculionidae, such as Anthonomus grandis, and Anthonomus pomorum, of Anthonomus spp.; Sitophilus granarius, and Sitophilus zeamais, of Sitophilus spp.; and others such as Echinocnemus squameus, Euscepes postfasciatus, Hylobius abietis, Hypera postica, Lissohoptrus oryzophilus, Otiorhynchus sulcatus, Sitona lineatus, and Sphenophorus venatus;
(j) Insect pests belonging to the family Elateridae, such as Melanotus fortnumi, and Melanotus tamsuyensis, of Melanotus spp.;
(k) Insect pests belonging to the family Nitidulidae, such as Epuraea domina;
(l) Insect pests belonging to the family Scarabaeidae, such as Anomala cuprea, and Anomala rufocuprea, of Anomala spp.; and others such as Cetonia aurata, Gametis jucunda, Heptophylla picea, Melolontha melolontha, and Popillia

japonica;
(m) Insect pests belonging to the family Scolytidae, such as Ips typographus;
(n) Insect pests belonging to the family Staphylinidae, such as Paederus fuscipes;
(o) Insect pests belonging to the family Tenebrionidae, such as Tenebrio molitor, and Tribolium castaneum;
(p) Insect pests belonging to the family Trogossitidae, such as Tenebroides mauritanicus.

(5) Insect pests of the order Diptera

(A) The infraorder Brachycera

(a) Insect pests belonging to the family Agromyzidae, such as Liriomyza bryoniae, Liriomyza chinensis, Liriomyza sativae, and Liriomyza trifolii, of Liriomyza spp.; and others such as Chromatomyia horticola, and Agromyza oryzae;
(b) Insect pests belonging to the family Anthomyiidae, such as Delia platura, and Delia radicum, of Delia spp.; and others such as Pegomya cunicularia;
(c) Insect pests belonging to the family Drosophilidae, such as Drosophila melanogaster, and Drosophila suzukii, of Drosophila spp.;
(d) Insect pests belonging to the family Ephydridae, such as Hydrellia griseola;
(e) Insect pests belonging to the family Psilidae, such as Psila rosae;
(f) Insect pests belonging to the family Tephritidae, such as Bactrocera cucurbitae, and Bactrocera dorsalis, of Bactrocera spp.; Rhagoletis cerasi, and Rhagoletis pomonella, of Rhagoletis spp.; and others such as Ceratitis capitata, and Dacus oleae.

(B) The infraorder Nematocera

(a) Insect pests belonging to the family Cecidomyiidae, such as Asphondylia yushimai, Contarinia sorghicola, Mayetiola destructor, and Sitodiplosis mosellana.

(6) Insect pests of the order Orthoptera

(a) Insect pests belonging to the family Acrididae, such as Schistocerca americana, and Schistocerca gregaria, of Schistocerca spp.; and others such as Chortoicetes terminifera, Dociostaurus maroccanus, Locusta migratoria, Locustana pardalina, Nomadacris septemfasciata, and Oxya yezoensis;
(b) Insect pests belonging to the family Gryllidae, such as Acheta domestica, and Teleogryllus emma;
(c) Insect pests belonging to the family Gryllotalpidae, such as Gryllotalpa orientalis;
(d) Insect pests belonging to the family Tettigoniidae, such as Tachycines asynamorus.

(7) Acarians (Acari)

(A) Acaridida of the order Astigmata

(a) Acarians belonging to the family Acaridae, such as Rhizoglyphus echinopus, and Rhizoglyphus robini, of Rhizoglyphus spp.; Tyrophagus neiswanderi, Tyrophagus perniciosus, Tyrophagus putrescentiae, and Tyrophagus similis, of Tyrophagus spp.; and others such as Acarus siro, Aleuroglyphus ovatus, and Mycetoglyphus fungivorus;

(B) Actinedida of the order Prostigmata

(a) Acarians belonging to the family Tetranychidae, such as Bryobia praetiosa, and Bryobia rubrioculus, of Bryobia spp.; Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis, and Eotetranychus uncatus, of Eotetranychus spp.; Oligonychus hondoensis, Oligonychus ilicis, Oligonychus karamatus, Oligonychus mangiferus, Oligonychus orthius, Oligonychus perseae, Oligonychus pustulosus, Oligonychus shinkajii, and Oligonychus ununguis, of Oligonychus spp.; Panonychus citri, Panonychus mori, and Panonychus ulmi, of Panonychus spp.; Tetranychus cinnabarinus, Tetranychus evansi, Tetranychus kanzawai, Tetranychus ludeni, Tetranychus quercivorus, Tetranychus phaselus, Tetranychus urticae, and Tetranychus viennensis, of Tetranychus spp.; Aponychus corpuzae, and Aponychus firmianae, of Aponychus spp.; Sasanychus akitanus, and Sasanychus pusillus, of Sasanychus spp.; Shizotetranychus celarius, Shizotetranychus longus, Shizotetranychus miscanthi, Shizotetranychus recki, and Shizotetranychus schizopus, of Shizotetranychus spp.; and others such as Tetranychina harti, Tuckerella pavoniformis, and Yezonychus sapporensis;

(b) Acarians belonging to the family Tenuipalpidae, such as Brevipalpus lewisi, Brevipalpus obovatus, Brevipalpus phoenicis, Brevipalpus russulus, and Brevipalpus californicus, of Brevipalpus spp.; Tenuipalpus pacificus, and Tenuipalpus zhizhilashviliae, of Tenuipalpus spp.; and others such as Dolichotetranychus floridanus;

(c) Acarians belonging to the family Eriophyidae, such as Aceria diospyri, Aceria ficus, Aceria japonica, Aceria kuko, Aceria paradianthi, Aceria tiyingi, Aceria tulipae, and Aceria zoysiea, of Aceria spp.; Eriophyes chibaensis, and Eriophyes emarginatae, of Eriophyes spp.; Aculops lycopersici, and Aculops pelekassi, of Aculops spp.; Aculus fockeui, and Aculus schlechtendali, of Aculus spp.; and others such as Acaphylla theavagrans, Calacarus carinatus, Colomerus vitis, Calepitrimerus vitis, Epitrimerus pyri, Paraphytoptus kikus, Paracalacarus podocarpi, and Phyllocotruta citri;

(d) Acarians belonging to the family Transonemidae, such as Tarsonemus bilobatus, and Tarsonemus waitei, of Tarsonemus spp.; and others such as Phytonemus pallidus, and Polyphagotarsonemus latus;

(e) Acarians belonging to the family Penthaleidae, such as Penthaleus erythrocephalus, and Penthaleus major, of Penthaleus spp.

The pest control agent, insecticide or acaricide, according to the present invention, may further contain additional components different from the compound according to the present invention. Examples of the additional components include conventional carriers available to conduct formulation. Examples of other additional components include conventional fungicides, insecticides, acaricides, nematicides, soil pest control agents, plant regulatory agents, herbicides, synergists, fertilizers, soil improvement agents, and animal feeds. There is a case where synergistic effects are exhibited by formulating such additional components.

Specific examples of the insecticides, acaricides, nematicides, soil insect pest control agents, and anthelmintic agents which can be mixed or used with the pest control agent, insecticide or acaricide, according to the present invention are shown below.

(1) Acetylcholinesterase inhibitors:

(a) carbamates: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, fenothiocarb, MTMC, aldoxycarb, allyxycarb, aminocarb, bufencarb, chloethocarb, metam sodium, promecarb;

(b) Organophosphate-based: acephate, azamethiphos, azinphos-ethyl, azinphos-methyl, cadusafos, chloroethoxyfos, chlorfenvinphos, chlormephos, chlorpyrifos, chlorpyrifos-methyl, coumaphos, cyanophos, demeton-S-methyl, diazinon, dichlorvos / DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimphos, temephos, terbufos, tetrachlorvinphos, thiometon, triazophos, trichlorfon, vamidothion, bromophos-ethyl, carbophenothion, cyanofenphos, demeton-S methyl sulfone, dialifos, dichlofenthion, dioxabenzofos, etrimfos, fensulfothion, flupyrazofos, fonofos, formothion, phosmethylan, isazophos, iodofenphos, methacrifos, pirimiphos-ethyl, phosphocarb, propaphos, prothoate, sulprophos.

(2) GABAergic chloride ion channel antagonists: acetoprole, chlordene, endosulfan, ethiprole, fipronil, pyrafluoprole, pyriprole; camphlechlor, heptachlor, dienochlor.

(3) Sodium channel modulators: acrinathrin, d-cis-trans allethrin, d-trans allethrin, bifenthrin, bioallethrin, bioallethrin S-cyclopentyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomers], deltamethrin, empenthrin [(EZ)-(1R)-isomer], esfenvalerate, ethofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrum, resmethrin, silafluofen, tefluthrin, tetramethrin [(1R)-isomer], tralomethrin, transfluthrin; allethrin, pyrethrin, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, terallethrin.
(4) Nicotinic acetylcholine receptor agonist: acetamiprid, clothianidin, dinotefuran, imidacloprid, nitenpyram, nithiazine, thiacloprid, thiamethoxam, sulfoxaflor, nicotine, flupyradifurone.
(5) Nicotinic acetylcholine receptor allosteric modulators: spinetoram, spinosad.
(6) Chloride channel activators: abamectin, emamectin benzoate, lepimectin, milbemectin; ivermectin, selamectin, doramectin, eprinomectin, moxidectin, milbemycin, milbemycin oxime, nemadectin.
(7) Juvenile hormone-like substances: hydroprene, kinoprene, methoprene, fenoxycarb, pyriproxyfen; diofenolan, epofenonane, triprene.
(8) Other non-specific inhibitors: methyl bromide, chloropicrin, sulfuryl fluoride, borax, tartar emetic.
(9) Homoptera selective feeding inhibitors: flonicamid, pymetrozine, pyrifluquinazon.
(10) Acarian growth inhibitors: clofentezine, diflovidazin, hexythiazox, etoxazole.
(11) Midgut inner membrane disrupting agent derived from microorganisms: Bacillus thuringiensis subspecies Isuraerenshi, Bacillus sphaericus, Bacillus thuringiensis subsp. Aizawai, Bacillus thuringiensis subspecies Kurstaki, Bacillus thuringiensis subspecies Tenebrionis, Bt crop protein: Cry1Ab, Cry1Ac, Cry1Fa, Cry1A.105, Cry2Ab, Vip3A, mCry3A, Cry3Ab, Cry3Bb, Cry34Ab1/Cry35Ab1.
(12) Mitochondrial ATP biosynthetic enzyme inhibitors: diafenthiuron, azocyclotin, cyhexatin, fenbutatin oxide, propargite, tetradifon.
(13) Oxidative phosphorylation uncouplers: chlorfenapyr, sulfluramid, DNOC, binapacryl, dinobuton, dinocap.
(14) Nicotinic acetylcholine receptor channel blockers: bensultap, cartap hydrochloride, nereistoxin, thiosultap-sodium, thiocyclam.
(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, fluazuron.
(16) Diptera molting disrupting agents: cyromazine.
(17) Molting hormone receptor agonists: chromafenozide, halofenozide, methoxyfenozide, tebufenozide.
(18) Octopamine receptor agonists: amitraz, demiditraz, chlordimeform.
(19) Mitochondrial electron transport system complex III inhibitors: acequinocyl, fluacrypyrim, hydramethylnon, bifenazate.
(20) Mitochondrial electron transport system complex I inhibitors: fenazaquin, fenpyroximate, pyrimidifen, pyridaben, tebufenpyrad, tolfenpyrad, rotenone.
(21) Voltage-dependent sodium channel blockers: indoxacarb, metaflumizone.
(22) Acetyl CoA carboxylase inhibitors: spirodiclofen, spiromesifen, spirotetramat, spiropidion.
(23) Mitochondrial electron transport system complex IV inhibitors: aluminum phosphide, calcium phosphide, phosphine, zinc phosphide, cyanide.
(24) Mitochondrial electron transport system complex II inhibitors: cyenopyrafen, cyflumetofen, pyflubumide.
(25) Ryanodine receptor modulators: chlorantraniliprole, cyantraniliprole, flubendiamide, cyclaniliprole, tetraniliprole.
(26) Mixed function oxidase inhibitor compounds: piperonyl butoxide.
(27) Latrophilin receptor agonists: depsipeptide, cyclic depsipeptide, 24-membered cyclic depsipeptide, emodepside.
(28) Other agents (mechanism of which has not been known): azadirachtin, benzoximate, bromopropylate, cryolite, dicofol, pyridalyl; benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimin, fluphenazine, gossyplure, japonilure, metoxadiazone, petroleum, sodium oleate, tetrasul, triarathene, afidopyropen, flometoquin, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazole-3-yl]-2-(1H-1,2,4-triazol-1-yl) benzonitrile (CAS: 943137-49-3), broflanilide, triflumezopyrim, dicloromezotiaz, oxazosulfyl, other meta-diamides.
(29) Anthelmintic agents:

(a) Benzimidazole-based: fenbendazole, albendazole, triclabendazole, oxibendazole, mebendazole, oxfendazole, parbendazole, flubendazole; febantel, netobimin, thiophanate; thiabendazole, cambendazole;
(b) Salicylanilide-based: closantel, oxyclozanide, rafoxanide, niclosamide;
(c) Substituted phenol-based: nitroxinil, nitroscanate;
(d) Pyrimidine-based: pyrantel, morantel;
(e) Imidazothiazole-based: levamisole, tetramisole;
(f) Tetrahydropyrimidine-based: praziquantel, epsiprantel;
(g) Other anthelmintic agents: cyclodiene, ryania, clorsulon, metronidazole, demiditraz; piperazine, diethylcarbamazine, dichlorophene, monepantel, tribendimidine, amidantel; thiacetarsamide, melorsamine, arsenamide.

Specific examples of fungicides which may be mixed or used with the pest control agent, insecticide or acaricide according to the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, ofurace;
(b) Adenosine deaminase inhibitors: bupirimate, dimethirimol, ethirimol;
(c) DNA/RNA synthesis inhibitors: hymexazol, octhilinone;
(d) DNA topoisomerase II inhibitors: oxolinic acid.

(2) Mitotic inhibitors and cell division inhibitors:

(a) β- tubulin polymerization inhibitors: benomyl, carbendazim, chlorfenazole, fuberidazole, thiabendazole, thiophanate, thiophanate-methyl, diethofencarb, zoxamide, ethaboxam;
(b) Cell division inhibitors: pencycuron;
(c) Spectrin-like protein delocalization inhibitors: fluopicolide.

(3) Respiration inhibitors:

(a) Complex I NADH oxidation-reduction enzyme inhibitors: diflumetorimu, tolfenpyrad;
(b) Complex II succinate dehydrogenase inhibitors: benodanil, flutolanil, mepronil, isofetamide, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, pyraziflumid;
(c) Complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, mandestrobin;
(d) Complex III ubiquinol reductase Qi inhibitors: cyazofamid, amisulbrom;
(e) Oxidative phosphorylation uncoupling agenst: binapacryl, meptyldinocap, dinocap, fluazinam, ferimzone;
(f) Oxidative phosphorylation inhibitors (ATP synthase inhibitors): fentin acetate, fentin chloride, fentin hydroxide;
(g) ATP production inhibitors: silthiofam;
(h) Complex III: Qx (unknown) inhibitor of cytochrome bc1 (ubiquinone reductase): ametoctradin.

(4) Amino acid and protein synthesis inhibitors

(a) Methionine biosynthesis inhibitors: andoprim, cyprodinil, mepanipyrim, pyrimethanil;
(b) Protein synthesis inhibitors: blasticidin-S, kasugamycin, kasugamycin hydrochloride, streptomycin, oxytetracycline.

(5) Signal transduction inhibitors:

(a) Signal transduction inhibitors: quinoxyfen, proquinazid;
(b) Inhibitors of MAP / histidine kinase in osmotic signal transduction: fenpiclonil, fludioxonil; chlozolinate, iprodione, procymidone, vinclozolin.

(6) Lipids and cell membrane synthesis inhibitors:

(a) Inhibitors of Phospholipid biosynthesis, methyltransferase: edifenphos, iprobenfos, pyrazophos, isoprothiolane;
(b) Lipid peroxidation agent: biphenyl, chloroneb, dicloran, quintozene, tecnazene, tolclofos-methyl, etridiazole;
(c) Agents that act on cell membrane: iodocarb, propamocarb, propamocarb hydrochloride, propamocarb fosetylate, prothiocarb;
(d) Microorganisms that disrupt cell membrane of pathogens: Bacillus subtilis bacteria, Bacillus subtilis QST713 strain, Bacillus subtilis FZB24 strain, Bacillus subtilis MBI600 strain, Bacillus subtilis strain D747;
(e) Agents that disrupt cell membrane: extract of Melaleuca alternifolia (tea tree).

(7) Inhibitors of sterol biosynthesis in cell membrane:

(a) Inhibitors of demethylation at the C14 position in sterol biosynthesis: triforine, pyrifenox, pyrisoxazole, fenarimol, flurprimidol, nuarimol, imazalil, imazalil sulfate, oxpoconazole, pefurazoate, procloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, fluconazole, fluconazole-cis, hexaconazole, imibenconazole, ipconazole, metconazole, myclobutanil, penconazole, propiconazole, simeconazole, tebuconazole, tetraconazole, triadimefon, triadimenol, triticonazole, prothioconazole, voriconazole, mefentrifluconazole;
(b) Inhibitors of Δ14 reductase and Δ8→Δ7-isomerase in sterol biosynthesis: aldimorph, dodemorph, dodemorph acetate, fenpropimorph, tridemorph, fenpropidin, piperalin, spiroxamine;
(c) Inhibitors of 3-keto reductase in C4 demethylation in sterol biosynthesis: fenhexamid, fenpyrazamine;
(d) Inhibitors of squalene epoxidase in sterol biosynthesis: pyributicarb, naftifin, terbinafine.

(8) Cell wall synthesis inhibitors

(a) Trehalase inhibitor: validamycin;
(b) Chitin synthesis inhibitors: polyoxin, polyoxorim;
(c) Cellulose synthase inhibitors: dimethomorph, flumorph, pyrimorph, benthiavalicarb, iprovalicarb, tolprocarb, valifenalate, mandipropamid.

(9) Melanin biosynthesis inhibitors

(a) Melanin biosynthesis reductase inhibitors: fthalide, pyroquilon, tricyclazole;
(b) Melanin biosynthesis anhydrase inhibitors: carpropamid, diclocymet, fenoxanil.

(10) Host plant resistance inducer:

(a) Agents that act on salicylic acid synthesis pathway: acibenzolar-S-methyl;
(b) Others: probenazole, tiadinil, isotianil, laminarin, Giant Knotweed Extract.

(11) Agents, action of which is unclear: cymoxanil, fosetyl aluminum, phosphoric acid (phosphate), tecloftalam, triazoxide, flusulfamide, diclomezine, methasulfocarb, cyflufenamid, metrafenone, piriofenon, dodine, dodine free base, flutianil.
(12) Agents having multi-functional points: copper (copper salt), Bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine acetate, iminoctadine triacetate, iminoctadine trialbesilate, anilazine, dithianon, chinomethionate, fluoroimide.
(13) Other agents: DBEDC, fluoro folpet, bis (8-quinolinolato) copper (II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophene, difenzoquat, difenzoquat methyl sulfonate, flumetover, fosetyl calcium, fosetyl sodium, irumamycin, natamycin, ntrothal-isopropyl, oxamocarb, pyrrolnitrin, tebufloquin, tolnifanide, zarilamid, algophase, amicarthiazol, oxathiapiprolin, metiram zinc, benthiazole, trichlamide, uniconazole, oxyfenthiin, picarbutrazox, fenpicoxamid, dichlobentiazox, quinofumelin.

Specific examples of plant regulatory agents which can be mixed or used with the pest control agent, insecticide or acaricide, according to the present invention are shown below.
1-Methylcyclopropane, 2,3,5-triiodobenzoic acid, IAA, IBA, MCPA, 4-CPA, 5-aminolevulinic acid, 6-benzylaminopurine, abscisic acid, aviglycine hydrochloride, ancymidol, butralin, calcium carbonate, calcium chloride, calcium formate, calcium peroxide, lime sulfur, calcium sulfate, chlormequat chloride, chlorpropham, choline chloride, cloprop, cyanamide, cyclanilide, daminozide, decyl alcohol, dichlorprop, dikegulac, dimethipin, diquat, ethephon, ethychlozate, flumetralin, flurprimidol, forchlorfenuron, gibberellin A, gibberellin A3, hymexazol, inabenfide, isoprothiolane, kinetin, maleic acid hydrazide, mefluidide, mepiquat chloride, oxidation type glutathione, paclobutrazol, pendimethalin, prohexadione calcium, prohydrojasmon, pyraflufen-ethyl, sintofen, sodium 1-naphthalene acetate, sodium cyanate, streptomycin, thidiazuron, triapenthenol, tribufos, trinexapac-ethyl, uniconazole P, and 1-nathtylacetamide.

[Ectoparasite control or expellant agent]

An ectoparasite control or expellant agent according to the present invention contains at least one selected from the group consisting of the compounds according to the present invention, as an active ingredient thereof. The ectoparasite control or expellant agent according to the present invention is excellent in controlling ectoparasites that cause harm to humans or livestock.

The ectoparasites parasitize the host animals, especially parasitize the body or the skin of warm-blooded animals or fish. More specifically, the ectoparasites parasitize the back, armpit, underbelly, inner thigh or the like of the host animals and obtain nutritional sources such as blood or dandruff from the animals to live. Examples of the ectoparasites include acarians, lice, fleas, mosquitoes, stable flies, flesh flies, Japanese fishlouse (Argulus japonicas) and the like.

Examples of the host animals to be treated with the ectoparasite control or expellant agent according to the present invention include warm-blooded animals such as pet animals such as dog or cat; pet birds; farm animals such as cattle, horse, pig, and sheep; domestic fowls. Additional examples thereof include: fish such as salmon, trout, puffer, carp, and goldfish; and insects such as honey-bee, stag beetle, and unicorn beetle.

The ectoparasites live in and on the host animals, especially warm-blooded animals. More specifically, the ectoparasites parasitize the back, armpit, underbelly, inner thigh or the like of the host animals and obtain nutritional sources such as blood or dandruff from the animals to live.

The ectoparasite control or expellant agent according to the present invention may be applied by a known veterinary method (topical, oral, parenteral or subcutaneous administration). Examples of the method include: a method in which a tablet, capsule or feed mixed with the ectoparasite control or expellant agent are orally administered to the animals; a method in which an immersion liquid, suppository or injection (intramuscular, subcutaneous, intravenous, intraabdominal or the like) is administered to the animals; a method in which an oil-based or aqueous liquid preparation is topically administered by conducting spraying, pouring on, spotting on or the like; and a method in which the ectoparasite control agent is topically administered to the animals by attaching a collar, an ear tag or the like made by molding, into an appropriate form, a mixture obtained by kneading the ectoparasite control agent into a resin.

Specific examples of the ectoparasites which can be controlled or exterminated by the ectoparasite control or expellant agent according to the present invention are shown below.

(1) Acarians (Acari)

Acarians belonging to the family Dermanyssidae, acarians belonging to the family Macronyssidae, acarians belonging to the family Laelapidae, acarians belonging to the family Varroidae, acarians belonging to the family Argasidae, acarians belonging to the family Ixodidae, acarians belonging to the family Psoroptidae, acarians belonging to the family Sarcoptidae, acarians belonging to the family Knemidokoptidae, acarians belonging to the family Demodixidae, acarians belonging to the family Trombiculidae, and insect-parasitic acari such as Coleopterophagus berlesei.

(2) Order Phthiraptera

Lice belonging to the family Haematopinidae, lice belonging to the family Linognathidae, biting lice belonging to the family Menoponidae, biting lice belonging to the family Philopteridae, and biting lice belonging to the family Trichodectidae.

(3) Order Siphonaptera

Fleas belonging to the family Pulicidae, such as Ctenocephalides canis and Ctenocephalides felis of Ctenocephalides spp.; fleas belonging to the family Tungidae, fleas belonging to the family Ceratophyllidae, and fleas belonging to the family Leptopsyllidae.

(4) Order Hemiptera.

(5) Insect pests of the order Diptera

Mosquitoes belonging to the family Culicidae, black flies belonging to the family Simuliidae, punkie belonging to the family Ceratopogonidae, horseflies belonging to the family Tabanidae, flies belonging to the family Muscidae, tsetse flies belonging to the family Glossinidae, flesh flies belonging to the family Sarcophagidae, flies belonging to the family Hippoboscidae, flies belonging to the family Calliphoridae, and flies belonging to the family Oestridae.

[Control agent against other pests]

In addition, the compound according to the present invention exhibits an excellent effect of controlling insect pests that have a sting or venom that can harm humans or livestock, insect pests carrying various pathogens / pathogenic bacteria, or insect pests that impart a discomforting sensation to humans (such as toxic insect pests, sanitary insect pests, or unpleasant insect pests).
Specific examples thereof are shown below.

(1) Insect pests of the order Hymenoptera

Sawflies belonging to the family Argidae, wasps belonging to the family Cynipidae, sawflies belonging to the family Diprionidae, ants belonging to the family Formicidae, wasps belonging to the family Mutillidae, and wasps belonging to the family Vespidae.

(2) Other insect pests

Blattodea, teracarians, araneae, centipedes, millipedes, crustacea and cimex lectularius.

[Preparation formulation]

Formulation examples of the pest control agent, insecticide, acaricide, or ectoparasite control or expellant agent are shown below. The present invention is not limited to these formulation examples. The terms "part" and "%" in the formulation examples are indicated based on mass.
The agricultural and horticultural preparation formulations are shown below.

(Formulation example 1: wettable powders)

40 parts of a compound according to the present invention, 53 parts of diatomaceous earth, 4 parts of higher alcohol sulfuric acid ester, and 3 parts of alkyl naphthalene sulfonate were mixed uniformly, and then finely pulverized to obtain wettable powders containing 40% of the active ingredient.

(Formulation example 2: emulsion)

30 parts of a compound according to the present invention, 33 parts of xylene, 30 parts of dimethylformamide, and 7 parts of polyoxyethylene alkyl allyl ether were mixed and dissolved to obtain an emulsion containing 30% of the active ingredient.

(Formulation example 3: granules)

5 parts of a compound according to the present invention, 40 parts of talc, 38 parts of clay, 10 parts of bentonite, and 7 parts of sodium alkyl sulfate were mixed uniformly, and then finely pulverized, followed by conducting granulation to obtain a particle diameter of 0.5 to 1.0 mm, and thus granules containing 5% of the active ingredient were obtained.

(Formulation example 4 granules)

5 parts of a compound according to the present invention, 73 parts of clay, 20 parts of bentonite, 1 part of sodium dioctyl sulfosuccinate, and 1 part of potassium phosphate were mixed and then pulverized, followed by adding water thereto, and then kneading the mixture. Then, granulation and drying were conducted to obtain granules containing 5% by mass of the active ingredient.

(Formulation example 5: suspension)

10 parts of a compound according to the present invention, 4 parts of polyoxyethylene alkyl allyl ether, 2 parts of sodium polycarboxylate, 10 parts of glycerin, 0.2 parts of xanthan gum, and 73.8 parts of water were mixed, and then wet-pulverized until the particle size became 3 $\mu$m or less to obtain a suspension containing 10% of the active ingredient.
Preparation formulations of ectoparasite control agents are shown below.

(Formulation example 6: granules)

5 parts of a compound according to the present invention was dissolved in an organic solvent to obtain a solution. The solution was sprayed on 94 parts of kaolin and 1 part of white carbon, followed by evaporating the solvent under reduced pressure to obtain granules. The granules may be mixed with animal feed to be used.

(Formulation example 7: injection agent)

0.1 to 1 part of a compound according to the present invention and 99 to 99.9 parts of peanut oil were mixed uniformly, and then filter-sterilized using a sterilizing filter to obtain an injection agent.

(Formulation example 8: pour-on agent)

5 parts of a compound according to the present invention, 10 parts of a myristic acid ester and 85 parts of isopropanol were mixed uniformly to obtain a pour-on agent.

(Formulation example 9: spot-on agent)

10 to 15 parts of a compound according to the present invention, 10 parts of a palmitic acid ester and 75 to 80 parts of isopropanol were mixed uniformly to obtain a spot-on agent.

(Formulation example 10: spray agent)

1 part of a compound according to the present invention, 10 parts of propylene glycol and 89 parts of isopropanol were mixed uniformly to obtain a spray agent.

(Compound synthesis example)

The compound according to the present invention is explained further specifically by showing examples below. The present invention is not limited to the following examples.

At first, the compound according to the present invention in which Q is represented by the formula (II-a) is explained.

Example 1

Synthesis of 6-(Difluoromethyl)-5-(4-((ethylthio)methyl)piperidine-1-carbonyl)-2-(4-(trifluoromethyl)phenyl)nicotinonitrile (compound No. a-53)

(Step 1) Synthesis of ethyl 2-(ethoxymethylene)-4,4-difluoro-3-oxobutanoate

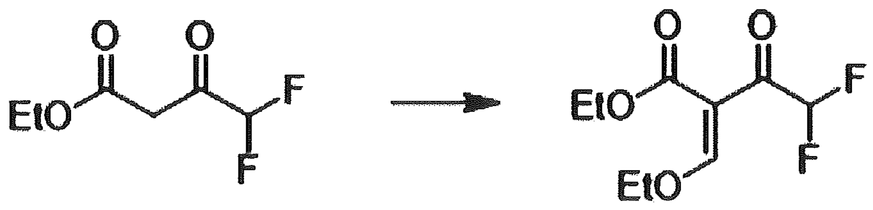

Ethyl 4,4-difluoroacetoacetate (8.3 g), triethyl orthoformate (14.8 g) and acetic anhydride (41.9 g) were mixed, and heated to reflux for 5 hours. Then, the resultant solution was concentrated under reduced pressure to obtain the target compound (11.1 g, at a yield of 100%). The NMR result of the target compound is shown below.

$^1$H-NMR ($CDCl_3$, $\delta$ ppm) 1.31 (3H, t), 1.43 (3H, t), 4.24-4.35 (4H, m), 6.20-6.55 (1H, m), 7.86 (1H, s).

(Step 2) Synthesis of ethyl 5-cyano-2-(difluoromethyl)-6-hydroxynicotinate

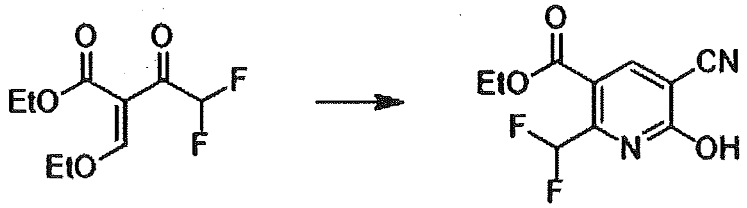

Sodium (1.27 g) was dissolved in ethanol (50 ml), and then cyanoacetic acid amide (4.62 g) was added thereto under ice-cooling, followed by adding ethyl 2-(ethoxymethylene)-4,4-difluoro-3-oxobutanoate (11.1 g) thereto. The resultant solution was stirred at room temperature overnight. Then, diluted hydrochloric acid was added thereto to adjust the pH to 1. The precipitate was filtered and then dried to obtain a target compound (9.94 g, at a yield of 82%). The NMR result of the target compound is shown below.
$^1$H-NMR ($CDCl_3$, δppm) 1.36 (3H, t), 4.34 (2H, q), 7.53 (1H, t), 8.39 (1H, s).

(Step 3) Synthesis of ethyl 6-chloro-5-cyano-2-(difluoromethyl)-nicotinate

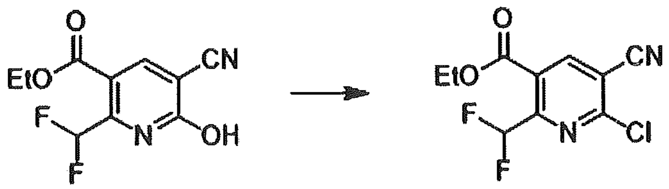

Ethyl 5-cyano-2-(difluoromethyl)-6-hydroxynicotinate (9.94 g) was suspended in dichloromethane (200 ml), and then N,N-dimethylformamide (0.75 g) and oxalyl chloride (26 g) were added thereto under ice-cooling. Then, the mixture was heated to reflux for 3 hours. The resultant solution was concentrated under reduced pressure, and then extracted with an ethyl acetate. The extracted phase was washed sequentially with a saturated aqueous sodium hydrogen carbonate solution and then with a saturated saline, followed by drying the resultant with anhydrous magnesium sulfate. The solvent was distilled off under reduced pressure, and the resultant residue was purified by silica gel column chromatography (eluent: n-hexane / ethyl acetate) to obtain the target compound (10.8 g, at a yield of 100%). The NMR result of the target compound is shown below.
$^1$H-NMR ($CDCl_3$, δ ppm) 1.43 (3H, t), 4.46 (2H, q), 7.39 (1H, t), 8.61 (1H, s).

(Step 4) Synthesis of ethyl 5-cyano-2-(difluoromethyl)-6-(4-(trifluoromethyl)phenyl) nicotinate

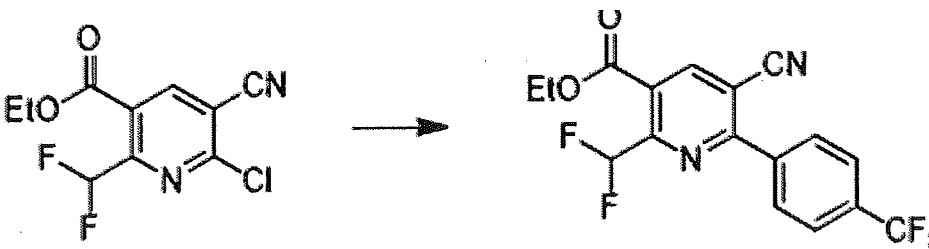

Ethyl 6-chloro-5-cyano-2-(difluoromethyl)-nicotinate (3.92 g) and 4-trifluoromethylphenylboronic acid (4.28 g) were dissolved in a mixture solvent composed of toluene (80 ml) and water (8 ml). Potassium carbonate (4.77 g) and dichlorobis[di-t-butyl(p-dimethylaminophenyl)phosphino]palladium (II) (0.53 g) were added thereto, and then the mixture was heated to reflux for 8 hours under nitrogen atmosphere. The resultant soulution was cooled to room temperature,

poured into water, and then extracted with an ethyl acetate. The extracted phase was washed with saturated saline, and then dried with anhydrous magnesium sulfate. The solvent was distilled off under reduced pressure, and the resultant residue was purified by silica gel column chromatography (eluent: n-hexane / ethyl acetate) to obtain the target compound (5.04 g, at a yield of 91%). The NMR result of the target compound is shown below.
$^1$H-NMR ($CDCl_3$, $\delta$ ppm) 1.41 (3H, t), 4.43 (2H, q), 6.91 (1H, t), 7.82 (2H, d), 8.09-8.11 (3H, m).

(Step 5) Synthesis of 5-cyano-2-(difluoromethyl)-6-(4-(trifluoromethyl)phenyl)nicotinate

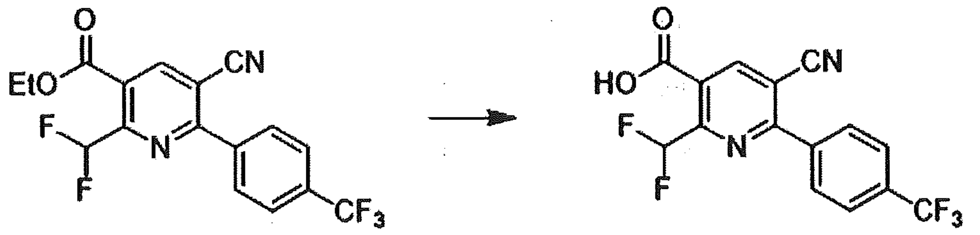

Ethyl 5-cyano-2-(difluoromethyl)-6-(4-(trifluoromethyl)phenyl)nicotinate (5.04 g) was dissolved in a mixture solvent composed of methanol (40 ml) and water (20 ml), and then sodium hydroxide (1.1 g) was added thereto, followed by stirring the mixture at room temperature for 2 hours. The resultant solution was concentrated under reduced pressure, poured into diluted hydrochloric acid, and then extracted with ethyl acetate. The extracted phase was washed with saturated saline, and then dried with anhydrous magnesium sulfate. The solvent was distilled off under reduced pressure to obtain the target compound (4.42 g, at a yield of 95%). The NMR result of the target compound is shown below.
$^1$H-NMR ($CDCl_3$, $\delta$ ppm) 6.95 (1H, t), 7.84 (2H, d), 8.10-8.13 (3H, m).

(Step 6) Synthesis of 6-(difluoromethyl)-5-(4-((ethylthio)methyl)piperidine-1-carbonyl)-2-(4-(trifluoromethyl)phenyl)nicotinonitrile

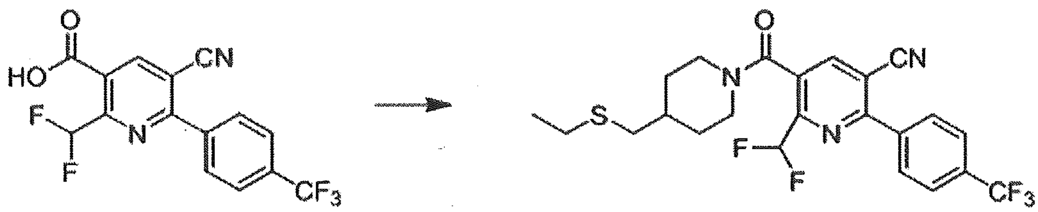

5-Cyano-2-(difluoromethyl)-6-(4-(trifluoromethyl)phenyl)nicotinate (0.4 g) was suspended in dichloromethane (20 ml), and then 4-((ethylthio)methyl)piperidine hydrochloride (0.34 g), 4-(N,N-dimethylamino)pyridine (0.29 g), and 1-[3-(diethylamino)propyl]-3-ethylcarbodiimide hydrochloride (0.45 g) were added thereto, followed by stirring the mixture at room temperature overnight. The resultant solution was poured into ice-water, and then extracted with chloroform. The extracted phase was washed sequentially with water, and then with saturated saline, followed by drying the resultant with anhydrous magnesium sulfate. The solvent was distilled off under reduced pressure, and the resultant residue was purified by silica gel column chromatography (eluent: n-hexane / ethyl acetate) to obtain the target compound (0.3 g, at a yield of 53%).

Example 2

Synthesis of 5-(4-((ethylthio)methyl)piperidine-1-carbonyl)-6-methyl-2-(4-(trifluoromethoxy)phenyl)pyridine-3-carbothioamide (compound No. b-1)

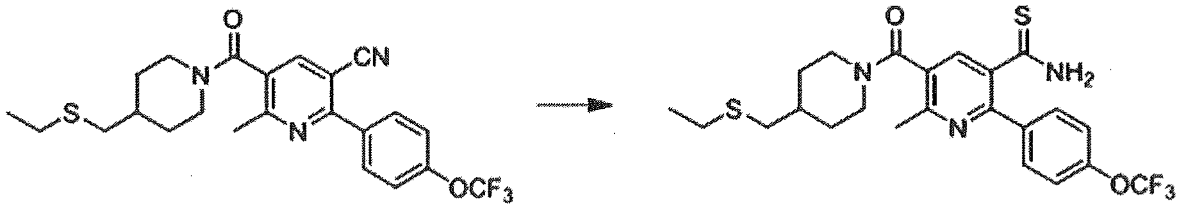

5-(4-((ethylthio)methyl)piperidine-1-carbonyl)-6-methyl-2-(4-(trifluoromethoxy) phenyl)nicotinonitrile (0.05 g) was suspended in N,N-dimethylformamide (1 ml), and then 70% sodium hydrosulfide (0.026 g) and magnesium chloride hexahydrate (0.033 g) were added thereto under ice-cooling, followed by stirring the mixture at room temperature for 5 hours. The resultant solution was poured into ice-water, and then extracted with ethyl acetate. The extracted phase was washed sequentially with water, and then with saturated saline, followed by drying the resultant with anhydrous magnesium sulfate. The solvent was distilled off under reduced pressure, and the resultant residue was purified by silica gel column chromatography (eluent: n-hexane / ethyl acetate) to obtain the target compound (0.04 g, at a yield of 75%). The NMR result of the target compound is shown below.

$^1$H-NMR ($CDCl_3$, δ ppm) 1.10-2.03 (m, 8H), 2.44-2.63 (m, 7H), 2.76-2.84 (m, 1H), 3.01-3.12 (m, 1H), 3.52-3.58 (m, 1H), 4.72-4.80 (m, 1H), 6.77 (brs, 1H), 7.29 (d, 1H), 7.54 (brs, 1H), 7.82 (d, 2H), 7.92 (s, 1H).

Some compounds according to the present invention, prepared in the same manner as the above-mentioned examples, are shown in Tables 1 to 6. Table 1 shows substituents of compounds of formula (I-a-1). n in the formula indicates the number of substituents (X3) on a benzene ring. Table 2 shows substituents of compounds of formula (I-a-2), Table 3 shows substituents of compounds of formula (I-a-3), Table 4 shows substituents of compounds of formula (I-a-4), Table 5 shows substituents of compounds of formula (I-a-5), and Table 6 shows substituents of compounds of formula (I-a-6).

The physical properties column of each table shows the properties, melting point (m.p.) or refractive index ($n_D$). In the tables, Ph represents a phenyl group, $^t$Bu represents a t-butyl group, Et represents an ethyl group, Me represents a methyl group, $^i$Pr represents an isopropyl group, $^c$Hex represents a cyclohexyl group, $^n$Bu represents a n-butyl group, $^n$Pr represents a n-propyl group, and $^c$Pr represents a cyclopropyl group. An arrow indicates a binding position.

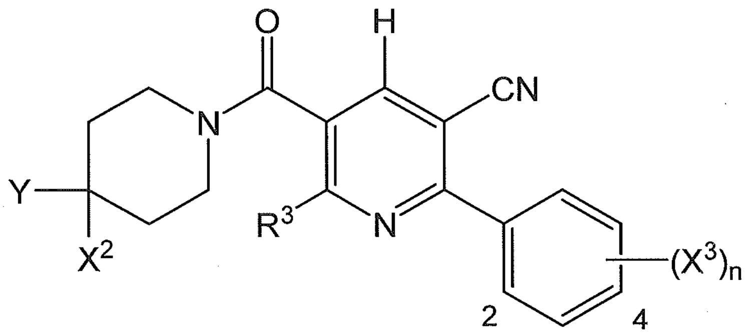

(I-a-1)

Table 1

| No. | Y | $X^2$ | $R^3$ | $(X^3)_n$ | Physical properties |
|---|---|---|---|---|---|
| a-1 | $PhCH_2$ | OH | $CF_2H$ | 4-$CF_3$ | AMORPHOUS |
| a-2 | $PhCH_2$ | H | $CF_2H$ | 4-$CF_3$ | m.p. 157-160°C |
| a-3 | $^t$Bu | H | $CF_2H$ | 4-$CF_3$ | VISCOUS OIL |
| a-4 | EtOC(=O) | H | $CF_2H$ | 4-$CF_3$ | VISCOUS OIL |
| a-5 | $^i$PrNHC(=O) | H | $CF_2H$ | 4-$CF_3$ | m.p. 200°C up |
| a-6 | 4-ClPhNHC(=O) | H | $CF_2H$ | 4-$CF_3$ | m.p. 188-190°C |
| a-7 | 4-ClPh$CH_2$NHC(=O) | H | $CF_2H$ | 4-$CF_3$ | m.p.191-193°C |
| a-8 | 4-FPhC(=O) | H | $CF_2H$ | 4-$CF_3$ | m.p. 174-176°C |

(continued)

| No. | Y | $X^2$ | $R^3$ | $(X^3)_n$ | Physical properties |
|---|---|---|---|---|---|
| a-9 | $PhCH_2CH_2CH_2$ | H | $CF_2H$ | 4-$CF_3$ | VISCOUS OIL |
| a-10 | $H_2NC(=S)$ | H | $CF_2H$ | 4-$CF_3$ | m.p. 189-191°C |
| a-11 | $^tBu$ | H | Me | 4-$CF_3$ | AMORPHOUS |
| a-12 | $CF_3$ | H | $CF_2H$ | 4-$CF_3$ | m.p. 78-80°C |
| a-13 | PhC(=O)NH | H | $CF_2H$ | 4-$CF_3$ | m.p. 220°C up |
| a-1 4 | $4\text{-}ClPhOCH_2$ | H | $CF_2H$ | 4-$CF_3$ | VISCOUS OIL |
| a-15 | MeO-N=CH | H | $CF_2H$ | 4-$CF_3$ | m.p. 148-149°C |
| a-16 | $^tBuO$-N=CH | H | $CF_2H$ | 4-$CF_3$ | m.p. 129-132°C |
| a-17 | $PhCH_2O$-N=CH | H | $CF_2H$ | 4-$CF_3$ | m.p. 60-62°C |
| a-18 | $CF_3CH_2O$-N=CH | H | $CF_2H$ | 4-$CF_3$ | m.p. 59-60°C |
| a-19 | $4\text{-}ClPhCH_2O$ | H | $CF_2H$ | 4-$CF_3$ | VISCOUS OIL |
| a-20 | $CF_3C(Cl)=CH$ | H | $CF_2H$ | 4-$CF_3$ | m.p. 180-182°C |
| a-21 | (3-Cl-5-$CF_3$-Pyridin-2-yl)$OCH_2$ | H | $CF_2H$ | 4-$CF_3$ | m.p. 114-116°C |
| a-22 | $^iPr$ | H | $CF_2H$ | 4-$CF_3$ | m.p. 132-135°C |
| a-23 | $HOC(Me)_2$ | H | $CF_2H$ | 4-$CF_3$ | VISCOUS OIL |
| a-24 | $^tBuOC(=O)N(Me)$ | H | $CF_2H$ | 4-$CF_3$ | m.p. 180-183°C |
| a-25 | MeC(=O)N(Me) | H | $CF_2H$ | 4-$CF_3$ | VISCOUS OIL |
| a-26 | 4-ClPhC(=O)N(Me) | H | $CF_2H$ | 4-$CF_3$ | VISCOUS OIL |
| a-27 | 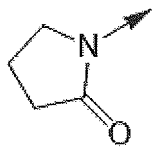 | H | $CF_2H$ | 4-$CF_3$ | m.p. 88-90°C |
| a-28 | MeC(=O) | H | $CF_2H$ | 4-$CF_3$ | m.p.58-60°C |
| a-29 | $^cHex$ | H | $CF_2H$ | 4-$CF_3$ | m.p. 155-156°C |
| a-30 | $^iPrO$ | H | $CF_2H$ | 4-$CF_3$ | m.p. 131-135°C |
| a-31 | EtO | H | $CF_2H$ | 4-$CF_3$ | m.p. 140-142°C |
| a-32 | 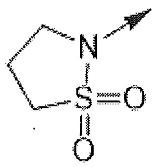 | H | $CF_2H$ | 4-$CF_3$ | m.p. 107-110°C |
| a-33 | 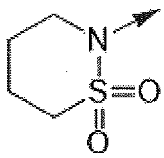 | H | $CF_2H$ | 4-$CF_3$ | VISCOUS OIL |
| a-34 | 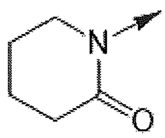 | H | $CF_2H$ | 4-$CF_3$ | m.p. 138-140°C |

(continued)

| No. | Y | $X^2$ | $R^3$ | $(X^3)_n$ | Physical properties |
|---|---|---|---|---|---|
| a-35 | 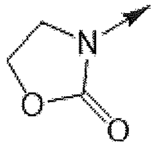 | H | $CF_2H$ | 4-$CF_3$ | VISCOUS OIL |

| No. | Y | $X^2$ | $R^3$ | $(X^3)_n$ | Physical properties |
|---|---|---|---|---|---|
| a-36 | 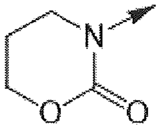 | H | $CF_2H$ | 4-$CF_3$ | VISCOUS OIL |
| a-37 | 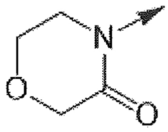 | H | $CF_2H$ | 4-$CF_3$ | m.p. 111-113°C |
| a-38 | 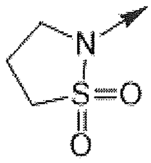 | H | Me | 4-$CF_3$ | m.p. 88-90°C |
| a-39 | 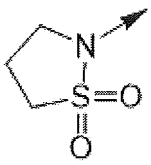 | H | $CF_3$ | 4-$CF_3$ | m.p. 167-168°C |
| a-40 | $MeSO_2N(Me)$ | H | $CF_2H$ | 4-$CF_3$ | m.p. 202-205°C |
| a-41 | $EtSO_2N(Me)$ | H | $CF_2H$ | 4-$CF_3$ | m.p. 120-122°C |
| a-42 | 4-$ClPhSO_2N(Me)$ | H | $CF_2H$ | 4-$CF_3$ | m.p. 151-155°C |
| a-43 | 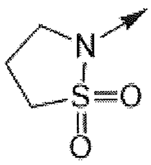 | H | $CF_3$ | 4-$OCF_3$ | m.p. 105-107°C |
| a-44 | 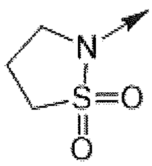 | H | Me | 4-$OCF_3$ | m.p. 98-100°C |
| a-45 | MeS | H | $CF_2H$ | 4-$CF_3$ | AMORPHOUS |
| a-46 | EtS | H | $CF_2H$ | 4-$CF_3$ | AMORPHOUS |
| a-47 | $^{i}PrS$ | H | $CF_2H$ | 4-$CF_3$ | AMORPHOUS |
| a-48 | $MeSO_2$ | H | $CF_2H$ | 4-$CF_3$ | m.p. 217-219°C |
| a-49 | $EtSO_2$ | H | $CF_2H$ | 4-$CF_3$ | m.p. 191-194°C |

(continued)

| No. | Y | $X^2$ | $R^3$ | $(X^3)_n$ | Physical properties |
|---|---|---|---|---|---|
| a-50 | $^iPrSO_2$ | H | $CF_2H$ | 4-$CF_3$ | m.p. 190-193°C |
| a-51 | $MeSCH_2$ | H | $CF_2H$ | 4-$CF_3$ | VISCOUS OIL |
| a-52 | $MeSO_2CH_2$ | H | $CF_2H$ | 4-$CF_3$ | m.p. 1 03-1 06°C |
| a-53 | $EtSCH_2$ | H | $CF_2H$ | 4-$CF_3$ | VISCOUS OIL |
| a-54 | $EtSO_2CH_2$ | H | $CF_2H$ | 4-$CF_3$ | m.p. 110-112°C |
| a-55 | 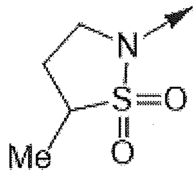 | H | $CF_2H$ | 4-$CF_3$ | m.p. 97-100°C |
| a-56 | $EtSCH_2$ | H | $CF_3$ | 4-$CF_3$ | m.p. 128-131°C |
| a-57 | $EtSCH_2$ | H | Me | 4-$OCF_3$ | AMORPHOUS |
| a-58 | $EtOCH_2$ | H | $CF_3$ | 4-$CF_3$ | m.p. 148-150°C |
| a-59 | N≡C-S | H | $CF_3$ | 4-$CF_3$ | m.p. 200-202°C |
| a-60 | $CF_3SCH_2$ | H | $CF_3$ | 4-$CF_3$ | m.p. 164-165°C |
| a-61 | $OF_3S(=O)CH_2$ | H | $CF_3$ | 4-$CF_3$ | m.p. 214-215°C |
| a-62 | $CF_3SO_2CH_2$ | H | $CF_3$ | 4-$CF_3$ | m.p. 192-194°C |
| a-63 | $CF_3S(=O)$ | H | $CF_3$ | 4-$CF_3$ | m.p. 72-74°C |
| a-64 | $CF_3SO_2$ | H | $CF_3$ | 4-$CF_3$ | m.p. 218-220°C |
| a-65 | $CF_3CF_2S$ | H | $CF_3$ | 4-$CF_3$ | m.p. 100-102°C |
| a-66 | $CF_3CF_2S(=O)$ | H | $CF_3$ | 4-$CF_3$ | m.p. 81-84°C |
| a-67 | C $F_3CF_2$S $O_2$ | H | $CF_3$ | 4-$CF_3$ | m.p. 198-201°C |
| a-68 | F | F | $CF_2H$ | 4-$CF_3$ | AMORPHOUS |
| a-69 | $EtSCH_2$ | H | Me | 4-$CF_3$ | m.p. 109-111°C |
| a-70 | $MeSCH_2CH_2$ | H | $CF_2H$ | 4-$CF_3$ | m.p. 151-154°C |
| a-71 | $MeS(=O)CH_2CH_2$ | H | $CF_2H$ | 4-$CF_3$ | AMORPHOUS |
| a-72 | $MeSO_2CH_2CH_2$ | H | $CF_2H$ | 4-$CF_3$ | AMORPHOUS |
| a-73 | $EtSCH_2CH_2$ | H | $CF_2H$ | 4-$CF_3$ | AMORPHOUS |
| a-74 | $EtSO_2CH_2CH_2$ | H | $CF_2H$ | 4-$CF_3$ | m.p. 138-140°C |
| a-75 | MeCHCl | H | $CF_3$ | 4-$CF_3$ | m.p. 177-179°C |
| a-76 | $CCl_2$=C H | H | $CF_3$ | 4-$CF_3$ | m.p. 175-176°C |
| a-77 | $CBr_2$=CH | H | $CF_3$ | 4-$CF_3$ | m.p. 190-191°C |
| a-78 | $EtS(=O)CH_2$ | H | $CF_3$ | 4-$CF_3$ | m.p. 90-92°C |
| a-79 | EtS $O_2$C $H_2$ | H | $CF_3$ | 4-$CF_3$ | m.p. 160-163°C |
| a-80 | $EtS(=O)(=NH)CH_2$ | H | $CF_3$ | 4-$CF_3$ | m.p. 92-94°C |
| a-81 | $EtS(=O)CH_2$ | H | Me | 4-$CF_3$ | AMORPHOUS |
| a-82 | EtS $O_2$C $H_2$ | H | Me | 4-$CF_3$ | AMORPHOUS |
| a-83 | EtSCHMe | H | $CF_3$ | 4-C $F_3$ | m.p. 119-121°C |

(continued)

| No. | Y | $X^2$ | $R^3$ | $(X^3)_n$ | Physical properties |
|---|---|---|---|---|---|
| a-84 | EtS(=O)(=NH)CHMe | H | $CF_3$ | 4-$CF_3$ | m.p. 134-136°C |
| a-85 | EtS(=O)CHMe | H | $CF_3$ | 4-$CF_3$ | m.p. 80-83°C |
| a-86 | EtS $O_2$CHMe | H | $CF_3$ | 4-$CF_3$ | m.p. 95-97°C |
| a-87 | $MeSCH_2$ | H | $CF_3$ | 4-$CF_3$ | m.p. 144-145°C |
| a-88 | MeS(=O)$CH_2$ | H | $CF_3$ | 4-$CF_3$ | m.p. 168-170°C |
| a-89 | $MeSO_2CH_2$ | H | $CF_3$ | 4-$CF_3$ | m.p. 211-212°C |
| a-90 | $MeSCH_2$ | H | Me | 4-$CF_3$ | m.p. 120-122°C |
| a-91 | MeS(=O)$CH_2$ | H | Me | 4-$CF_3$ | m.p. 143-145°C |
| a-92 | $MeSO_2CH_2$ | H | Me | 4-$CF_3$ | m.p. 200-201°C |
| a-93 | $EtSCH_2$ | H | Et | 4-$CF_3$ | m.p. 100-102°C |
| a-94 | $BrCH_2$ | H | Et | 4-$CF_3$ | m.p. 136-138°C |
| a-95 | $EtSCH_2$ | H | $CF_3$ | 4-(3-$CF_3$-1H-pyrazol-1-yl) | m.p. 82-84°C |
| a-96 | $BrCH_2$ | H | $CF_3$ | 4-(3-$CF_3$-1 H-pirazol-1-yl) | m.p. 90-93°C |
| a-97 | $BrCH_2$ | H | Me | 4-$CF_3$ | m.p. 166-167°C |
| a-98 | EtS(=O)(=N-CN)CHMe | H | $CF_3$ | 4-$CF_3$ | m.p. 180-182°C |
| a-99 | $^{n}Bu$ | H | $CF_3$ | 4-$CF_3$ | m.p. 167-169°C |
| a-100 | $EtSCH_2$ | H | $^{c}Pr$ | 4-$CF_3$ | m.p. 112-114°C |
| a-101 | $EtSCH_2$ | H | Ph | 4-$CF_3$ | m.p. 66-68°C |
| a-102 | $EtSCH_2$ | H | $CF_3$ | 4-$OCHF_2$ | AMORPHOUS |
| a-103 | $EtSCH_2$ | H | $CF_3$ | 2-F, 4-$CF_3$ | m.p. 123-124°C |
| a-104 | (Pyrrolidin-1-yl)$SO_2$ | H | $CF_3$ | 4-$CF_3$ | m.p. 200-203°C |
| a-105 | $Me_2NSO_2$ | H | $CF_3$ | 4-$CF_3$ | m.p. 114-116°C |
| a-106 | $^{t}BuSCH_2$ | H | Me | 4-$CF_3$ | m.p. 145-148°C |
| a-107 | $EtSCH_2$ | H | Me | 4-$OCHF_2$ | VISCOUS OIL |
| a-108 | $MeSO_2$NHC(=O) | H | $CF_3$ | 4-$CF_3$ | m.p. 137-140°C |
| a-109 | $MeSO_2$N(Me)C)(=O) | H | $CF_3$ | 4-$CF_3$ | m.p. 66-68°C |
| a-110 | NC-$CH_2$ | H | Me | 4-$CF_3$ | m.p. 72-75°C |
| a-111 | $^{t}BuSO_2CH_2$ | H | Me | 4-$CF_3$ | AMORPHOUS |
| a-112 | $^{i}PrSCH_2$ | H | Me | 4-$CF_3$ | m.p. 114-116°C |
| a-113 | $EtSCH_2$ | H | $^{n}Pr$ | 4-$CF_3$ | m.p. 103-105°C |
| a-114 | EtS C $H_2$ | H | $^{i}Pr$ | 4-$CF_3$ | m.p. 116-119°C |
| a-115 | $PhNHSO_2$ | H | $CF_3$ | 4-$CF_3$ | m.p. 130-133°C |
| a-116 | PhN(Me)$SO_2$ | H | $CF_3$ | 4-$CF_3$ | AMORPHOUS |
| a-117 | $Me_2$NC(=O)$CH_2$ | H | $CF_3$ | 4-$CF_3$ | m.p. 164-166°C |
| a-118 | $EtSCH_2$ | H | $CF_3$ | 4-$OCF_3$ | m.p. 130-134°C |
| a-119 | EtS C $H_2$ | H | $CH_2OMe$ | 4-$CF_3$ | VISCOUS OIL |
| a-120 | EtS(=O)$CH_2$ | H | $^{i}Pr$ | 4-$CF_3$ | m.p. 122-125°C |
| a-121 | EtS(=O)$CH_2$ | H | $^{n}Pr$ | 4-$CF_3$ | m.p. 143-146°C |

(continued)

| No. | Y | $X^2$ | $R^3$ | $(X^3)_n$ | Physical properties |
|---|---|---|---|---|---|
| a-122 | $^iPrS(=O)CH_2$ | H | Me | $4-CF_3$ | m.p. 87-90°C |
| a-123 | $^tBuS(=O)CH_2$ | H | Me | $4-CF_3$ | VISCOUS OIL |
| a-124 | $EtS(=O)CH_2$ | H | $CH_2OMe$ | $4-CF_3$ | VISCOUS OIL |
| a-125 | $EtS(-O)CH_2$ | H | $CF_3$ | $4-OCF_3$ | m.p. 158-160°C |
| a-126 | $EtSCH_2$ | H | Et | $4-OCHF_2$ | m.p. 100-101°C |
| a-127 | $EtS(=O)CH_2$ | H | Et | $4-OCHF_2$ | m.p. 60-62°C |
| a-128 | $EtS(=O)CH_2$ | H | $CF_3$ | $2-F, 4-CF_3$ | m.p. 143-144°C |
| a-129 | $MeO(=O)CH_2N(Me)$ | H | $CF_3$ | $4-CF_3$ | m.p. 85-88°C |
| a-130 | $EtC(=O)N(Me)$ | H | $CF_3$ | $4-CF_3$ | m.p. 132-135°C |
| a-131 | $MeC(=O)N(Me)CH_2$ | H | $CF_3$ | $4-CF_3$ | m.p. 58-60°C |
| a-132 | $EtSCH_2$ | H | $CH_2CH_2OMe$ | $4-CF_3$ | VISCOUS OIL |
| a-133 | $EtS(=O)CH_2$ | H | $CH_2CH_2OMe$ | $4-CF_3$ | VISCOUS OIL |
| a-134 | $EtSCH_2$ | H | $CF_3$ | $4-CH=C(Me)_2$ | m.p. 140-142°C |
| a-135 | $EtSCH_2$ | H | Me | $2-F, 4-OCF_3$ | AMORPHOUS |
| a-136 | $EtSCH_2$ | H | $CF_3$ | $2-F, 4-OCF_3$ | m.p. 140-142°C |
| a-137 | $EtSCH_2$ | H | $CF_3$ | $2-OMe, 4-CF_3$ | m.p. 58-61°C |
| a-138 | $EtSCH_2$ | H | Et | $4-(2,2-F_2-^cPr)$ | m.p. 44-46°C |
| a-139 | $EtS(=O)CH_2$ | H | Et | $4-(2,2-F_2-^cPr)$ | m.p. 61-64°C |
| a-140 | $MeC(=O)SCH_2$ | H | $CF_3$ | $4-CF_3$ | m.p. 166-167°C |
| a-141 | $EtS(=O)CH_2$ | H | $CF_3$ | $2-OMe, 4-CF_3$ | m.p. 100-103°C |
| a-142 | $EtSCH_2$ | H | Me | $2-F, 4-CF_3$ | m.p. 106-109°C |
| a-143 | $EtS(=O)CH_2$ | H | Me | $2-F, 4-CF_3$ | m.p. 67-70°C |
| a-144 | $EtSCH_2$ | H | Et | $2-F, 4-CF_3$ | AMORPHOUS |
| a-145 | $EtS(=O)CH_2$ | H | Et | $2-F, 4-CF_3$ | m.p. 67-70°C |
| a-146 | $EtSCH_2$ | H | $CH_2OMe$ | $2-F, 4-CF_3$ | AMORPHOUS |
| a-147 | $EtSCH_2$ | H | $CH_2OMe$ | $4-OCHF_2$ | AMORPHOUS |
| a-148 | $EtS(=O)CH_2$ | H | $CH_2OMe$ | $4-OCHF_2$ | m.p. 56-58°C |
| a-149 | $EtS(=O)CH_2$ | H | $CF_3$ | $4-CH=C(Me)_2$ | m.p. 41-44°C |
| a-150 | $EtS(=O)CH_2$ | H | Me | $4-OCHF_2$ | m.p. 58-61°C |
| a-151 | $EtS(=O)CH_2$ | H | $CF_3$ | $4-OCHF_2$ | m.p. 51-54°C |
| a-152 | $EtSCH_2$ | H | Et | $4-(2,2-Cl_2-^cPr)$ | m.p. 67-70°C |
| a-153 | $EtS(=O)CH_2$ | H | Et | $4-(2,2-Cl_2-^cPr)$ | AMORPHOUS |
| a-154 | $EtSCH_2$ | H | $CF_3$ | $4-OCH_2CF_2CF_3$ | AMORPHOUS |
| a-155 | $EtSCH_2$ | H | Me | $4-CHF_2$ | AMORPHOUS |
| a-156 | $EtSCH_2$ | H | Me | $4-CF_2CF_3$ | m.p. 56-59°C |
| a-157 | $EtSCH_2$ | H | Me | 4-CN | m.p. 76-79°C |
| a-158 | $EtS(=O)CH_2$ | H | Me | 4-CN | m.p. 52-55°C |
| a-159 | $EtSC H_2$ | H | Me | $2-Cl, 4-CF_3$ | m.p. 138-141°C |

(continued)

| No. | Y | $X^2$ | $R^3$ | $(X^3)_n$ | Physical properties |
|---|---|---|---|---|---|
| a-160 | $EtSCH_2$ | H | Me | 2-(2-Cl-4-CF3-Ph), 4-$CF_3$ | m.p. 66-69°C |
| a-161 | $MeSO_2N(Me)CH_2$ | H | $CF_3$ | 4-$CF_3$ | m.p. 104-106°C |
| a-162 | $EtOC(=O)CH_2$ | H | $CF_3$ | 4-$CF_3$ | m.p. 144-1 46°C |
| a-163 | MeO-N(Me)C(=O) | H | $CF_3$ | 4-$CF_3$ | m.p. 81-84°C |
| a-164 | MeO-N(Me)C(=O)$OH_2$ | H | $CF_3$ | 4-$CF_g$ | m.p. 76-78°C |
| a-165 | $EtN(Me)C(=O)CH_2$ | H | $CF_3$ | 4-$CF_3$ | m.p. 58-60°C |
| a-166 | (Pyrolidin-1-yl)C(=O)$CH_2$ | H | $CF_3$ | 4-$CF_3$ | m.p. 152-1 54°C |
| a-167 | $MeNHC(=O)CH_2$ | H | $CF_3$ | 4-$CF_3$ | m.p. 214-218°C |
| a-168 | (5-Me-1,2,4-oxadiazol -3-yl)$CH_2$ | H | $CF_3$ | 4-$CF_3$ | m.p. 141-143°C |
| a-169 | EtN(Me)C(=O) | H | $CF_3$ | 4-$CF_3$ | m.p. 100-103°C |
| a-170 | 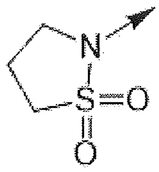 | H | Me | 4-$OCHF_2$ | m.p. 1 54-156°C |
| a-171 | $EtN(Me)CH_2$ | H | $CF_3$ | 4-$CF_3$ | m.p. 50-53°C |
| a-172 | $Me_2NSO_2CH_2$ | H | $CF_3$ | 4-$CF_3$ | m.p. 199-201°C |
| a-173 | $EtSCH_2$ | H | $CF_3$ | 4-(CH=N-OEt) | m.p. 54-57°C |
| a-174 | $EtS(=O)CH_2$ | H | $CF_3$ | 4-(CH=N-OEt) | m.p. 53-57°C |
| a-175 | $EtSCH_2$ | H | C $F_3$ | 4-Cl | m.p. 55-58°C |
| a-176 | $EtS(=O)CH_2$ | H | $CF_3$ | 4-Cl | m.p. 70-74°C |
| a-177 | $EtSCH_2$ | H | $CHF_2$ | 3-$CF_3$ | m.p. 47-50°C |
| a-178 | $EtSCH_2$ | H | $CF_3$ | 4-$CH_2CF_2H$ | m.p. 53-55°C |
| a-179 | $EtSCH_2$ | H | $CF_3$ | 4-$OCF_2CF_2H$ | m.p. 133-134°C |
| a-180 | $EtS(=O)CH_2$ | H | $CF_3$ | 4-$OCF_2CF_2H$ | m.p. 177-178°C |
| a-181 | $EtSCH_2$ | H | $CF_3$ | 4-$OCH_2CF_2CF_2H$ | m.p. 55-58°C |
| a-182 | $EtS(=O)CH_2$ | H | $CF_3$ | 4-$OCH_2CF_2CF_2H$ | m.p. 79-83°C |
| a-183 | 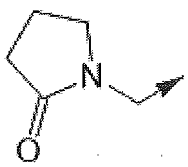 | H | $CF_3$ | 4-$CF_3$ | m.p. 226-228°C |
| a-184 | EtSC $H_2$ | H | Me | 2-OMe, 4-$CF_3$ | m.p. 67-70°C |
| a-185 | $HOC(=O)CH_2$ | H | $CF_3$ | 4-$CF_3$ | m.p 243-245°C |
| a-186 | $EtSCH_2$ | H | Me | 4-OH | m.p. 139-140°C |
| a-187 | $EtSCH_2$ | H | Me | 4-$OCH_2OMe$ | AMORPHOUS |
| a-188 | $ETSCH_2$ | H | Me | 4-SMe | m.p. 124-127°C |
| a-189 | $EtSCH_2$ | H | C $F_2OF_3$ | 4-$CF_3$ | m.p. 128-130°C |
| a-190 | $EtSCH_2$ | H | $CH_2SMe$ | 4-$CF_3$ | m.p. 54-56°C |

(continued)

| No. | Y | $X^2$ | $R^3$ | $(X^3)_n$ | Physical properties |
|---|---|---|---|---|---|
| a-191 | $EtSCH_2$ | H | $CH_2SO_2Me$ | 4-$CF_3$ | m.p. 76-79°C |
| a-192 | $EtSCH_2$ | H | OMe | 4-$CF_3$ | m.p. 137-138°C |
| a-193 | $EtSCH_2$ | H | $CH_2S(=O)Me$ | 4-$CF_3$ | m.p. 64-67°C |
| a-194 | $EtC(=O)CH_2$ | H | Me | 4-$CF_3$ | m.p. 108-110°C |
| a-195 | $EtC(=N\text{-}OEt)CH_2$ | H | Me | 4-$CF_3$ | AMORPHOUS |
| a-196 | EtSC $H_2$ | OH | Me | 4-$CF_3$ | m.p. 64-66°C |
| a-197 | $EtS(=O)CH_2$ | OH | Me | 4-$CF_3$ | m.p. 87-90°C |
| a-198 | $EtSO_2CH_2$ | OH | Me | 4-$CF_3$ | m.p. 198-200°C |
| a-199 | $EtSCH_2$ | OMe | Me | 4-$CF_3$ | m.p. 56-58°C |
| a-200 | $EtS(=O)CH_2$ | OMe | Me | 4-$CF_3$ | m.p. 40-43°C |
| a-201 | $EtSO_2CH_2$ | OMe | Me | 4-$CF_3$ | m.p. 70-72°C |
| a-202 | $EtCF_2CH_2$ | H | Me | 4-$CF_3$ | m.p. 131-132°C |
| a-203 | $EtSCH_2$ | OMe | Me | 4-$OCHF_5$ | m.p. 43-45°C |

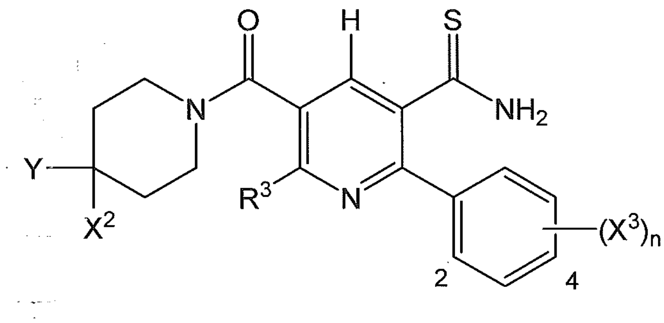

(I-a-2)

Table 2

| No . | Y | $X^2$ | $R^3$ | $(X^3)n$ | Physical properties |
|---|---|---|---|---|---|
| b-1 | $EtSCH_2$ | H | Me | 4-$OCF_3$ | m.p. 120-122°C |
| b-2 | $EtSCH_2$ | H | $CF_3$ | 4-$CF_3$ | m.p. 155-157°C |
| b-3 | $EtSCH_2$ | H | Me | 4-$CF_3$ | m.p. 225-226°C |
| b-4 | $EtS(=O)CH_2$ | H | $CF_3$ | 4-$CF_3$ | m.p. 254-257°C |
| b-5 | $EtSO_2CH_2$ | H | $CF_3$ | 4-$CF_3$ | m.p. 247-249°C |
| b-6 | $EtS(=O)CH_2$ | H | Me | 4-$CF_3$ | m.p. 105-108°C |
| b-7 | $EtSO_2CH_2$ | H | Me | 4-$CF_3$ | m.p. 127-130°C |
| b-8 | 4-ClPhC(=O)N(Me) | H | $CF_2H$ | 4-$CF_3$ | m.p. 264-265°C |
| b-9 | 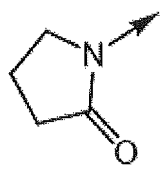 | H | $CF_2H$ | 4-$CF_3$ | m.p. 266-268°C |
| b-1 0 | $EtSO_2N(Me)$ | H | $CF_2H$ | 4-$CF_3$ | m.p. 224-226°C |

(continued)

| No . | Y | $X^2$ | $R^3$ | $(X^3)n$ | Physical properties |
|---|---|---|---|---|---|
| b-11 | 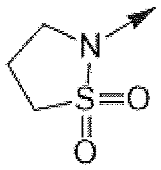 | H | Me | 4-$CF_3$ | m.p. 97-100°C |
| b-12 | 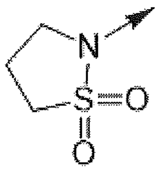 | H | $CF_3$ | 4-$CF_3$ | m.p. 270-271°C |
| b-13 | 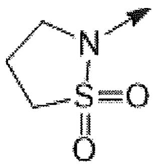 | H | Me | 4-$OCF_3$ | m.p. 150-152°C |
| b-14 | 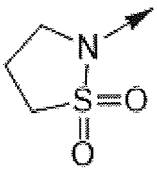 | H | $CF_3$ | 4-$OCF_3$ | m.p. 256-258°C |
| b-15 | EtSCHMe | H | $CF_3$ | 4-$CF_3$ | m.p. 147-150°C |
| b-16 | EtS(=O)CHMe | H | $CF_3$ | 4-$CF_3$ | m.p. 229-231°C |
| b-17 | $EtSO_2CHMe$ | H | $CF_3$ | 4-$CF_3$ | m.p. 235-238°C |
| b-18 | $MeSCH_2$ | H | $CF_3$ | 4-$CF_3$ | m.p. 123-125°C |
| b-19 | $MeS(=O)CH_2$ | H | $CF_3$ | 4-$CF_3$ | m.p. 246-249°C |
| b-20 | $MeSO_2CH_2$ | H | $CF_3$ | 4-$CF_3$ | m.p. 240-242°C |
| b-21 | $MeSCH_2$ | H | Me | 4-$CF_3$ | m.p. 219-221°C |
| b-22 | $MeS(=O)CH_2$ | H | Me | 4-$CF_3$ | m.p. 165-167°C |
| b-23 | $MeSO_2CH_2$ | H | Me | 4-$CF_3$ | m.p. 260-262°C |
| b-24 | $EtSCH_2$ | H | Me | 4-$SCF_3$ | m.p. 193-194°C |
| b-25 | $EtSCH_2$ | H | Et | 4-$CF_3$ | m.p. 147-150°C |
| b-26 | $EtS(=O)CH_2$ | H | Et | 4-$CF_3$ | m.p. 126-128°C |
| b-27 | $^tBuSCH_2$ | H | Me | 4-$CF_3$ | m.p. 211-213°C |
| b-28 | $EtSCH_2$ | H | $CF_3$ | 4-$OCHF_2$ | m.p. 90-93°C |
| b-29 | $EtSCH_2$ | H | Me | 4-$OCHF_2$ | m.p. 189-191°C |
| b-30 | $EtSCH_2$ | H | $CF_3$ | 2-F, 4-$CF_3$ | m.p. 135-138°C |
| b-31 | $EtSCH_2$ | H | $^cPr$ | 4-$CF_3$ | m.p. 150-153°C |
| b-32 | $EtSCH_2$ | H | Ph | 4-$CF_3$ | m.p. 125-128°C |
| b-33 | $^iPrSCH_2$ | H | Me | 4-$CF_3$ | m.p. 202-204°C |
| b-34 | $EtSCH_2$ | H | $^nPr$ | 4-$CF_3$ | m.p. 145-148°C |
| b-35 | $EtSCH_2$ | H | $^iPr$ | 4-$CF_3$ | m.p. 155-158°C |

(continued)

| No . | Y | $X^2$ | $R^3$ | $(X^3)n$ | Physical properties |
|---|---|---|---|---|---|
| b-36 | $EtSCH_2$ | H | $CF_3$ | 4-$OCF_3$ | m.p. 1 24-1 27°C |
| b-37 | $EtSCH_2$ | H | $CH_2OMe$ | 4-$CF_3$ | m.p. 1 87-190°C |
| b-38 | $^tBuS(=O)CH_2$ | H | Me | 4-$CF_3$ | m.p. 201-205°C |
| b-39 | $EtS(=O)CH_2$ | H | $CF_3$ | 4-$OCF_3$ | m.p. 202-205°C |
| b-40 | $EtSCH_2$ | H | Et | 4-$OCHF_2$ | m.p. 195-197°C |
| b-41 | $EtS(=O)CH_2$ | H | Et | 4-$OCHF_2$ | m.p. 80-82°C |
| b-42 | $EtS(=O)CH_2$ | H | $CF_3$ | 2-F, 4-$CF_3$ | m.p. 228-229°C |
| b-43 | $EtSCH_2$ | H | Me | 4-$CF_3$ | VISCOUS OIL |
| b-44 | $^iPrS(=O)CH_2$ | H | Me | 4-$CF_3$ | m.p. 161-165°C |
| b-45 | $EtS(=O)CH_2$ | H | $^nPr$ | 4-$CF_3$ | AMORPHOUS |
| b-46 | $EtS(=O)CH_2$ | H | $^iPr$ | 4-$CF_3$ | m.p. 231-235°C |
| b-47 | $EtS(=O)CH_2$ | H | $CH_2OMe$ | 4-$CF_3$ | AMORPHOUS |
| b-48 | $EtSCH_2$ | H | $CH_2CH_2OMe$ | 4-$CF_3$ | VISCOUS OIL |
| b-49 | $ETSCH_2$ | H | Me | 2-F, 4-$OCF_3$ | m.p. 208-209°C |
| b-50 | $EtSCH_2$ | H | $CF_3$ | 2-F, 4-$OCF_3$ | m.p. 91-93°C |
| b-51 | $EtSOH_2$ | H | Et | 4-(2,2-$F_2$-$^cPr$) | m.p. 100-102°C |
| b-52 | $EtS(=O)CH_2$ | H | Et | 4-(2,2-$F_2$-$^cPr$) | m.p. 108-110°C |
| b-53 | $EtSCH_2$ | H | $CF_3$ | 2-OMe, 4-$CF_3$ | m.p. 124-126°C |
| b-54 | $EtS(=O)CH_2$ | H | $CF_3$ | 2-OMe, 4-$CF_3$ | m.p. 210-211°C |
| b-55 | $EtSCH_2$ | H | Me | 2-F, 4-$CF_3$ | m.p. 222-223°C |
| b-56 | $EtS(=O)CH_2$ | H | Me | 2-F, 4-$CF_3$ | m.p. 159-163°C |
| b-57 | $EtSCH_2$ | H | Et | 2-F, 4-$CF_3$ | m.p. 204-206°C |
| b-58 | $EtS(=O)CH_2$ | H | Et | 2-F, 4-$CF_3$ | m.p. 196-197°C |
| b-59 | $EtSCH_2$ | H | $MeOCH_2$ | 2-F, 4-$CF_3$ | m.p. 157-159°C |
| b-60 | $EtSOH_2$ | H | $MeOCH_2$ | 4-$OCHF_2$ | m.p. 81-83°C |
| b-61 | $EtS(=O)CH_2$ | H | $MeOCH_2$ | 4-$OCHF_2$ | m.p. 117-120°C |
| b-62 | $ETSCH_2$ | H | $CF_3$ | 4-CH=C$(Me)_2$ | m.p. 108-111°C |
| b-63 | $EtS(=O)CH_2$ | H | $CF_3$ | 4-CH=C$(Me)_2$ | m.p. 122-125°C |
| b-64 | $EtS(=O)CH_2$ | H | Me | 4-$OCHF_2$ | m.p. 225-228°C |
| b-65 | $EtS(=O)CH_2$ | H | $CF_3$ | 4-$OCHF_2$ | m.p. 130-135°C |
| b-66 | EtC(=O)N(Me) | H | $CF_3$ | 4-$CF_3$ | m.p. 227-230°C |
| b-67 | $MeC(=O)N(Me)CH_2$ | H | $CF_3$ | 4-$CF_3$ | m.p. 237-238°C |
| b-68 | $Me_2NC(=O)CH_2$ | H | $CF_3$ | 4-$CF_3$ | m.p. 235-236°C |
| b-6 9 | $EtSCH_2$ | H | Et | 4-(2,2-$Cl_2$-$^cPr$) | m.p. 110-112°C |
| b-70 | $EtS(=O)CH_2$ | H | Et | 4-(2,2-$Cl_2$-$^cPr$) | m.p. 124-126°C |
| b-71 | $EtSCH_2$ | H | $CF_3$ | 4-$OCH_2CF_2CF_3$ | AMORPHOUS |
| b-72 | $EtSCH_2$ | H | Me | 4-$CHF_2$ | m.p. 214-216°C |
| b-73 | $EtSCH_2$ | H | Me | 4-$CF_2CF_3$ | m.p. 123-125°C |

(continued)

| No . | Y | $X^2$ | $R^3$ | $(X^3)n$ | Physical properties |
|---|---|---|---|---|---|
| b-74 | $EtSCH_2$ | H | Me | $4-C(=S)NH_2$ | m.p. 247-249°C |
| b-75 | $EtS(=O)CH_2$ | H | Me | $4-C(=S)NH_2$ | m.p. 183-185°C |
| b-76 | $EtSCH_2$ | H | Me | 2-Cl, $4-CF_3$ | m.p. 91-95°C |
| b-77 | $MeSO_2N(Me)CH_2$ | H | $CF_3$ | $4-CF_3$ | m.p. 226-228°C |
| b-78 | 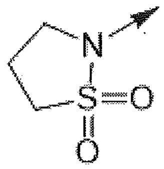 | H | Me | $4-OCHF_2$ | m.p. 224-226°C |
| b-79 | $Me_2NSO_2CH_2$ | H | $CF_3$ | $4-CF_3$ | m.p. 262-264°C |
| b-80 | $EtSCH_2$ | H | $CF_3$ | 4-(CH=N-OEt) | m.p. 102-104°C |
| b-81 | $EtS(=O)CH_2$ | H | C $F_3$ | 4-(CH=N-OEt) | m.p. 110-112°C |
| b-82 | $EtSOH_2$ | H | $CF_3$ | 4-Cl | m.p. 187-189°C |
| b-83 | $EtS(=O)CH_2$ | H | $CF_3$ | 4-Cl | m.p. 234-237°C |
| b-84 | $EtSCH_2$ | H | $CF_3$ | $4-OCF_2CF_2H$ | m.p. 100-103°C |
| b-85 | $EtS(=O)CH_2$ | H | $CF_3$ | $4-OCF_2CF_2H$ | m.p. 215-217°C |
| b-86 | $EtSCH_2$ | H | $CF_3$ | $4-OCH_2CF_2CF_2H$ | m.p. 224-226°C |
| b-87 | EtS(=O)C $H_2$ | H | $CF_3$ | $4-OCH_2CF_2CF_2H$ | m.p. 136-140°C |
| b-88 | $EtSCH_2$ | H | $CH_2SMe$ | $4-CF_3$ | m.p. 88-90°C |
| b-89 | $MeNHC(=O)CH_2$ | H | $CF_3$ | $4-CF_3$ | m.p. 178-180°C |
| b-90 | $EtSCH_2$ | H | $CF_2CF_3$ | $4-CF_3$ | m.p. 139-141°C |
| b-91 | $EtSCH_2$ | H | $CH_2SO_2Me$ | $4-CF_3$ | m.p. 125-127°C |
| b-92 | $EtSCH_2$ | H | OMe | $4-CF_3$ | m.p. 151-152°C |
| b-93 | $EtSCH_2$ | H | $CH_2S(-O)Me$ | $4-CF_3$ | m.p. 118-121°C |
| b-94 | $EtSCH_2$ | OMe | Me | $4-CF_3$ | m.p. 125-128°C |
| b-95 | $EtSCH_2$ | OMe | Me | $4-OCHF_2$ | m.p. 102-104°C |

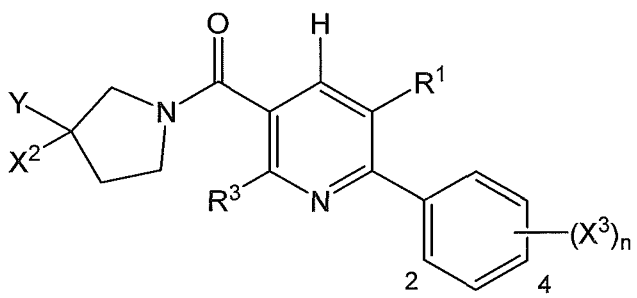

(I-a-3)

Table 3

| No. | Y | $X^2$ | $R^3$ | $R^1$ | $(X^3)n$ | Physical properties |
|---|---|---|---|---|---|---|
| c-1 | $EtSCH_2$ | H | Me | CN | $4-CF_3$ | m.p. 126-128°C |

(continued)

| No. | Y | $X^2$ | $R^3$ | $R^1$ | $(X^3)n$ | Physical properties |
|---|---|---|---|---|---|---|
| c-2 | $EtS(=O)CH_2$ | H | Me | CN | $4-CF_3$ | AMORPHOUS |
| c-3 | $EtSCH_2$ | H | Me | $C(=S)NH_2$ | $4-CF_3$ | m.p. 100-103°C |
| c-4 | $EtS(=O)CH_2$ | H | Me | $C(=S)NH_2$ | $4-CF_3$ | AMORPHOUS |
| c-5 | $EtSCH_2$ | H | Me | CN | $4-CF_3$ | m.p. 150-152°C |
| c-6 | $EtS(=O)CH_2$ | H | Me | CN | $4-CF_3$ | m.p. 97-98°C |
| c-7 | $EtS O_2C H_2$ | H | Me | CN | $4-CF_3$ | m.p. 163-164°C |

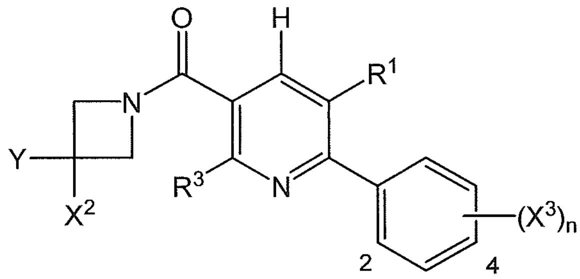

(I-a-4)

Table 4

| No . | Y | $X^2$ | $R^3$ | $R^1$ | $(X^3)n$ | Physical properties |
|---|---|---|---|---|---|---|
| d-1 | $EtSCH_2$ | H | Me | CN | $4-CF_3$ | AMORPHOUS |
| d-2 | $EtS(=O)CH_2$ | H | Me | CN | $4-CF_3$ | AMORPHOUS |
| d-3 | $EtSCH_2$ | H | Me | $C(=S)NH_2$ | $4-CF_3$ | m.p. 195-198°C |
| d-4 | EtS | H | Me | CN | $4-CF_3$ | m.p. 143-144°C |
| d-5 | EtS(=O) | H | Me | CN | $4-CF_3$ | m.p. 74-77°C |
| d-6 | $EtSO_2$ | H | Me | CN | $4-CF_3$ | m.p. 212-213°C |

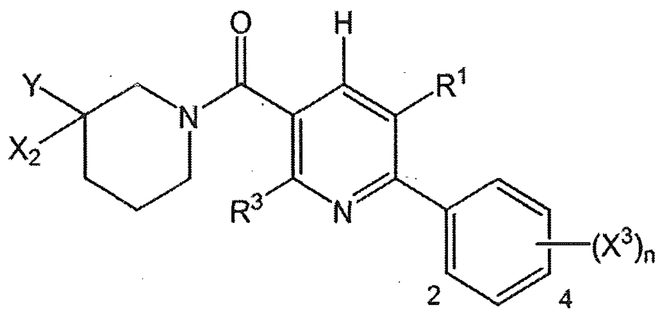

(I-a-5)

Table 5

| No. | Y | $X^2$ | $R^3$ | $R^1$ | $(X^3)n$ | Physical properties |
|---|---|---|---|---|---|---|
| e-1 | $EtSCH_2$ | H | $CF_3$ | CN | $4-CF_3$ | m.p. 130-133°C |
| e-2 | $EtSCH_2$ | H | $CF_3$ | $C(=S)NH_2$ | $4-CF_3$ | m.p. 122-124°C |
| e-3 | EtS | H | $C F_3$ | CN | $4-CF_3$ | m.p. 125-127°C |

(continued)

| No. | Y | $X^2$ | $R^3$ | $R^1$ | $(X^3)n$ | Physical properties |
|---|---|---|---|---|---|---|
| e-4 | EtS | H | $CF_3$ | $C(=S)NH_2$ | 4-$CF_3$ | m.p. 85-87°C |
| e-5 | $Me_2NC(=O)$ | H | Me | CN | 4-$CF_3$ | m.p. 97-99°C |
| e-6 | $Me_2NC(=O)CH_2$ | H | Me | CN | 4-$CF_3$ | m.p. 68-70°C |
| e-7 | MeS | H | Me | CN | 4-$CF_3$ | AMORPHOUS |
| e-8 | MeS(=O) | H | Me | CN | 4-$CF_3$ | m.p. 85-88°C |
| e-9 | $MeSO_2$ | H | Me | CN | 4-$CF_3$ | m.p. 92-95°C |
| e-10 | (5-Me-1,2,4-oxadiazol-3-yl)$CH_2$ | H | Me | CN | 4-$CF_3$ | m.p. 53-56°C |
| e-11 | $^nPrS$ | H | Me | CN | 4-$CF_3$ | AMORPHOUS |

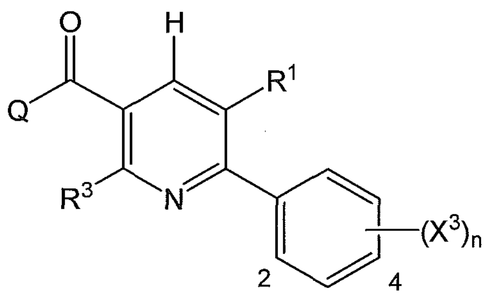

(I-a-6)

Table 6

| No. | Q | $R^1$ | $R^3$ | $(X^3)n$ | Physical properties |
|---|---|---|---|---|---|
| f-1 | 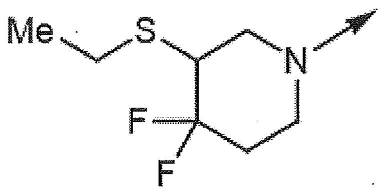 | CN | Me | 4-$CF_3$ | m.p. 57-60°C |
| f-2 | 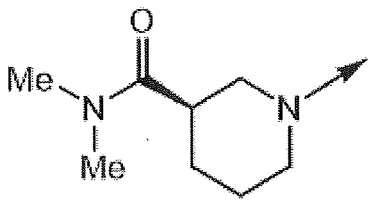 | CN | Me | 4-$CF_3$ | m.p. 77-80°C |
| f-3 | 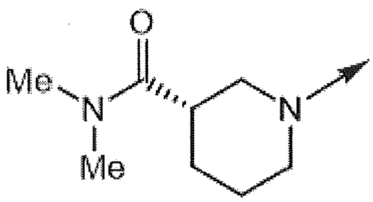 | CN | Me | 4-$CF_3$ | m.p. 77-80°C |
| f-4 | 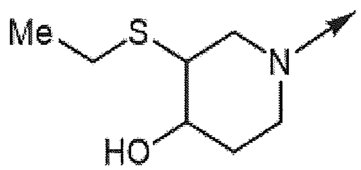 | CN | Me | 4-$CF_3$ | m.p. 57-60°C |

(continued)

| No. | Q | $R^1$ | $R^3$ | $(X^3)n$ | Physical properties |
|---|---|---|---|---|---|
| f-5 | 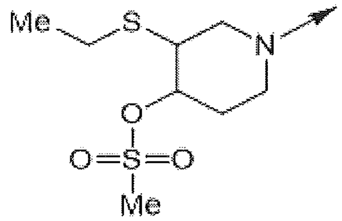 | CN | Me | 4-$CF_3$ | m.p. 77-80°C |
| f-6 | 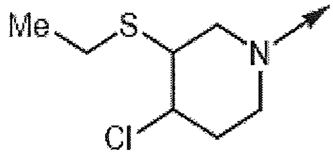 | CN | Me | 4-$CF_3$ | m.p. 50-53°C |

Among the compounds shown in Tables 1 to 5, the compounds having viscous oil properties or amorphous properties were subjected to $^1$H-NMR ($CDCl_3$) measurement. The resultant measurement values are shown below.

Compound No. (a-1): $^1$H-NMR (400 MHz, $CDCl_3$) $\delta$ ppm: 1.79-1.98 (m, 4H), 2.58-3.33 (m, 5H), 4.62-4.73 (m, 1H), 6.85 (t, 1H), 6.99-8.12 (m, 10H).

Compound No. (a-3): $^1$H-NMR (400 MHz, $CDCl_3$) $\delta$ ppm: 0.88 (s, 9H), 1.24-1.92 (m, 5H), 2.68-3.50 (m, 3H), 4.78-4.85 (m, 1H), 6.82 (t, 1H), 7.81 (d, 2H), 8.06 (s, 1H), 8.10 (d, 2H).

Compound No. (a-4): $^1$H-NMR (400 MHz, $CDCl_3$) $\delta$ ppm: 1.26 (t, 3H), 1.64-2.13 (m, 4H), 2.56-2.65 (m, 1H), 3.11-3.51 (m, 3H), 4.16 (q, 2H), 4.38-4.56 (m, 1H), 6.81 (t, 1H), 7.81 (d, 2H), 8.06 (s, 1H), 8.10 (d, 2H).

Compound No. (a-9): $^1$H-NMR (400 MHz, $CDCl_3$) $\delta$ ppm: 1.09-1.92 (m, 9H), 2.61 (t, 2H), 2.77-3.45 (m, 3H), 4.67-4.74 (m, 1H), 6.82 (t, 1H), 7.15-8.11 (m, 10H).

Compound No. (a-11): $^1$H-NMR (400 MHz, $CDCl_3$) $\delta$ ppm: 0.89 (s, 9H), 1.24-2.19 (m, 5H), 2.59-2.75 (m, 4H), 3.00-3.12 (m, 1H), 3.43-3.51 (m, 1H), 4.81-4.89 (m, 1H), 7.77-8.05 (m, 5H).

Compound No. (a-14): $^1$H-NMR (400 MHz, $CDCl_3$) $\delta$ ppm: 1.22-2.16 (m, 5H), 2.85-3.55 (m, 3H), 3.75-3.89 (m, 2H), 4.77-4.85 (m, 1H), 6.69-6.99 (m, 3H), 7.23 (d, 2H), 7.82 (d, 2H), 8.07 (s, 1H), 8.11 (d, 2H).

Compound No. (a-19): $^1$H-NMR (400 MHz, $CDCl_3$) $\delta$ ppm: 1.63-2.02 (m, 4H), 3.11-4.05 (m, 5H), 4.53 (q, 2H), 6.82 (t, 1H), 7.25-7.36 (m, 4H), 7.81 (d, 2H), 8.06 (s, 1H), 8.10 (d, 2H).

Compound No. (a-23): $^1$H-NMR (400 MHz, $CDCl_3$) $\delta$ ppm: 1.19-1.93 (m, 11H), 2.76-3.56 (m, 3H), 4.82-4.89 (m, 1H), 6.82 (t, 1H), 7.81 (d, 2H), 8.07 (s, 1H), 8.10 (d, 2H).

Compound No. (a-25): $^1$H-NMR (400 MHz, $CDCl_3$) $\delta$ ppm: 1.58-2.16 (m, 7H), 2.82-2.95 (m, 3H), 3.19-3.55 (m, 2H), 4.74-4.92 (m, 2H), 6.82 (t, 1H), 7.82 (d, 2H), 8.07-8.12 (m, 3H).

Compound No. (a-26): $^1$H-NMR (400 MHz, $CDCl_3$) $\delta$ ppm: 1.57-2.05 (m, 4H), 2.80-3.59 (m, 5H), 4.72-4.95 (m, 2H), 6.85 (t, 1H), 7.31-7.41 (m, 4H), 7.82 (d, 2H), 8.10-8.12 (m, 3H).

Compound No. (a-33): $^1$H-NMR (400 MHz, $CDCl_3$) $\delta$ ppm: 1.55-2.15 (m, 8H), 2.86-2.54 (m, 7H), 4.18-4.25 (m, 1H), 4.82-4.88 (m, 1H), 6.85 (t,1H), 7.82 (d,2H), 8.06 (s,1H), 8.10 (d,2H).

Compound No. (a-35): $^1$H-NMR (400 MHz, $CDCl_3$) $\delta$ ppm: 1.59-2.02 (m, 4H), 2.86-2.94 (m, 1H), 3.19-3.61 (m, 4H), 3.96-4.05 (m, 1H), 4.31-4.39 (m, 2H), 4.86-4.92 (m, 1H), 6.82 (t, 1H), 7.82 (d, 2H), 8.08-8.11 (m, 3H).

Compound No. (a-36): $^1$H-NMR (400 MHz, $CDCl_3$) $\delta$ ppm: 1.58-2.05 (m, 6H), 2.85-2.92 (m, 1H), 3.18-3.56 (m, 4H), 4.22-4.26 (m, 2H), 4.39-4.54 (m, 1H), 4.86-4.90 (m, 1H), 6.86 (t, 1H), 7.82 (d, 2H), 8.07-8.12 (m, 3H).

Compound No. (a-45): $^1$H-NMR (400 MHz, $CDCl_3$) $\delta$ ppm: 1.53-2.12 (m, 7H), 2.83-3.54 (m, 4H), 4.30-4.52 (m, 1H), 6.81 (t, 1H), 7.81 (d, 2H), 8.06 (s, 1H), 8.10 (d, 2H).

Compound No. (a-46): $^1$H-NMR (400 MHz, $CDCl_3$) $\delta$ ppm: 1.26 (t, 3H), 1.54-2.13 (m, 4H), 2.58 (q, 2H), 2.92-3.54 (m, 4H), 4.25-4.52 (m, 1H), 6.81 (t, 1H), 7.80-8.11 (m, 5H).

Compound No. (a-47): $^1$H-NMR (400 MHz, $CDCl_3$) $\delta$ ppm: 1.26-1.29 (m, 6H), 1.53-2.14 (m, 4H), 2.95-3.52 (m, 5H), 4.20-4.46 (m, 1H), 6.81 (t, 1H), 7.81 (d, 2H), 8.05 (s, 1H), 8.10 (d, 2H).

Compound No. (a-51): $^1$H-NMR (400 MHz, $CDCl_3$) $\delta$ ppm: 1.22-2.10 (m, 5H), 2.11 (s, 3H), 2.43-2.51 (m, 2H), 2.81-2.90 (m, 1H), 3.09-3.17 (m, 1H), 3.40-3.51 (m, 1H), 4.71-4.82 (m, 1H), 6.82 (t, 1H), 7.81 (d, 2H), 8.06 (s, 1H), 8.11 (d, 2H).

Compound No. (a-53): $^1$H-NMR (400 MHz, $CDCl_3$) $\delta$ ppm: 1.25 (t, 3H), 1.71-2.05 (m, 4H), 2.42-2.54 (m, 5H), 2.79-2.88 (m, 1H), 3.08-3.17 (m, 1H), 3.39-3.50 (m, 1H), 3.71-3.82 (m, 1H), 6.82 (t, 1H), 7.81 (d, 2H), 8.06 (s, 1H),

8.10 (d, 2H).

Compound No. (a-57): $^1$H-NMR (400 MHz, $CDCl_3$) δ ppm: 1.09-2.05 (m, 10H), 2.42-3.13 (m, 8H), 3.42-3.48 (m, 1H), 4.74-4.82 (m, 1H), 7.34 (d, 2H), 7.86 (s, 1H), 7.98 (d, 2H).

Compound No. (a-68): $^1$H-NMR (400 MHz, $CDCl_3$) δ ppm: 1.91-2.17 (m, 4H), 3.39-4.46 (m, 2H), 3.62-4.27 (m, 2H), 6.83 (t, 1H), 7.82 (d, 2H), 8.08-8.12 (m, 3H).

Compound No. (a-71): $^1$H-NMR (400 MHz, $CDCl_3$) δ ppm: 1.22-1.39 (m, 2H), 1.65-1.99 (m, 4H), 2.58 (s, 3H), 2.65-2.91 (m, 3H), 3.13 (t, 1H), 3.39-3.50 (m, 1H), 4.71-4.82 (m, 1H), 6.82 (t, 1H), 7.80 (d, 2H), 8.05 (s, 1H), 8.10 (d, 2H).

Compound No. (a-72): $^1$H-NMR (400 MHz, $CDCl_3$) δ ppm: 1.21-1.36 (m, 2H), 1.65-1.95 (m, 4H), 2.78-3.19 (m, 7H), 3.39-3.50 (m, 1H), 4.74-4.82 (m, 1H), 6.82 (t, 1H), 7.82 (d, 2H), 8.05 (s, 1H), 8.10 (d, 2H).

Compound No. (a-73): $^1$H-NMR (400 MHz, $CDCl_3$) δ ppm: 1.11-1.32 (m, 5H), 1.62-1.95 (m, 4H), 2.52-2.60 (m, 3H), 2.78-3.46 (m, 3H), 4.72-4.82 (m, 1H), 6.81 (t, 1H), 7.81 (d, 2H), 8.05 (s, 1H), 8.10 (d, 2H).

Compound No. (a-81): $^1$H-NMR (400 MHz, $CDCl_3$) δ ppm: 1.18-1.51 (m, 5H), 1.80-2.46 (m, 5H), 2.55-2.80 (m, 5H), 2.87 (t, 1H), 3.16 (t, 1H), 3.48 (t, 1H), 4.81 (t, 1H), 7.78 (d, 2H), 7.87 (s, 1H), 8.03 (d, 2H).

Compound No. (a-82): $^1$H-NMR (400 MHz, $CDCl_3$) δ ppm: 1.29-1.54 (m, 5H), 2.05-2.19 (m, 2H), 2.46 (s, 1H), 2.65 (s, 3H), 2.85-3.07 (m, 5H), 3.17 (t, 1H), 3.45 (d, 1H), 4.80 (d, 1H), 7.78 (d, 2H), 7.87 (s, 1H), 8.03 (d, 2H).

Compound No. (a-102): $^1$H-NMR (400 MHz, $CDCl_3$) δ ppm: 1.11-1.36 (m, 5H), 1.70-2.05 (m, 2H), 2.42-2.57 (m, 5H), 2.75-2.87 (m, 1H), 3.05-3.15 (m, 1H), 3.29-3.41 (m, 1H), 4.75 (t, 1H), 6.61 (t, 1H), 7.28 (d, 2H), 8.01-8.11 (m, 3H).

Compound No. (a-107): $^1$H-NMR (400 MHz, $CDCl_3$) δ ppm: 1.16-1.43 (m, 2H), 1.86-2.05 (m, 3H), 2.63 (s, 3H), 2.81 (t, 1H), 3.10 (t, 1H), 3.29-3.52 (m, 3H), 4.81 (d, 1H), 6.58 (t, 1H), 7.27 (d, 2H), 7.83 (s, 1H), 7.97 (d, 2H).

Compound No. (a-111): $^1$H-NMR (400 MHz, $CDCl_3$) δ ppm: 1.16-1.26 (m, 2H), 1.33 (s, 9H), 1.69-2.06 (m, 4H), 2.44-2.87 (m, 6H), 3.12 (t, 1H), 3.44-3.48 (m, 1H), 4.77-4.82 (m, 1H), 7.79 (d, 2H), 8.00 (s, 1H), 8.05 (d, 2H).

Compound No. (a-116): $^1$H-NMR (400 MHz, $CDCl_3$) δ ppm: 1.73-2.10 (m, 3H), 2.23 (d, 1H), 2.82-3.34 (m, 3H), 3.40 (s, 3H), 3.50 (t, 1H), 4.79 (dd, 1H), 7.28-7.41 (m, 5H), 7.84 (d, 2H), 8.07-8.18 (m, 3H).

Compound No. (a-119): $^1$H-NMR (400 MHz, $CDCl_3$) δ ppm: 1.11-1.32 (m, 5H), 1.71-2.05 (m, 4H), 2.46-2.60 (m, 4H), 2.75-3.16 (m, 3H), 3.43-3.52 (m, 4H), 4.52-4.89 (m, 3H), 7.78 (d, 2H), 7.89 (s, 1H), 8.04 (d, 2H).

Compound No. (a-123): $^1$H-NMR (400 MHz, $CDCl_3$) δ ppm: 1.16-1.52 (m, 11H), 1.73-2.63 (m, 9H), 2.84 (t, 1H), 3.08-3.21 (m, 1H), 3.53-3.61 (m, 1H), 4.75-4.82 (m, 1H), 6.95 (s, 1H), 7.69 (d, 2H), 7.90 (d,2H).

Compound No. (a-124): $^1$H-NMR (400 MHz, $CDCl_3$) δ ppm: 1.19-1.32 (m, 5H), 1.71-2.49 (m, 4H), 2.63-3.22 (m, 5H), 3.43-3.52 (m, 4H), 4.58-4.91 (m, 3H), 7.78 (d, 2H), 7.89 (s, 1H), 8.04 (d, 2H).

Compound No. (a-132): $^1$H-NMR (400 MHz, $CDCl_3$) δ ppm: 1.11-1.32 (m, 5H), 1.69-2.05 (m, 4H), 2.46-2.58 (m, 4H), 2.75-3.98 (m, 9H), 4.69-4.82 (m, 3H), 7.77-7.80 (m, 3H), 8.06 (d, 2H).

Compound No. (a-133): $^1$H-NMR (400 MHz, $CDCl_3$) δ ppm: 1.11-1.32 (m, 5H), 1.69-2.05 (m, 4H), 2.46-2.58 (m, 4H), 2.75-3.98 (m, 9H), 4.69-4.82 (m, 3H), 7.77-7.80 (m, 3H), 8.06 (d, 2H).

Compound No. (a-135): $^1$H-NMR (400 MHz, $CDCl_3$) δ ppm: 1.08-1.40 (m, 5H), 1.70-2.06 (m, 3H), 2.40-2.90 (m, 8H), 3.09 (t, 1H), 3.45 (d, 1H), 4.77 (t, 1H), 7.10-7.20 (m, 2H), 7.59-7.90 (m, 2H).

Compound No. (a-144): $^1$H-NMR (400 MHz, $CDCl_3$) δ ppm: 1.08-1.41 (m, 8H), 1.70-2.10 (m, 3H), 2.42-2.60 (m, 4H), 2.79-3.15 (m, 4H), 3.47 (d, 1H), 4.80 (d, 1H), 7.52 (d, 1H), 7.58 (d, 1H), 7.72 (t, 1H), 7.83 (d, 1H).

Compound No. (a-146): $^1$H-NMR (400 MHz, $CDCl_3$) δ ppm:1.21-1.35 (m, 5H), 1.70-2.10 (m, 3H), 2.45-2.57 (m, 4H), 2.78 (t, 1H), 2.90-3.17 (m, 1H), 3.40-3.59 (m, 4H), 4.53-4.88 (m, 3H), 7.52 (d, 1H), 7.58 (d, 1H), 7.72 (t, 1H), 7.89 (s, 1H).

Compound No. (a-147): $^1$H-NMR (400 MHz, $CDCl_3$) δ ppm: 1.15-1.35 (m, 5H), 1.67-2.10 (m, 3H), 2.40-2.57 (m, 4H), 2.78 (t, 1H), 2.90-3.17 (m, 1H), 3.35-3.59 (m, 4H), 4.50-4.88 (m, 3H), 6.58 (t, 1H), 7.25 (d, 2H), 7.85 (s, 1H), 7.95 (d, 2H).

Compound No. (a-153): $^1$H-NMR (400 MHz, $CDCl_3$) δ ppm: 1.15-1.52 (m, 8H), 1.78-2.46 (m, 6H), 2.65-3.20 (m, 8H), 3.48 (d, 1H), 4.82 (d, 1H), 7.40 (d, 2H), 7.80 (s, 1H), 7.97 (d, 2H).

Compound No. (a-154): $^1$H-NMR (400 MHz, $CDCl_3$) δ ppm: 1.01-1.35 (m, 5H), 1.63-2.05 (m, 3H), 2.40-2.58 (m, 4H), 2.70-2.90 (m, 1H), 2.98-3.12 (m, 1H), 3.28-3.43 (m, 1H), 4.12 (s, 1H), 4.50 (t, 1H), 4.65-4.80 (m, 1H), 7.10 (d, 2H), 7.89 (s, 1H), 8.08 (d, 2H).

Compound No. (a-155): $^1$H-NMR (400 MHz, $CDCl_3$) δ ppm: 1.08-1.40 (m, 5H), 1.70-2.06 (m, 3H), 2.40-2.87 (m, 8H), 3.10 (t, 1H), 3.45 (d, 1H), 4.77 (t, 1H), 6.71 (t, 1H), 7.67 (d, 2H), 7.83 (s, 1H), 8.00 (d, 2H).

Compound No. (a-187): $^1$H-NMR (400 MHz, $CDCl_3$) δ ppm: 1.08-1.35 (m, 5H), 1.70-2.06 (m, 3H), 2.40-2.87 (m, 8H), 3.06 (t, 1H), 3.43 (d, 1H), 3.48 (s, 3H), 4.77 (t, 1H), 5.23 (s, 2H), 7.17 (d, 2H), 7.80 (s, 1H), 7.90 (d, 2H).

Compound No. (a-195): $^1$H-NMR (400 MHz, $CDCl_3$) δ ppm: 1.01-1.40 (m, 8H), 1.62-2.31 (m, 7H), 2.63-2.71 (m, 3H), 2.85 (t, 1H), 3.01-3.11 (m, 1H), 3.42 (d, 1H), 4.11 (q, 2H), 4.72 (t, 1H), 7.79-7.90 (m, 3H), 8.03 (d, 2H).

Compound No. (b-43): $^1$H-NMR (400 MHz, $CDCl_3$) δ ppm: 1.26 (t, 3H), 1.39-1.54 (m, 2H), 1.95-2.16 (m, 4H), 2.46-2.58 (m, 7H), 3.10-3.31 (m, 2H), 3.71-3.88 (m, 4H), 5.64-5.72 (m, 1H), 7.76 (d, 2H), 7.78 (s, 1H), 8.02 (d, 2H).

Compound No. (b-45): $^1$H-NMR (400 MHz, $CDCl_3$) δ ppm: 0.97 (t, 3H), 1.11-2.52 (m, 12H), 2.73-3.21 (m, 4H),

3.56-3.62 (m, 1H), 4.78-4.82 (m, 1H), 6.86 (s, 1H), 7.69 (d, 2H), 7.93 (d, 2H).
Compound No. (b-47): $^1$H-NMR (400 MHz, $CDCl_3$) δ ppm: 1.19-1.32 (m, 5H), 1.71-2.49 (m, 4H), 2.69-3.22 (m, 5H), 3.43-3.52 (m, 4H), 4.58-4.91 (m, 3H), 6.88 (s, 1H), 7.71 (d, 2H), 7.92 (d, 2H).
Compound No. (b-48): $^1$H-NMR (400 MHz, $CDCl_3$) δ ppm: 1.11-1.35 (m, 5H), 1.69-2.05 (m, 4H), 2.46-2.58 (m, 4H), 2.75-3.98 (m, 9H), 4.69-4.82 (m, 3H), 7.77-7.80 (m, 3H), 8.06 (d, 2H).
Compound No. (b-71): $^1$H-NMR (400 MHz, $CDCl_3$) δ ppm: 1.03-1.35 (m, 5H), 1.63-2.05 (m, 3H), 2.40-2.58 (m, 4H), 2.70-2.85 (m, 1H), 2.95-3.12 (m, 1H), 3.31-3.50 (m, 1H), 4.15 (s, 1H), 4.45 (t, 1H), 4.60-4.78 (m, 1H), 7.00 (d, 2H), 7.38-7.45 (m, 1H), 7.76-7.82 (m, 3H), 8.15 (s, 1H).
Compound No. (c-2): $^1$H-NMR (400 MHz, $CDCl_3$) δ ppm: 1.29-1.41 (m, 3H), 1.80-1.95 (m, 1H), 2.28-2.44 (m, 1H), 2.50-2.99 (m, 8H), 3.10-3.43 (m, 2H), 3.55-3.71 (m, 1H), 3.82-4.16 (m, 1H), 7.80 (d, 2H), 7.91 (d, 1H), 8.05 (d, 2H).
Compound No. (c-4): $^1$H-NMR (400 MHz, $CDCl_3$) δ ppm: 1.25-1.38 (m, 3H), 1.75-1.90 (m, 1H), 2.18-2.40 (m, 1H), 2.51-2.92 (m, 8H), 3.10-4.05 (m, 4H), 7.40-7.60 (m, 1H), 7.65 (d, 2H), 7.80-7.91 (m, 3H), 7.95-8.10 (m, 1H).
Compound No. (d-1): $^1$H-NMR (400 MHz, $CDCl_3$) δ ppm: 1.27 (t, 3H), 2.35-2.81 (m, 8H), 3.59-3.81 (m, 4H), 7.80 (d, 2H), 7.99-8.18 (m, 3H).
Compound No. (d-2): $^1$H-NMR (400 MHz, $CDCl_3$) δ ppm: 1.38 (t, 3H), 2.72-3.10 (m, 7H), 3.29-3.41 (m, 1H), 3.98-4.18 (m, 2H), 4.30 (t, 1H), 4.53 (t, 1H), 7.78 (d, 2H), 7.98 (s, 1H), 8.05 (d, 2H).
Compound No. (e-7): $^1$H-NMR (400 MHz, $CDCl_3$) δ ppm: 1.42-2.25 (m, 7H), 2.60-2.90 (m, 4H), 3.10-3.86 (m, 3H), 4.08-4.38 (m, 1H), 7.75 (d, 2H), 7.87 (s, 1H), 8.06 (d, 2H).
Compound No. (e-11): $^1$H-NMR (400 MHz, $CDCl_3$) δ ppm: 0.80-1.03 (m, 3H), 1.40-2.35 (m, 8H), 2.59-2.80 (m, 4H), 2.88-4.40 (m,4H), 7.75 (d, 2H), 7.87 (s, 1H), 8.06 (d, 2H).

Next, compounds according to the present invention, in which Q is represented by formula (II-b), are explained.

Example 3

Synthesis of 6-(difluoromethyl)-5-(2-ethyl-2,8-diazaspiro[4.5]decane-8-carbonyl) -2-(4-(trifluoromethyl)phenyl)nicotinonitrile

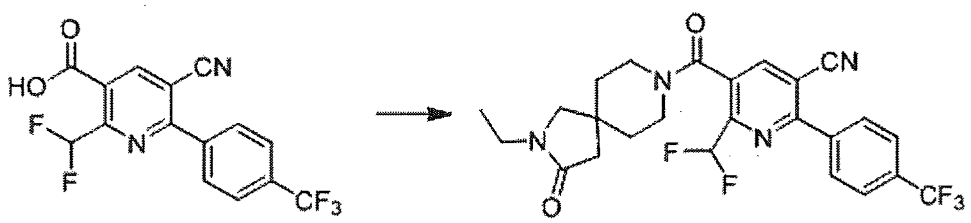

5-cyano-2-(difluoromethyl)-6-(4-(trifluoromethyl)phenyl)nicotinate (0.25 g) was suspended in dichloromethane (10 ml), and then 2-ethyl-2,8-diazaspiro[4.5]decan-3-one hydrochloride (0.1 g), 4-(N,N-dimethylamino)pyridine (0.36 g) and 1-[3-(diethylamino)propyl]-3-ethylcarbodiimide hydrochloride (0.28 g) were added thereto, followed by stirring the mixture at room temperature overnight.

The resultant solution was poured into ice-water and then extracted with chloroform, followed by conducting washing sequentially with water and then with saturated saline. Then, the resultant was dried with anhydrous magnesium sulfate. The solvent vas distilled off under reduced pressure, and the resultant residue was purified by silica gel column chromatography (eluent: n-hexane / ethyl acetate) to obtain the target compound (0.12g, at a yield of 32%). The NMR result of the target compound is shown below.
$^1$H-NMR ($CDCl_3$, δ ppm) 1.11 (t, 3H), 1.54-2.41 (m, 6H), 3.17-4.25 (m, 8H), 6.82 (t, 1H), 7.82 (d, 2H), 8.07 (s, 1H), 8.10 (d, 2H)

Example 4

Synthesis of 6-(difluoromethyl)-5-(2-oxa-8-azaspiro[4.5]decane-8-carbonyl)-2-(4-(trifluoromethyl)phenyl)nicotinonitrile

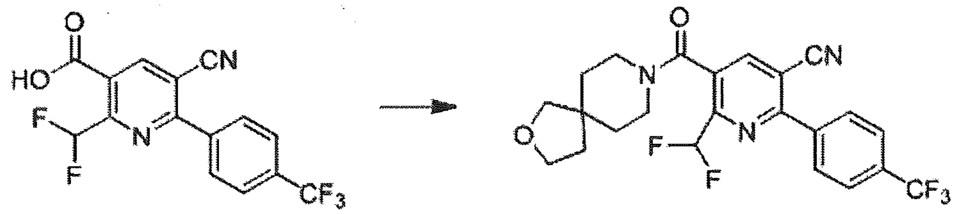

5-Cyano-2-(difluoromethyl)-6-(4-(trifluoromethyl)phenyl)nicotinate (0.25 g) was suspended in dichloromethane (10 ml), and then 2-oxa-8-azaspiro[4.5]decane hydrochloride (0.1 g), 4-(N,N-dimethylamino)pyridine (0.36 g) and 1-[3-(diethylamino)propyl]-3-ethylcarbodiimide hydrochloride (0.28 g) were added thereto, followed by stirring the mixture at room temperature overnight.

The resultant solution was poured into ice-water and then extracted with chloroform, followed by conducting washing sequentially with water and then with saturated saline. Then, the resultant was dried with anhydrous magnesium sulfate. The solvent was distilled off under reduced pressure, and the resultant residue was purified by silica gel column chromatography (eluent: n-hexane / ethyl acetate) to obtain the target compound (0.13 g, at a yield of 38%). The NMR result of the target compound is shown below.

$^1$H-NMR ($CDCl_3$, $\delta$ ppm) 1.58-1.92 (m, 6H), 3.27-3.92 (m, 8H), 6.82 (t, 1H), 7.82 (m, 2H), 8.06 (s, 1H), 8.11 (d, 2H)

Some compounds according to the present invention, prepared in the same manner as the above-mentioned examples, are shown in Table 7. Table 7 shows substituents of compounds of formula (I-b). n in the formula indicates the number of substituents (X3) on a benzene ring. The physical properties column of Table 7 shows the properties, melting point (m.p.) or refractive index ($n_D$). In Table 7, Me represents a methyl group, and Ph represents a phenyl group. An arrow indicates a binding position.

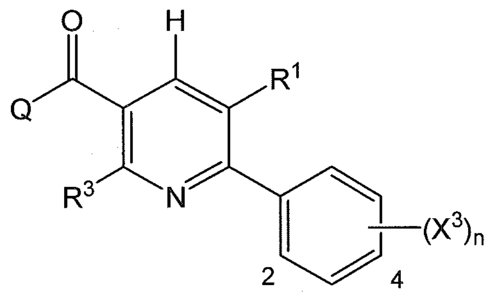

(I-b)

Table 7

| No. | Q | $R^1$ | $R^3$ | $(X^3)n$ | Physical properties |
|---|---|---|---|---|---|
| A-1 | 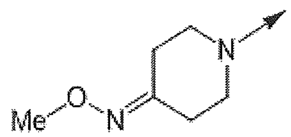 | CN | $OF_2H$ | 4-$CF_3$ | m.p. 131-134°C |
| A-2 | 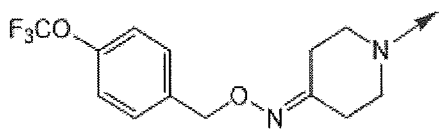 | CN | $CF_2H$ | 4-$CF_3$ | AMORPHOUS |
| A-3 | 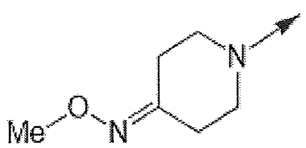 | CN | Me | 4-$CF_3$ | AMORPHOUS |

(continued)

| No. | Q | $R^1$ | $R^3$ | $(X^3)n$ | Physical properties |
|---|---|---|---|---|---|
| A-4 | 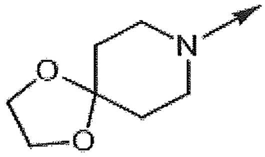 | ON | $OF_2H$ | 4-$CF_3$ | m.p.186-189°C |
| A-5 | 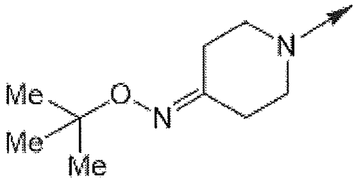 | CN | $OF_2H$ | 4-$CF_3$ | m.p.158-160°C |
| A-6 | 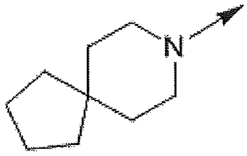 | CN | $CF_2H$ | 4-$CF_3$ | AMORPHOUS |
| A-7 | 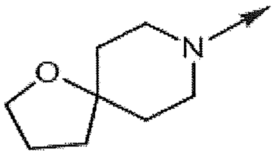 | CN | $OF_2H$ | 4-$CF_3$ | m.p.176-179°C |
| A-8 | 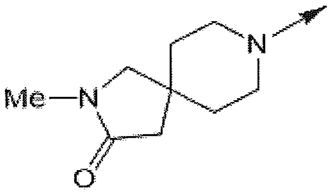 | CN | $CF_2H$ | 4-$CF_3$ | VISCOUS OIL |
| A-9 | 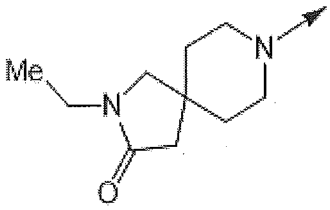 | CN | $CF_2H$ | 4-$CF_3$ | VISCOUS OIL |
| A-10 | 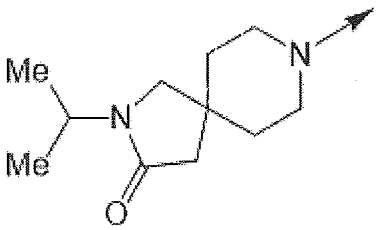 | CN | $CF_2H$ | 4-$CF_3$ | VISCOUS OIL |
| A-11 | 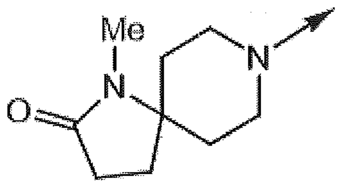 | CN | $CF_2H$ | 4-$CF_3$ | VISCOUS OIL |

(continued)

| No. | Q | $R^1$ | $R^3$ | $(X^3)n$ | Physical properties |
|---|---|---|---|---|---|
| A-12 | 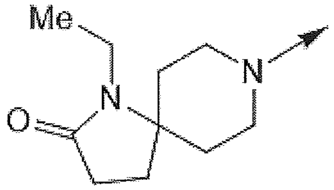 | CN | $CF_2H$ | 4-$CF_3$ | VISCOUS OIL |
| A-13 | 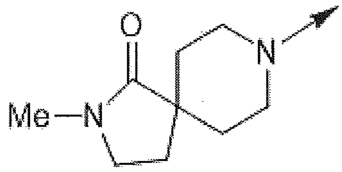 | CN | $CF_2H$ | 4-$CF_3$ | VISCOUS OIL |
| A-14 | 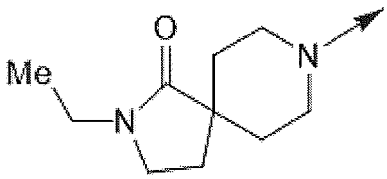 | CN | $CF_2H$ | 4-$CF_3$ | VISCOUS OIL |
| A-15 | 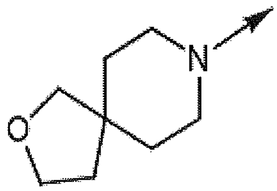 | CN | $CF_2H$ | 4-$CF_3$ | AMORPHOUS |
| A-16 | 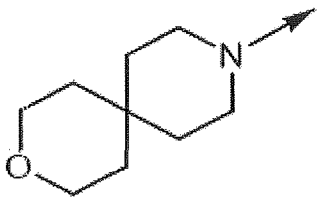 | CN | $OF_2H$ | 4-$CF_3$ | m.p. 190-195°C |
| A-1 7 | 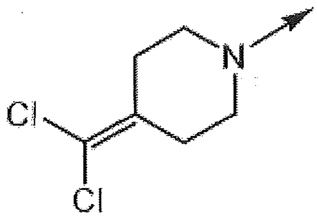 | CN | $CF_3$ | 4-$CF_3$ | m.p. 200-202°C |
| A-18 | 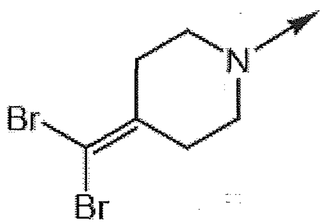 | CN | $CF_3$ | 4-$CF_3$ | m.p. 189-190°C |

(continued)

| No. | Q | $R^1$ | $R^3$ | $(X^3)n$ | Physical properties |
|---|---|---|---|---|---|
| A-19 | 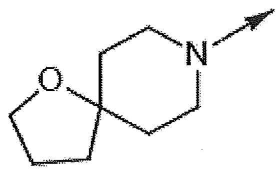 | $C(=S)NH_2$ | $CF_2H$ | $4\text{-}CF_3$ | m.p. 224-227°C |
| A-20 | 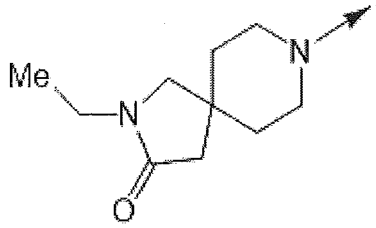 | $C(=S)NH_2$ | $OF_2H$ | $4\text{-}CF_3$ | m.p. 263-265°C |
| A-21 | 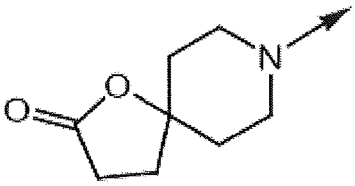 | CN | $CF_3$ | $4\text{-}CF_3$ | VISCOUS OIL |
| A-22 | 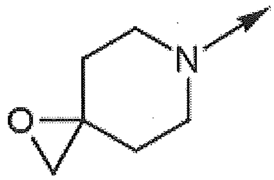 | CN | Me | $4\text{-}CF_3$ | m.p. 196-198°C |
| A-23 | 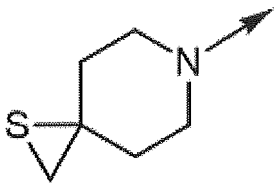 | CN | Me | $4\text{-}CF_3$ | m.p. 169-170°C |
| A-24 | 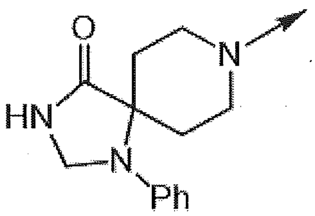 | CN | Me | $4\text{-}CF_3$ | m.p. 257-258°C |
| A-25 | 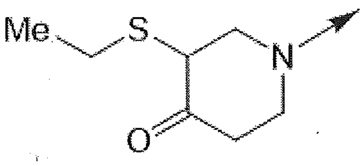 | CN | Me | $4\text{-}CF_3$ | AMORPHOUS |

(continued)

| No. | Q | $R^1$ | $R^3$ | $(X^3)n$ | Physical properties |
|---|---|---|---|---|---|
| A-26 | 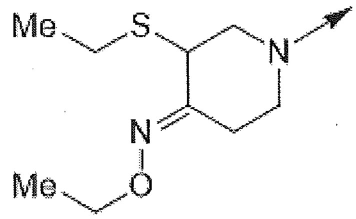 | CN | Me | 4-$CF_3$ | m.p. 66-67°C |

Among the compounds shown in Table 7, the compounds having viscous oil properties or amorphous properties were subjected to $^1$H-NMR ($CDCl_3$) measurement. The resultant measurement values are shown below.

Compound No. (A-2): $^1$H-NMR (400 MHz, $CDCl_3$) δ ppm: 2.36-2.82 (m, 4H), 3.34-4.03 (m, 4H), 5.07 (s, 2H), 6.83 (t, 1H), 7.17-7.38 (m, 4H), 7.82 (d, 2H), 8.08-8.12 (m, 3H).

Compound No. (A-3): $^1$H-NMR (400 MHz, $CDCl_3$) δ ppm: 2.32-2.80 (m, 7H), 3.37-4.05 (m, 7H), 7.79 (d, 2H), 7.80 (s, 1H), 8.05 (d, 2H).

Compound No. (A-6): $^1$H-NMR (400 MHz, $CDCl_3$) δ ppm: 1.39-1.69 (m, 12H), 3.22-3.26 (m, 2H), 3.75-3.79 (m, 2H), 6.82 (t, 1H), 7.81 (d, 2H), 8.06 (s, 1H), 8.10 (d, 2H).

Compound No. (A-8): $^1$H-NMR (400 MHz, $CDCl_3$) δ ppm: 1.55-2.74 (m, 4H), 2.86 (s, 3H), 3.19-4.22 (m, 8H), 6.82 (t, 1H), 7.81 (d, 2H), 8.07 (s, 1H), 8.10 (d, 2H).

Compound No. (A-9): $^1$H-NMR (400 MHz, $CDCl_3$) δ ppm: 1.11 (t, 3H), 1.54-2.41 (m, 6H), 3.17-4.25 (m, 8H), 6.82 (t, 1H), 7.82 (d, 2H), 8.07 (s, 1H), 8.10 (d, 2H).

Compound No. (A-10): $^1$H-NMR (400 MHz, $CDCl_3$) δ ppm: 1.09-1.13 (m, 6H), 1.52-1.79 (m, 4H), 2.32-2.40 (m, 2H), 3.11-3.71 (m, 6H), 4.32-4.43 (m, 1H), 6.83 (t, 1H), 7.82 (d, 2H), 8.06 (s, 1H), 8.10 (d, 2H).

Compound No. (A-11): $^1$H-NMR (400 MHz, $CDCl_3$) δ ppm: 1.32-2.11 (m, 6H), 2.40-2.48 (m, 2H), 2.76 (s, 3H), 2.88-3.55 (m, 3H), 4.79-4.88 (m, 1H), 6.85 (t, 1H), 7.82 (d, 2H), 8.09-8.412 (m, 3H).

Compound No. (A-12): $^1$H-NMR (400 MHz, $CDCl_3$) δ ppm: 1.14-3.54 (m, 16H), 4.82-4.89 (m, 1H), 6.85 (t, 1H), 7.82 (d, 2H), 8.09-8.12 (m, 3H).

Compound No. (A-13): $^1$H-NMR (400 MHz, $CDCl_3$) δ ppm: 1.40-2.04 (m, 6H), 2.86 (s, 3H), 3.17-3.75 (m, 5H), 4.28-4.33 (m, 1H), 6.82 (t, 1H), 7.81 (d, 2H), 8.09-8.12 (m, 3H).

Compound No. (A-14): $^1$H-NMR (400 MHz, $CDCl_3$) δ ppm: 1.14 (t, 3H), 1.40-2.16 (m, 6H), 3.17-3.73 (m, 7H), 4.24-4.32 (m, 1H), 6.82 (t, 1H), 7.81 (d, 2H), 8.09-8.12 (m, 3H).

Compound No. (A-15): $^1$H-NMR (400 MHz, $CDCl_3$) δ ppm: 1.58-1.92 (m, 6H), 3.27-3.92 (m, 8H), 6.82 (t, 1H), 7.82 (m, 2H), 8.06 (s, 1H), 8.11 (d, 2H).

Compound No. (A-21): $^1$H-NMR (400 MHz, $CDCl_3$) δ ppm: 1.65-2.20 (m, 5H), 2.58-2.72 (m, 3H), 3.21-3.52 (m, 3H), 4.50-4.69 (m, 1H), 7.82 (d, 2H), 8.10-8.18 (m, 3H).

Compound No. (A-25): $^1$H-NMR (400 MHz, $CDCl_3$) δ ppm: 1.18-1.31 (m, 3H), 2.25-3.96 (m, 11H), 4.85-5.01 (m, 1H), 7.80 (d, 2H), 7.95-8.20 (m, 3H).

Next, compounds according to the present invention, in which Q is represented by formula (II-c); are explained.

Example 5

Preparation of t-butyl-4-(5-cyano-2-(trifluoromethyl)-6-(4-(trifluoromethyl) phenyl)nicotinoyl)piperazine-1-carboxylate (compound No. B-1)

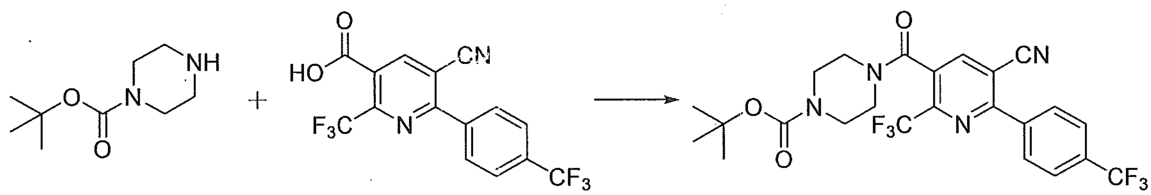

5-Cyano-2-(trifluoromethyl)-6-(4-(trifluoromethyl)phenyl)nicotinate (1.0 g) was dissolved in dichloromethane (14 ml). N,N-dimethylformamide (0.02 g) and oxalyl chloride (0.48 ml) were added thereto under ice-cooling, followed by stirring the mixture at room temperature for 1 hour. The resultant solution was concentrated under reduced pressure. The concentrate was dissolved in dichloromethane (14 ml). T-butylpiperazine-1-carboxylate (0.77 g) and triethylamine (1.2 ml) were added thereto, and then stirred at room temperature overnight. The solvent was distilled off from the resultant solution under reduced pressure, and the resultant residue was purified by silica gel column chromatography (eluent: n-hexane / ethyl acetate = 3/1) to obtain 1.1 g of the target compound at a yield of 75%. The physical properties are shown below.
Melting point: 190-192°C
$^1$H-NMR ($CDCl_3$, δ ppm) 1.47 (9H, s), 3.23 (2H, m), 3.38-3.91 (6H, m), 7.84 (2H, d), 8.13 (1H, s), 8.15 (2H, m).

Example 6

Preparation of 5-(4-(ethylsulfonyl)piperazine-1-carbonyl) 6-(trifluoromethyl)-2-(4-(trifluoromethyl)phenyl)nicotinonitrile (compound No. B-2)

(Step 1)

Synthesis of 5-(piperazine-1-carbonyl)-6-(trifluoromethyl)-2-(4-trifluoromethyl) phenyl)nicotinonitrile hydrochloride

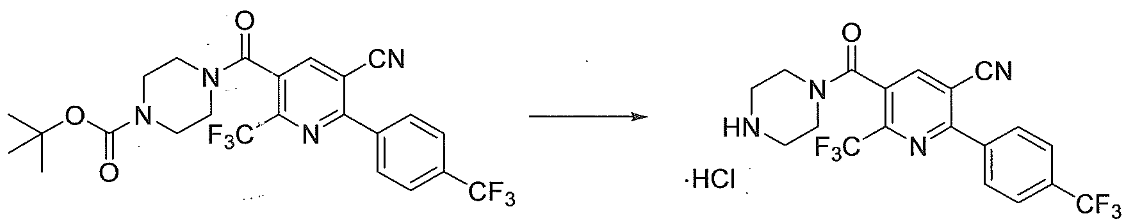

T-butyl 4-(5-cyano-2-(trifluoromethyl)-6-(4-(trifluoromethyl)phenyl)nicotinoyl) piperazine-1-carboxylate (1.0 g) was dissolved in 1,4-dioxane (9 ml). A 1,4-dioxane solution of 4M hydrochloric acid (2.4 ml) was added thereto, and then stirred at room temperature overnight.
The solvent was distilled off from the resultant solution under reduced pressure to obtain the target compound (0.85 g) at a yield of 100%. The physical properties are shown below.
$^1$H-NMR ($CD_3OD$, δ ppm) 3.11 (1H, m), 3.20-3.38 (3H, m), 3.50-3.68 (2H, m), 3.90 (1H, m), 4.10 (1H, m), 7.90 (2H, d), 8.20 (2H, d), 8.67 (1H, s).

(Step 2)

Synthesis of 5-(4-(ethylsulfonyl)piperazine-1-carbonyl)6-(trifluoromethyl)-2-(4-(trifluoromethyl)phenyl)nicotinonitrile

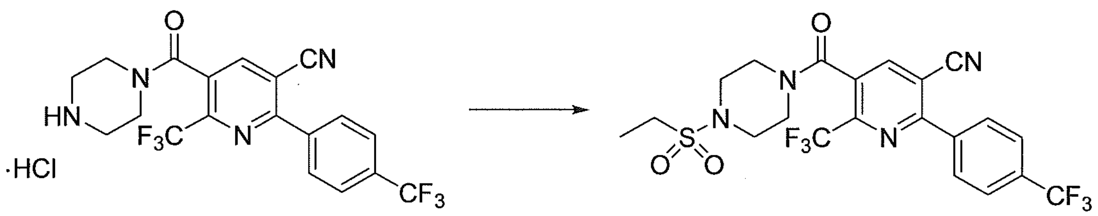

5-(Piperazine-1-carbonyl)-6-(trifluoromethyl)-2-(4-trifluoromethyl)phenyl) nicotinonitrile hydrochloride (0.15 g) was dissolved in dichloromethane (3.5 ml). Ethanesulfonyl chloride (0.05 ml) and triethylamine (0.15 ml) were added thereto, and then stirred at room temperature overnight.
The solvent was distilled off from the resultant solution under reduced pressure. The resultant residue was purified by silica gel column chromatography (eluent: n-hexane / ethyl acetate = 1/1) to obtain the target compound (0.13

g) at a yield of 70%. The physical properties are shown below.
Melting point: 224-226°C
$^1$H-NMR ($CDCl_3$, $\delta$ ppm) 1.39 (3H, t), 3.00 (2H, q), 3.20 (1H, m), 3.35 (4H, m), 3.52 (1H, m), 3.73 (1H, m), 4.10 (1H, m), 7.83 (2H, d), 8.13 (2H, d), 8.15 (1H, s).

Some compounds according to the present invention, prepared in the same manner as the above-mentioned examples, are shown below.

Compound No. B-3 (Melting point: 126-128°C)

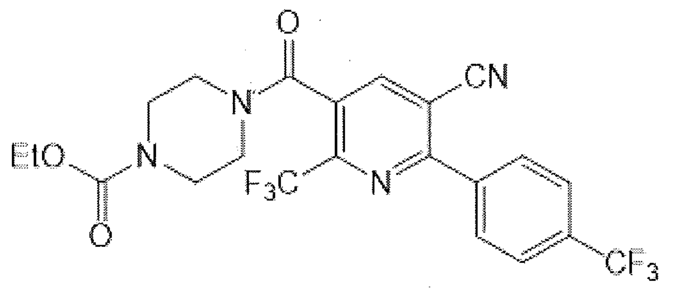

Compound No. B-4

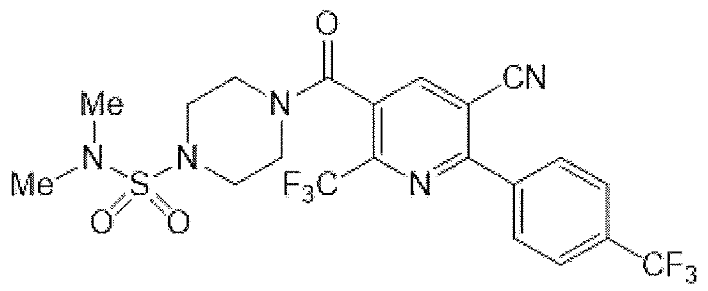

$^1$H-NMR ($CDCl_3$, $\delta$ ppm) 2.81 (s, 3H), 2.85 (s, 3H), 3.17-3.45 (m, 6H), 3.76-4.04 (m, 2H), 7.83 (d, 2H), 8.13-8.15 (m, 3H).

(Biological test)

The following test examples show that the compound according to the present invention is useful as an active ingredient of an insecticide, acaricide or ectoparasite control agent.

(Preparation of test emulsion)

5 parts by mass of a compound according to the present invention, 93.6 parts by mass of dimethylformamide, and 1.4 parts by mass of polyoxyethylene alkyl aryl ether were mixed and dissolved to obtain an emulsion (I) containing 5% by mass of the active ingredient.

(Test Example 1) Efficacy Test against Mythimna separata

0.8 g of commercially-available artificial feed (Insect LFS, manufactured by Nosan Corporation) and 1 $\mu$l of the emulsion (I) were mixed well, and then 0.2 g of the mixture per treated area was packed into plastic test containers (each having a capacity of 1.4 ml) as test feeds. Two second-instar larvae of Mythimna separata were left per treated area, and then the test containers were sealed with plastic covers. The test containers were placed in a thermostatic chamber at 25°C, and, after 5 days passed therefrom, the insecticidal rate and the feed intake amount were measured. The test was repeated twice.

The compounds shown in Table 8 were subjected to the efficacy test against Mythimna separata. All of the compounds exhibited, against Mythimna separata, an insecticidal rate of 100% or the feed intake amount of 10% or less, relative to the solvent control area.

Table 8

| No. | | | | |
|---|---|---|---|---|
| a-1 | a-60 | a-128 | a-180 | b-70 |
| a-6 | a-61 | a-137 | a-181 | b-73 |
| a-7 | a-62 | a-141 | a-182 | b-82 |
| a-12 | a-63 | a-152 | a-202 | b-83 |
| a-18 | a-64 | a-153 | b-1 | b-84 |
| a-20 | a-68 | a-154 | b-2 | b-85 |
| a-36 | a-74 | a-156 | b-26 | b-86 |
| a-40 | a-100 | a-162 | b-53 | b-87 |
| a-56 | a-101 | a-176 | b-54 | A-18 |
| a-57 | a-103 | a-179 | b-69 | A-21 |

(Test Example 2) Efficacy Test against Tetranychus kanzawai

Five female adults of Tetranychus kanzawai from Okayama Prefecture were left on the primary leaves of mung beans. Then, the emulsion (I) was diluted with water such that the concentration of the compound became 125 ppm by mass to obtain a test agent. The test agent was sprayed on the mung beans and air-dried. Thereafter, the mung beans were placed in a thermostatic chamber at a temperature of 25°C and a humidity of 65%. The acarian survival was investigated 10 days after spraying. The test was repeated twice.
The compounds shown in Table 9 were subjected to the efficacy test against Tetranychus kanzawai. All of the compounds exhibited, against Tetranychus kanzawai, an acaricidal rate of 90% or more.

Table 9

| No. | | | | | | | | |
|---|---|---|---|---|---|---|---|---|
| a-3 | a-47 | a-82 | a-105 | a-133 | b-18 | b-43 | e-3 | A-15 |
| a-11 | a-49 | a-83 | a-107 | a-134 | b-19 | b-44 | e-5 | A-18 |
| a-12 | a-50 | a-84 | a-110 | a-135 | b-21 | b-45 | e-6 | A-26 |
| a-14 | a-51 | a-85 | a-112 | a-136 | b-22 | b-46 | e-7 | B-2 |
| a-15 | a-52 | a-86 | a-113 | a-137 | b-23 | b-47 | e-8 | B-3 |
| a-18 | a-53 | a-87 | a-114 | a-138 | b-24 | b-48 | e-9 | B-4 |
| a-22 | a-54 | a-88 | a-115 | a-139 | b-25 | b-49 | e-10 | |
| a-25 | a-55 | a-89 | a-116 | a-160 | b-26 | b-50 | e-11 | |
| a-26 | a-56 | a-90 | a-117 | a-185 | b-28 | b-51 | f-1 | |
| a-27 | a-57 | a-91 | a-118 | a-187 | b-29 | b-52 | f-2 | |
| a-30 | a-58 | a-92 | a-119 | a-188 | b-30 | b-74 | f-3 | |
| a-31 | a-63 | a-93 | a-120 | a-189 | b-31 | b-75 | f-4 | |
| a-32 | a-70 | a-94 | a-121 | a-202 | b-32 | c-1 | f-5 | |
| a-36 | a-71 | a-95 | a-122 | b-1 | b-33 | c-2 | f-6 | |
| a-38 | a-72 | a-96 | a-124 | b-2 | b-34 | c-3 | A-1 | |
| a-39 | a-73 | a-97 | a-125 | b-4 | b-35 | c-4 | A-3 | |
| a-41 | a-74 | a-99 | a-126 | b-5 | b-36 | d-1 | A-4 | |
| a-42 | a-76 | a-100 | a-127 | b-6 | b-37 | d-2 | A-7 | |
| a-43 | a-78 | a-101 | a-128 | b-7 | b-39 | d-3 | A-8 | |
| a-44 | a-79 | a-102 | a-130 | b-15 | b-40 | d-5 | A-9 | |
| a-45 | a-80 | a-103 | a-131 | b-16 | b-41 | e-1 | A-10 | |
| a-46 | a-81 | a-104 | a-132 | b-17 | b-42 | e-2 | A-13 | |

(Test Example 3) Efficacy Test against Tetranychus urticae

Common beans were grown in a 3-sun pot and eight female adults of Tetranychus urticae from Aomori Prefecture

were left on the primary leaves thereof. The emulsion (I) was diluted with water such that the concentration of the compound according to the present invention became 125 ppm by mass. The diluent was sprayed on the common beans. The common beans were placed in a thermostatic chamber at a temperature of 25°C and a humidity of 65%. The acarian survival was investigated 10 days after spraying. The test was repeated twice.

The compounds shown in Table 10 were subjected to the efficacy test against Tetranychus urticae. All of the compounds exhibited an acaricidal rate of 90% or more.

Table 10

| No. | | | | | | | | |
|---|---|---|---|---|---|---|---|---|
| a-3 | a-78 | a-118 | a-152 | a-197 | b-26 | b-60 | b-93 | B-2 |
| a-11 | a-79 | a-119 | a-153 | a-198 | b-28 | b-61 | b-94 | B-4 |
| a-22 | a-80 | a-120 | a-154 | a-199 | b-29 | b-62 | b-95 | |
| a-25 | a-81 | a-121 | a-155 | a-200 | b-30 | b-63 | c-1 | |
| a-26 | a-82 | a-122 | a-156 | a-201 | b-31 | b-64 | c-2 | |
| a-27 | a-83 | a-124 | a-157 | a-202 | b-32 | b-65 | c-3 | |
| a-30 | a-84 | a-125 | a-158 | a-203 | b-33 | b-66 | c-4 | |
| a-31 | a-85 | a-126 | a-159 | b-1 | b-34 | b-67 | d-1 | |
| a-32 | a-86 | a-127 | a-161 | b-2 | b-35 | b-68 | d-2 | |
| a-38 | a-87 | a-128 | a-164 | b-3 | b-36 | b-69 | d-3 | |
| a-39 | a-88 | a-130 | a-165 | b-4 | b-37 | b-70 | d-4 | |
| a-41 | a-89 | a-131 | a-166 | b-5 | b-39 | b-71 | d-5 | |
| a-42 | a-90 | a-132 | a-167 | b-6 | b-40 | b-72 | d-6 | |
| a-45 | a-91 | a-133 | a-170 | b-7 | b-41 | b-73 | e-1 | |
| a-46 | a-92 | a-134 | a-171 | b-8 | b-42 | b-74 | e-3 | |
| a-51 | a-93 | a-135 | a-172 | b-9 | b-43 | b-75 | e-4 | |
| a-52 | a-95 | a-136 | a-173 | b-10 | b-44 | b-76 | e-5 | |
| a-53 | a-96 | a-137 | a-174 | b-11 | b-45 | b-77 | e-11 | |
| a-54 | a-100 | a-138 | a-175 | b-12 | b-46 | b-78 | f-4 | |
| a-55 | a-101 | a-139 | a-176 | b-13 | b-47 | b-79 | f-5 | |
| a-56 | a-102 | a-140 | a-177 | b-14 | b-48 | b-80 | f-6 | |
| a-57 | a-103 | a-141 | a-178 | b-15 | b-49 | b-81 | A-1 | |
| a-60 | a-104 | a-142 | a-179 | b-16 | b-50 | b-82 | A-3 | |
| a-61 | a-105 | a-143 | a-180 | b-17 | b-51 | b-83 | A-7 | |
| a-63 | a-107 | a-144 | a-181 | b-18 | b-52 | b-84 | A-8 | |
| a-69 | a-110 | a-145 | a-182 | b-19 | b-53 | b-85 | A-9 | |
| a-70 | a-112 | a-146 | a-183 | b-20 | b-54 | b-86 | A-10 | |
| a-71 | a-113 | a-147 | a-184 | b-21 | b-55 | b-87 | A-15 | |
| a-72 | a-114 | a-148 | a-189 | b-22 | b-56 | b-88 | A-18 | |
| a-73 | a-115 | a-149 | a-190 | b-23 | b-57 | b-89 | A-20 | |
| a-74 | a-116 | a-150 | a-193 | b-24 | b-58 | b-90 | A-21 | |
| a-76 | a-117 | a-151 | a-196 | b-25 | b-59 | b-92 | A-26 | |

(Test Example 4) Efficacy Test against Tetranychus urticae (root-dip treatment)

The emulsion (I) was diluted with water such that the concentration of the compound according to the present invention became 31 ppm by mass. Roots of common beans at the seedling stage were washed in advance, and then dipped in the diluent. After 4 days passed from the dipping, 10 female adults of Tetranychus urticae were left on the roots, and then the roots were placed in a thermostatic chamber at a temperature of 25°C and a humidity of 60%. The acarian survival was investigated 10 days after leaving the acarians. The control value was calculated in accordance with the following formula.

$$\text{Control value (\%)} = 100-\{(Nt)/(Nc)\times 100\}$$

Nc: the number of surviving acarians at untreated area; Nt: the number of surviving acarians at treated area

The compounds shown in Table 11 were subjected to the efficacy test against Tetranychus urticae. All of the compounds exhibited a control value of 90% or more.

Table 11

| No. |
|---|
| a-32 |
| a-53 |
| a-54 |
| a-56 |
| a-57 |
| a-69 |

(Test Example 5) Efficacy Test against Tetranychus urticae (soil irrigation treatment)

The emulsion (I) was diluted with water such that the concentration of the compound according to the present invention became 500 ppm by mass. 4 ml of the diluent was irrigated at the base of a seedling of common bean planted in a cell, and then the cell was placed in a thermostatic chamber at a temperature of 25°C and a humidity of 60%. After 4 days passed from the irrigation at the base, the common bean was transferred to a 3-sun pot, and then 10 female adults of Tetranychus urticae were left, followed by placing the pot in a thermostatic chamber at a temperature of 25°C and a humidity of 60%. After 14 days passed from leaving the acarians, the acarian survival was investigated. The control value was calculated in accordance with the following formula.

$$\text{Control value (\%)} = 100-\{(Nt)/(Nc)\times 100\}$$

Nc: the number of surviving acarians at untreated area; Nt: the number of surviving acarians at treated area

The compounds shown in Table 12 were subjected to the efficacy test against Tetranychus urticae. All of the compounds exhibited a control value of 90% or more.

Table 12

| No. |
|---|
| a-32 |
| a-53 |
| a-54 |
| a-56 |
| a-57 |
| a-69 |

Since the compounds randomly selected from the compounds according to the present invention exhibited the above-described effects, it is understood that the compound according to the present invention, involving aspects of compounds that are not exemplified above, is a compound that exhibits pest control effects, particularly insecticidal effects, acaricidal effects, or ectoparasite control effects, without causing harmful effects on plants, and provides less toxicity on humans, animals, or fish, and fewer effects on the environment.

INDUSTRIAL APPLICABILITY

The cyclic amine compound according to the present invention makes it possible to control pests that cause problems in agricultural crops and hygiene. The cyclic amine compound according to the present invention particularly makes it possible to control agricultural insect pests and acarians at a low concentration effectively. In addition, the cyclic amine compound according to the present invention makes it possible to effectively control ectoparasites that cause harm to humans or livestock.

## Claims

1. A compound of formula (I) or a salt thereof:

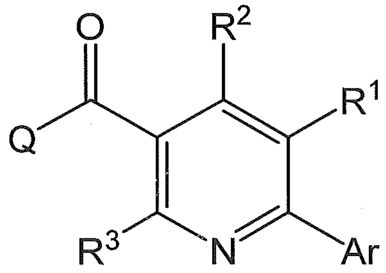

(I)

in the formula (I),

$A_1$ represents a substituted or unsubstituted C6-10 aryl group,
$R^1$ represents a cyano group or a substituted or unsubstituted thiocarbamoyl group,
$R^2$ represents a hydrogen atom, a halogeno group, a substituted or unsubstituted C1-6 alkyl group, a hydroxyl group, or a substituted or unsubstituted C1-6 alkoxy group,
$R^3$ represents a hydrogen atom, a halogeno group, a substituted or unsubstituted C1-6 alkyl group, a substituted or unsubstituted C3-8 cycloalkyl group, a hydroxyl group, a substituted or unsubstituted C1-6 alkoxy group, a substituted or unsubstituted C6-10 aryl group, or a substituted or unsubstituted 5- or 6-membered heteroaryl group,
Q represents a cyclic amino group of formula (II-a), a cyclic amino group of formula (II-b), or a cyclic amino group of formula (II-c):

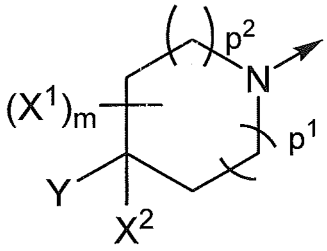

(II-a)

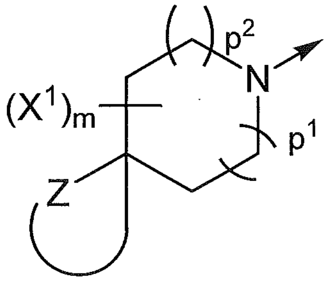

(II-b)

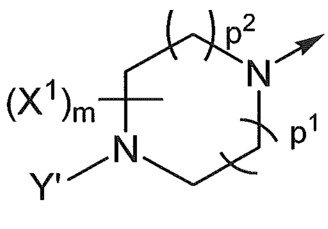

(II-c)

in the formulae, an arrow indicates a binding position,

$p^1$ indicates a number of methylenes in parentheses and is 0 or 1,
$p^2$ indicates a number of methylenes in parentheses and is an integer of 0 to 2,
$X^1$ indicates a substituent on the cyclic amino group, and is a halogeno group, a substituted or unsubstituted C1-6 alkyl group, a hydroxyl group, a substituted or unsubstituted C1-6 alkylsulfonyloxy group, or a substituted or unsubstituted C1-6 alkylthio group,
m indicates a number of $X^1$ and is an integer of 0 to 4,
$X^2$ represents a hydrogen atom, a halogeno group, a substituted or unsubstituted C1-6 alkyl group, a hydroxyl group, or a substituted or unsubstituted C1-6 alkoxy group,
Y represents a halogeno group, a substituted or unsubstituted C1-6 alkyl group, a substituted or unsubstituted C2-6 alkenyl group, a substituted or unsubstituted C1-6 alkoxy group, a substituted or unsubstituted C1-6 alkylcarbonyl group, a substituted or unsubstituted C1-6 alkoxycarbonyl group, a thiocarbamoyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 arylcarbonyl group, a group of $R^aO\text{-}N{=}CR^b\text{-}$, a group of $R^cCONR^d\text{-}$, a group of $R^cCOCH_2NR^d\text{-}$, a group of $R^eSO_2NR^f\text{-}$, a group of $R^gR^hN\text{-}CO\text{-}$, a group of $R^gR^hN\text{-}SO_2\text{-}$, or a cyanothio group,
$R^a$ represents a hydrogen atom or a substituted or unsubstituted C1-6 alkyl group,
$R^b$ represents a hydrogen atom or a substituted or unsubstituted C1-6 alkyl group,
$R^c$ represents a substituted or unsubstituted C1-6 alkyl group, a substituted or unsubstituted C1-6 alkoxy group, or a substituted or unsubstituted C6-10 aryl group,
$R^d$ represents a hydrogen atom or a substituted or unsubstituted C1-6 alkyl group,
$R^c$ and $R^d$ may be combined to form a substituted or unsubstituted C3-6 alkylene group, a substituted or unsubstituted C2-6 alkyleneoxy group, or a substituted or unsubstituted CI-6 alkyleneoxy CI-6 alkylene group,
$R^e$ represents a substituted or unsubstituted C1-6 alkyl group, or a substituted or unsubstituted C6-10 aryl group,
$R^f$ represents a hydrogen atom or a substituted or unsubstituted C1-6 alkyl group, $R^e$ and $R^f$ may be combined to form a substituted or unsubstituted C3-6 alkylene group,
$R^g$ represents a substituted or unsubstituted CI-6 alkyl group, a substituted or unsubstituted C1-6 alkoxy group, a substituted or unsubstituted C6-10 aryl group, or a substituted or unsubstituted C1-6 alkylsulfonyl group,
$R^h$ represents a hydrogen atom or a substituted or unsubstituted C1-6 alkyl group,
$R^g$ and $R^h$ may be combined to form a substituted or unsubstituted C3-6 alkylene group,
Z represents an oxo group, a group of $G^b\text{-}O\text{-}N{=}$, a substituted or unsubstituted C2-6 alkylene group, a group of $\text{-}O\text{-}G^a\text{-}$, a group of $\text{-}O\text{-}G^a\text{-}O\text{-}$, a group of $\text{-}O\text{-}CO\text{-}G^a\text{-}$, a group of $\text{-}G^a\text{-}O\text{-}G^a\text{-}$, a group of $\text{-}NG^b\text{-}CO\text{-}G^a\text{-}$, a group of $\text{-}CO\text{-}NG^b\text{-}G^a\text{-}$, or a group of $\text{-}G^a\text{-}NG^b\text{-}CO\text{-}G^a\text{-}$,
$G^a$ each independently represents a substituted or unsubstituted CI-6 alkylene group, $G^b$ each independently represents a substituted or unsubstituted C1-6 alkyl group,
Y' represents a substituted or unsubstituted C1-6 alkylcarbonyl group, a substituted or unsubstituted CI-6 alkoxycarbonyl group, a group of $R^{g'}R^hN\text{-}CO\text{-}$, a group of $R^{g'}R^hN\text{-}CS\text{-}$, a group of $R^{g'}R^hN\text{-}CO\text{-}CO\text{-}$, a substituted or unsubstituted CI-6 alkylsulfonyl group, or a group of $R^{g'}R^hN\text{-}SO_2\text{-}$,
$R^{g'}$ represents a substituted or unsubstituted C1-6 alkyl group, a substituted or unsubstituted C1-6 alkoxy group, or a substituted or unsubstituted C6-10 aryl group, and
$R^{g'}$ and $R^h$ may be combined to form a substituted or unsubstituted C3-6 alkylene group.

**2.** The compound of formula (I) or the salt thereof according to claim 1, wherein Q is a cyclic amino group of the formula (II-a), Y represents a substituted C1-6 alkyl group, a substituent on the substituted C1-6 alkyl group is a halogeno group, a hydroxyl group, a C1-6 alkoxy group, a C1-6 haloalkoxy group, a C1-6 alkylthio group, a C1-6 alkylsulfinyl group, a C1-6 alkylsulfonyl group, a CI-6 haloalkylthio group, a CI-6 haloalkylsulfinyl group, a C1-6 haloalkylsulfonyl group, a substituted or unsubstituted C6-10 aryl group, a substituted or unsubstituted C6-10 aryloxy group, a substituted or unsubstituted 5- or 6-membered heteroaryl group, a substituted or unsubstituted 5- or 6-membered heteroaryloxy group, a S-C1-6 alkylsulfonimidoyl group, a N-cyano-S-CI-6 alkylsulfonimidoyl group, a carboxyl group, a CI-6 alkylcarbonyl group, a C1-6 alkoxycarbonyl group, a C1-6 alkylcarbonylthio group, a group of $R^jR^kN\text{-}$, a group of $R^jR^kN\text{-}CO\text{-}$, a group of $R^jR^kN\text{-}SO_2\text{-}$, a group of $R^mCONR^n\text{-}$, a group of $R^mSO_2NR^n\text{-}$, a group of $R^pO\text{-}N{=}CR^q\text{-}$, or a cyano group,
$R^j$ represents a C1-6 alkyl group or a CI-6 alkoxy group, $R^k$ represents a hydrogen atom or a C1-6 alkyl group, $R^j$ and $R^k$ may be combined to form a C3-6 alkylene group,
$R^m$ represents a C1-6 alkyl group, $R^n$ represents a hydrogen atom or a C1-6 alkyl group,
$R^p$ represents a C1-6 alkyl group, and $R^q$ represents a hydrogen atom or a C1-6 alkyl group.

**3.** A pest control agent comprising, as an active ingredient thereof, at least one selected from the group consisting of a compound and a salt thereof of claim 1.

4. An insecticide or acaricide comprising, as an active ingredient thereof, at least one selected from the group consisting of a compound and a salt thereof of claim 1.

5. An ectoparasite control or expellant agent comprising, as an active ingredient thereof, at least one selected from the group consisting of a compound and a salt thereof of claim 1.

**INTERNATIONAL SEARCH REPORT**

International application No.
PCT/JP2018/030637

A. CLASSIFICATION OF SUBJECT MATTER
Int.Cl. See extra sheet

According to International Patent Classification (IPC) or to both national classification and IPC

B. FIELDS SEARCHED

Minimum documentation searched (classification system followed by classification symbols)
Int.Cl. See extra sheet

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2015/032280 A1 (SINOCHEM CORPORATION) 12 March 2015, claims, general formulas I-29, 30, 31, table 71, example 7 & CN 104418800 A | 1,3-5<br>2 |
| A | CN 106467537 A (SINOCHEM CORPORATION) 01 March 2017, claims, examples (Family: none) | 1-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 November 2018 (05.11.2018) | 13 November 2018 (13.11.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/030637

C (Continuation). DOCUMENTS CONSIDERED TO BE RELEVANT

| Category* | Citation of document, with indication, where appropriate, of the relevant passages | Relevant to claim No. |
|---|---|---|
| A | WO 2016/096942 A1 (BAYER CROPSCIENCE AKTIENGESELLSCHAFT) 23 June 2016, claims, compound 536 & AR 103024 A | 1-5 |
| A | JP 2003-206230 A (YAMANOUCHI PHARMACEUTICAL CO., LTD.) 22 July 2003, claims, compound A75 (Family: none) | 1-5 |
| A | JP 2011-524400 A (F. HOFFMANN-LA ROCHE AG.) 01 September 2011, claims, example 42 & US 2009/0318467 A1, claims, example 42 & WO 2009/153182 A1 & EP 2297137 A1 & KR 10-2011-0010785 A & CN 102066359 A & KR 10-1357966 B | 1-5 |
| P,A | WO 2017/195703 A1 (NIPPON SODA CO., LTD.) 16 November 2017, claims, examples (Family: none) | 1-5 |

Form PCT/ISA/210 (continuation of second sheet) (January 2015)

**INTERNATIONAL SEARCH REPORT**

International application No.
PCT/JP2018/030637

CLASSIFICATION OF SUBJECT MATTER

C07D213/85(2006.01)i, A01N43/40(2006.01)i, A01N43/56(2006.01)i, A01N43/60(2006.01)i, A01N43/72(2006.01)i, A01N43/76(2006.01)i, A01N43/80(2006.01)i, A01N43/836(2006.01)i, A01N43/84(2006.01)i, A01N43/86(2006.01)i, A01N43/90(2006.01)i, A01N47/02(2006.01)i, A01N47/16(2006.01)i, A01N47/40(2006.01)i, A01P7/02(2006.01)i, A01P7/04(2006.01)i, A61K31/444(2006.01)i, A61K31/4545(2006.01)i, A61K31/496(2006.01)i, A61K31/5355(2006.01)i, A61K31/5377(2006.01)i, A61K31/541(2006.01)i, A61P33/14(2006.01)i, C07D401/06(2006.01)i, C07D401/14(2006.01)i, C07D413/14(2006.01)i, C07D417/14(2006.01)i, C07D471/10(2006.01)i, C07D491/107(2006.01)i, C07D491/113(2006.01)i, C07D495/10(2006.01)i

Form PCT/ISA/210 (extra sheet) (January 2015)

INTERNATIONAL SEARCH REPORT

International application No.
PCT/JP2018/030637

Minimum documentation searched (classification system followed by classification symbols)
C07D213/85, A01N43/40, A01N43/56, A01N43/60, A01N43/72, A01N43/76, A01N43/80, A01N43/836, A01N43/84, A01N43/86, A01N43/90, A01N47/02, A01N47/16, A01N47/40, A01P7/02, A01P7/04, A61K31/444, A61K31/4545, A61K31/496, A61K31/5355, A61K31/5377, A61K31/541, A61P33/14, C07D401/06, C07D401/14, C07D413/14, C07D417/14, C07D471/10, C07D491/107, C07D491/113, C07D495/10

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017159583 A
- JP 2017174777 A
- JP 2017221113 A
- WO 2015032280 A1